(19) Europäisches Patentamt
European Patent Office
Office européen des brevets

(11) EP 3 040 345 A1

(12) EUROPEAN PATENT APPLICATION

(43) Date of publication:
**06.07.2016 Bulletin 2016/27**

(51) Int Cl.:
***C07K 14/435*** *(2006.01)* ***C12N 5/075*** *(2010.01)*
***C12N 5/10*** *(2006.01)* ***C12Q 1/02*** *(2006.01)*

(21) Application number: **14200741.8**

(22) Date of filing: **31.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Centre National de la Recherche Scientifique (CNRS)**
  **75016 Paris (FR)**
- **Université de Montpellier 2**
  **34090 Montpellier (FR)**
- **Université Laval**
  **Québec, QC G1V 0A6 (CA)**
- **INSTITUT NATIONAL DE LA RECHERCHE AGRONOMIQUE (INRA)**
  **75007 Paris (FR)**

(72) Inventors:
- **Chahine, Mohammed**
  **Québec, Québec QB GIJ 2G3 (CA)**
- **Gosselin-Badaroudine, Pascal**
  **Québec, Québec GIJ 2G3 (CA)**
- **Charnet, Pierre**
  **34270 Saint Mathieu de Tréviers (FR)**
- **Cens, Thierry**
  **34920 Le Crès (FR)**
- **Rousset, Matthieu**
  **34000 Montpellier (FR)**
- **Collet, Claude**
  **84130 Le Pontet (FR)**

(74) Representative: **Icosa**
**83 avenue Denfert-Rochereau**
**75014 Paris (FR)**

# (54) Sodium channel subunits of pollinator insects and uses thereof

(57) The present invention relates to the cloning of the sodium channel Para and its regulatory subunits in pollinator insect such as *Apis mellifera* and its uses thereof.

EP 3 040 345 A1

SEQUENCE LISTING

<110> Centre national de la recherche scientifique

<120> SODIUM CHANNEL SUBUNITS OF POLLINATOR INSECTS AND USES THEREOF

<130> CV - 315/EP

<160> 38

<170> BiSSAP 1.2

<210> 1
<211> 6135
<212> DNA
<213> Apis mellifera

<220>
<221> source
<222> 1..6135
<223> /mol_type="unassigned DNA"
/note="Para subunit"
/organism="Apis mellifera"

<400> 1
atgtccgaag attctgactc tgtatcagag gaagaaagaa gtttgttccg tccgttcacg 60

cgggagtccc tggcagcgat cgaggctcgc atcgccgagg aacatgccaa gcagaaggag 120

ctcgagaaga agagagcgga aggcgaggga ggttttggac ggaagaaaaa gaaaaaagaa 180

gttcgatatg acgacgagga cgaggacgag ggtcctcagc cggatccgat gctcgaacaa 240

ggagcaccga tcccggtccg actgcacaac gaatttccac ccgagttggc ctccacacct 300

ctcgaggata tcgatagctt ctatcataat caaaggacct tcgtcgtcat cagcaagggg 360

aaggacatat tcaggttctc agcgacagat gcgatgtgga tcctcgaccc gttcaatccg 420

atacgacgag tggccattta tatattggtt cacccactgt tctccctctt cattatcact 480

acaatattgg ttaactgcat actcatgatc atgcccacta cgcccaccat cgagtctacg 540

gaagtgatat ttacgggcat ctacacattt gagtccgccg ttaaggtgat ggcgaggggt 600

ttcattctgc agccttttac ctatcttaga gatgcatgga attggctcga cttcgtagtt 660

atagctttag cttatgtgac gatgggcata gatctaggca accttgccgc tctcaggaca 720

tttcgagtcc tccgagcctt gaagactgtc gctattgtac caggtctgaa aaccattgtc 780

ggcgctgtga tagaatccgt gaagaacctg cgcgatgtga taatcttgac gatgttctct 840

ctttccgtct ttgcgctgat gggcctccag atttacatgg gggtgctcac gcaaaagtgt 900

ataaagaact ttcccgagga cggctcctgg ggcaatctga ccgatgaaaa ctgggagcga 960

ttcgttagta atgaaactaa ttggtacgtg gacgaggcga aaaacatgcc tttgtgtgga 1020

aattcatctg gagcagggca gtgccttcct ggttacacgt gtttacaagg atacggtgaa 1080

aatccgaatt acggttacac gagtttcgac accttcggct gggctttatt atccgccttt 1140

cgtctaatga cgcaagatta ttgggaaaat ctgtaccagt tggttctaag atcggccggt 1200
ccatggcaca tgttgttctt catcgtgatc atttttcttg gctcctttta tctcgtcaac 1260
ttgattcttg ctattgtcgc catgtcttac gatgaattgc agaagaaagc tgaggaagag 1320
gaggcagccg aggaggaagc tataagagaa gccgaggaag cggccctggc gaaggagaac 1380
aaattggcgg cacaagcggc agcccgagag gcggcagcac atgcggcggt gacagccgct 1440
gaccagatag tcaaatctcc atcggatttc tcgtgtcaca gctacgaatt gttcgttggc 1500
caagagaaag ggaacgacga taacaacaag gagaggatga gcatacgttc gatcgaatcg 1560
gtcagcgagc acagagtgaa acagatcaac aatcacacga ccaccactaa agtgcgaaaa 1620
gtcagcgccg tctctcgcgc cgctaatggc cagtttactt atgcctacca ggaaagcctg 1680
cggaaggcga gccttagcct gcccggctca ccgttcaatc ttcggcggag cagccgcggt 1740
agccatcagt tcacgatcag aaacgggagg ggacgtttcg tggggccgcc gggcggcgac 1800
cggaagccgt tggttctgtc cacgttcctc gacgcccagg aacatctgcc gtacgcggac 1860
gactcgaacg cggtcacccc catgtccgag gagaacggga cgatcgtcgt gcccgtctac 1920
tacgcgaacc ttgggtccag gcattcctcg tacacgtcgc acgcctcacg cctgtcgtac 1980
acgtcccacg gcgatctgat cggcgggata gcgaacgcgg gcaaaccgat gaccaaggag 2040
agccagttga ggatcagatc tgtcaggccg ccgtctgtca atgggcatat cacggataac 2100
aatcagaagt accattacga gggtgatctg gaagatgcca tgggcaaggc gaagcaacaa 2160
gacaatccgt ttatagagcc ttctcaacag catgccgtgg ttgatatgaa agacgtgatg 2220
gtgttgaacg atatcattga acaggcggcg ggacgtcaga gcagaactcc ggagcaagga 2280
gacgacgatg aagaagggcc gacatttaag gacaaattat tgaccgcggt gctacgttgc 2340
atcgatatct tctgcgtgtg ggactgttgc tggttgtggc ttgagtttca aaaatacgtg 2400
gctcttttgg tgttcgatcc attcgtagag ttgttcatca ccctttgtat cgtcgtcaac 2460
acactattca tggcacttga tcatcatgat atggacaagg atatggaaag ggtgctcaag 2520
acgggaaatt atttcttcac ggcaacattc ggtatcgaag caacgttgaa gctgatagca 2580
atgagtccga aattttactt tcaagaagga tggaatattt tcgacttcat tatcgtcgct 2640
ctttcgctct tggagttagg attagagggt gtgcaaggtc tttccgtgtt acgatcgttc 2700
agattgttga gagtgttcaa attggcaaaa tcgtggccta cgttgaatct gttaatttcc 2760
atcatgggta gaacggtagg cgcgttgggc aatctgacgt tcgtgttgtg catcatcatc 2820
ttcattttcg ccgtgatggg catgcaattg ttcggcaaga actacactga caacgtggac 2880
cgatttccgg acggtgatct accgagatgg aatttcaccg acttcatgca ctcgttcatg 2940
atcgtgtttc gggtgctgtg cggggaatgg atcgagtcta tgtgggactg catgctcgtg 3000
ggcgatgttt cttgcatacc gttctttctg gccactgtcg tcatcggtaa tctggttgtg 3060

ctgaatctct tcttggcttt gttgctgagc aacttcggct cgtcgaatct atcggcgccg 3120
accgcagaca acgatacgaa caagatcgcg gaagcgatcg atcgaatagc acgattcgtt 3180
aagtggatca agcgaaatgt cctttatctt gctaaaatga tgcgagccaa actcaccaat 3240
cagatatccg atcaggcgcc agatggaatt gatcgcgatg gggacctaga tcttgcagat 3300
ggcgaactcg atgcgtatag agacaagaag agtgccaagg agctgaacca attggaagtt 3360
gctattgggg acgggatgga attcacgatt catggagacc taaagaacaa attgaagaag 3420
ggtaaattgt gcatgaacaa cagcaaagtt atagcaaatt cgataaatca tcgtgactac 3480
aggctggata atgattatat taatcaaaat gaggatgata ccatcagtaa taaatcatat 3540
ggcagccata aaaatagggc gttcaaggac gaaagtcata aggggagcat ggattcattg 3600
gatggagaag aaaagaagga tgcgagcaaa gaagatttgg aacaagaaga agatcttggc 3660
gaagaaggag aggaagggga aggtgtgata ggcgatgcta ttatccaagc agaggaagat 3720
cccatcgaat ccgattatcc agccgattgt tgtccagaaa attgttacaa gaagttccca 3780
ttcttggctg gcgacgacga cgcaccgttt tggcagggtt gggctaattt gagactgaag 3840
accttccaac taattgagaa caagtatttt gagactgccg tcattttgat gatccttttg 3900
agtagtatgg ctctcgcctt ggaagatgtg catcttcaac aacgacccat tctgcaagat 3960
atcttgtact acatggaccg aatatttact gtgatttttt tcatcgaaat gttaatcaaa 4020
tggttggctc ttggcttcaa aaaatacttc acgaacgctt ggtgctggct tgattttatc 4080
attgtcatgg tgtcactcat taacttcgta gcgtcgctct gtggcgctgg cgggatccaa 4140
gccttcaaaa caatgaggac cctaagggcc ctaaggccac tcagggcgat gtctagaatg 4200
cagggaatgc gggtagtcgt caatgctttg gttcaagcca ttccatccat tttcaacgtg 4260
ttactggtgt gtcttatctt ctggttgatc ttcgccatta tgggtgtaca gctttttgcc 4320
ggcaaatatt ttaagtgtgt agatgcgaat aaaacaacac tcagccatga aatcattcct 4380
gatcgaaacg cctgcttggc tgagaactac acttgggaaa attctccaat gaattttgac 4440
cacgtaggaa aagcttattt atgcttgttt caagtggcta cgttcaaagg atggattcaa 4500
atcatgaacg atgccatcga ctctagggag ctcaacaaac aaccaattcg tgaaacgaac 4560
atctacatgt atttctattt tgtattcttc attatattcg gatcgttttt tacccttaat 4620
ctattcattg gtgtgatcat cgataacttt aacgagcaaa agaaaaaggc aggaggttcc 4680
ctcgagatgt tcatgacaga agatcagaag aaatattaca acgctatgaa aaagatgggt 4740
agcaagaaac cacttaaagc cattccacgt ccaaggtgga ggccgcaagc aatagtgttt 4800
gaaatagtga cagacaaaaa gttcgatatg ataattatgt tgttcatcgg attgaacatg 4860
cttacaatga cgttggacca ttatcaacaa actcagactt tcagcgatgt tctggactat 4920

ctaaacatga tattcattgt gatattcacc agcgagtgtc tcatgaagat ctttgctcta 4980

cgttaccact atttcaagga gccatggaat ctctttgatt ttgttgttgt tatattgtca 5040

atattaggtc tggttctcag cgatattatt gagaagtact tcgtatcgcc aacattgctt 5100

cgtgtagtaa gggtggcaaa agtcggtcgt gtgcttcgac tcgtgaaagg tgccaagggt 5160

attcgaactc ttcttttcgc cttggcgatg tcgttaccag ctctcttcaa tatttgccta 5220

ttactgtttt tggtgatgtt tatctttgcg atatttggaa tgtcgttctt catgcacgtg 5280

aaagataaga gcggactgga cgatgtgtac aattttaaaa cgttcggaca gtcgatgata 5340

ttgctattcc agatgtcaac gtccgctggt tgggacggtg ttcttgacgg tataataaac 5400

gaagaggact gccaggaacc aaacaacgag ataggatatc cgggcaattg tggttcgtct 5460

acgatcggca ttgcctatct tctctcgtat ttggtgatca gctttttaat cgtgataaat 5520

atgtacatcg ccgtgatctt ggagaattat tctcaagcga ccgaggatgt gcaggaaggt 5580

ttgacggatg acgattacga tatgtattac gagatctggc aacagtttga tccagacggg 5640

acacagtaca tcagatatga tcagttatcc gatttcttgg acgtgctgga accgccattg 5700

cagatacaca agccgaacaa gtacaagatc gtatcgatgg acattcctat atgcaaagga 5760

gatatgatgt tctgcgtgga catcttggac gctctcacca aagacttctt cgcgcgtaag 5820

ggtaatccga ttgaggagac agcagagatc gaggttcaaa cgcgtccagg tgagactggt 5880

tatgaaccgg tctcttccac tttgtggcgt cagcgcgaag aatattgcgc gcgtttgatc 5940

caaaatgctt ggagaaagca caaacagcag cgtctaggcg gacccagcga agaaagcgat 6000

gacgctgaca cggatccacg ggtcagacaa accgcagtct tagtcgagag tgacggtttt 6060

gtaacgaaaa atggccacag agttgtcatc catagccgat cgccgagcgt aacctcgaga 6120

accgcggacg tctga 6135

<210> 2
<211> 2044
<212> PRT
<213> Apis mellifera

<220>
<223> Para subunit

<400> 2
Met Ser Glu Asp Ser Asp Ser Val Ser Glu Glu Glu Arg Ser Leu Phe
1 5 10 15
Arg Pro Phe Thr Arg Glu Ser Leu Ala Ala Ile Glu Ala Arg Ile Ala
20 25 30
Glu Glu His Ala Lys Gln Lys Glu Leu Glu Lys Lys Arg Ala Glu Gly
35 40 45
Glu Gly Gly Phe Gly Arg Lys Lys Lys Lys Lys Glu Val Arg Tyr Asp
50 55 60
Asp Glu Asp Glu Asp Glu Gly Pro Gln Pro Asp Pro Met Leu Glu Gln
65 70 75 80
Gly Ala Pro Ile Pro Val Arg Leu His Asn Glu Phe Pro Pro Glu Leu

```
                85                  90                  95
Ala Ser Thr Pro Leu Glu Asp Ile Asp Ser Phe Tyr His Asn Gln Arg
            100                 105                 110
Thr Phe Val Val Ile Ser Lys Gly Lys Asp Ile Phe Arg Phe Ser Ala
        115                 120                 125
Thr Asp Ala Met Trp Ile Leu Asp Pro Phe Asn Pro Ile Arg Arg Val
    130                 135                 140
Ala Ile Tyr Ile Leu Val His Pro Leu Phe Ser Leu Phe Ile Ile Thr
145                 150                 155                 160
Thr Ile Leu Val Asn Cys Ile Leu Met Ile Met Pro Thr Thr Pro Thr
                165                 170                 175
Ile Glu Ser Thr Glu Val Ile Phe Thr Gly Ile Tyr Thr Phe Glu Ser
            180                 185                 190
Ala Val Lys Val Met Ala Arg Gly Phe Ile Leu Gln Pro Phe Thr Tyr
        195                 200                 205
Leu Arg Asp Ala Trp Asn Trp Leu Asp Phe Val Val Ile Ala Leu Ala
    210                 215                 220
Tyr Val Thr Met Gly Ile Asp Leu Gly Asn Leu Ala Ala Leu Arg Thr
225                 230                 235                 240
Phe Arg Val Leu Arg Ala Leu Lys Thr Val Ala Ile Val Pro Gly Leu
                245                 250                 255
Lys Thr Ile Val Gly Ala Val Ile Glu Ser Val Lys Asn Leu Arg Asp
            260                 265                 270
Val Ile Ile Leu Thr Met Phe Ser Leu Ser Val Phe Ala Leu Met Gly
        275                 280                 285
Leu Gln Ile Tyr Met Gly Val Leu Thr Gln Lys Cys Ile Lys Asn Phe
    290                 295                 300
Pro Glu Asp Gly Ser Trp Gly Asn Leu Thr Asp Glu Asn Trp Glu Arg
305                 310                 315                 320
Phe Val Ser Asn Glu Thr Asn Trp Tyr Val Asp Glu Ala Lys Asn Met
                325                 330                 335
Pro Leu Cys Gly Asn Ser Ser Gly Ala Gly Gln Cys Leu Pro Gly Tyr
            340                 345                 350
Thr Cys Leu Gln Gly Tyr Gly Glu Asn Pro Asn Tyr Gly Tyr Thr Ser
        355                 360                 365
Phe Asp Thr Phe Gly Trp Ala Leu Leu Ser Ala Phe Arg Leu Met Thr
    370                 375                 380
Gln Asp Tyr Trp Glu Asn Leu Tyr Gln Leu Val Leu Arg Ser Ala Gly
385                 390                 395                 400
Pro Trp His Met Leu Phe Phe Ile Val Ile Ile Phe Leu Gly Ser Phe
                405                 410                 415
Tyr Leu Val Asn Leu Ile Leu Ala Ile Val Ala Met Ser Tyr Asp Glu
            420                 425                 430
Leu Gln Lys Lys Ala Glu Glu Glu Glu Ala Ala Glu Glu Glu Ala Ile
        435                 440                 445
Arg Glu Ala Glu Glu Ala Ala Leu Ala Lys Glu Asn Lys Leu Ala Ala
    450                 455                 460
Gln Ala Ala Ala Arg Glu Ala Ala Ala His Ala Ala Val Thr Ala Ala
465                 470                 475                 480
Asp Gln Ile Val Lys Ser Pro Ser Asp Phe Ser Cys His Ser Tyr Glu
                485                 490                 495
Leu Phe Val Gly Gln Glu Lys Gly Asn Asp Asp Asn Asn Lys Glu Arg
            500                 505                 510
Met Ser Ile Arg Ser Ile Glu Ser Val Ser Glu His Arg Val Lys Gln
        515                 520                 525
Ile Asn Asn His Thr Thr Thr Thr Lys Val Arg Lys Val Ser Ala Val
    530                 535                 540
Ser Arg Ala Ala Asn Gly Gln Phe Thr Tyr Ala Tyr Gln Glu Ser Leu
545                 550                 555                 560
Arg Lys Ala Ser Leu Ser Leu Pro Gly Ser Pro Phe Asn Leu Arg Arg
                565                 570                 575
Ser Ser Arg Gly Ser His Gln Phe Thr Ile Arg Asn Gly Arg Gly Arg
            580                 585                 590
```

```
Phe Val Gly Pro Pro Gly Gly Asp Arg Lys Pro Leu Val Leu Ser Thr
        595                 600                 605
Phe Leu Asp Ala Gln Glu His Leu Pro Tyr Ala Asp Asp Ser Asn Ala
    610                 615                 620
Val Thr Pro Met Ser Glu Glu Asn Gly Thr Ile Val Val Pro Val Tyr
625                 630                 635                 640
Tyr Ala Asn Leu Gly Ser Arg His Ser Ser Tyr Thr Ser His Ala Ser
                645                 650                 655
Arg Leu Ser Tyr Thr Ser His Gly Asp Leu Ile Gly Gly Ile Ala Asn
            660                 665                 670
Ala Gly Lys Pro Met Thr Lys Glu Ser Gln Leu Arg Ile Arg Ser Val
        675                 680                 685
Arg Pro Pro Ser Val Asn Gly His Ile Thr Asp Asn Asn Gln Lys Tyr
    690                 695                 700
His Tyr Glu Gly Asp Leu Glu Asp Ala Met Gly Lys Ala Lys Gln Gln
705                 710                 715                 720
Asp Asn Pro Phe Ile Glu Pro Ser Gln Gln His Ala Val Val Asp Met
                725                 730                 735
Lys Asp Val Met Val Leu Asn Asp Ile Ile Glu Gln Ala Ala Gly Arg
            740                 745                 750
Gln Ser Arg Thr Pro Glu Gln Gly Asp Asp Asp Glu Glu Gly Pro Thr
        755                 760                 765
Phe Lys Asp Lys Leu Leu Thr Ala Val Leu Arg Cys Ile Asp Ile Phe
    770                 775                 780
Cys Val Trp Asp Cys Cys Trp Leu Trp Leu Glu Phe Gln Lys Tyr Val
785                 790                 795                 800
Ala Leu Leu Val Phe Asp Pro Phe Val Glu Leu Phe Ile Thr Leu Cys
                805                 810                 815
Ile Val Val Asn Thr Leu Phe Met Ala Leu Asp His His Asp Met Asp
            820                 825                 830
Lys Asp Met Glu Arg Val Leu Lys Thr Gly Asn Tyr Phe Phe Thr Ala
        835                 840                 845
Thr Phe Gly Ile Glu Ala Thr Leu Lys Leu Ile Ala Met Ser Pro Lys
    850                 855                 860
Phe Tyr Phe Gln Glu Gly Trp Asn Ile Phe Asp Phe Ile Ile Val Ala
865                 870                 875                 880
Leu Ser Leu Leu Glu Leu Gly Leu Glu Gly Val Gln Gly Leu Ser Val
                885                 890                 895
Leu Arg Ser Phe Arg Leu Leu Arg Val Phe Lys Leu Ala Lys Ser Trp
            900                 905                 910
Pro Thr Leu Asn Leu Leu Ile Ser Ile Met Gly Arg Thr Val Gly Ala
        915                 920                 925
Leu Gly Asn Leu Thr Phe Val Leu Cys Ile Ile Ile Phe Ile Phe Ala
    930                 935                 940
Val Met Gly Met Gln Leu Phe Gly Lys Asn Tyr Thr Asp Asn Val Asp
945                 950                 955                 960
Arg Phe Pro Asp Gly Asp Leu Pro Arg Trp Asn Phe Thr Asp Phe Met
                965                 970                 975
His Ser Phe Met Ile Val Phe Arg Val Leu Cys Gly Glu Trp Ile Glu
            980                 985                 990
Ser Met Trp Asp Cys Met Leu Val Gly Asp Val Ser Cys Ile Pro Phe
        995                 1000                1005
Phe Leu Ala Thr Val Val Ile Gly Asn Leu Val Val Leu Asn Leu Phe
    1010                1015                1020
Leu Ala Leu Leu Leu Ser Asn Phe Gly Ser Ser Asn Leu Ser Ala Pro
1025                1030                1035                1040
Thr Ala Asp Asn Asp Thr Asn Lys Ile Ala Glu Ala Ile Asp Arg Ile
                1045                1050                1055
Ala Arg Phe Val Lys Trp Ile Lys Arg Asn Val Leu Tyr Leu Ala Lys
            1060                1065                1070
Met Met Arg Ala Lys Leu Thr Asn Gln Ile Ser Asp Gln Ala Pro Asp
        1075                1080                1085
Gly Ile Asp Arg Asp Gly Asp Leu Asp Leu Ala Asp Gly Glu Leu Asp
```

```
    1090                1095                1100
Ala Tyr Arg Asp Lys Lys Ser Ala Lys Glu Leu Asn Gln Leu Glu Val
1105                1110                1115                1120
Ala Ile Gly Asp Gly Met Glu Phe Thr Ile His Gly Asp Leu Lys Asn
                1125                1130                1135
Lys Leu Lys Lys Gly Lys Leu Cys Met Asn Asn Ser Lys Val Ile Ala
            1140                1145                1150
Asn Ser Ile Asn His Arg Asp Tyr Arg Leu Asp Asn Asp Tyr Ile Asn
        1155                1160                1165
Gln Asn Glu Asp Asp Thr Ile Ser Asn Lys Ser Tyr Gly Ser His Lys
    1170                1175                1180
Asn Arg Ala Phe Lys Asp Glu Ser His Lys Gly Ser Met Asp Ser Leu
1185                1190                1195                1200
Asp Gly Glu Glu Lys Lys Asp Ala Ser Lys Glu Asp Leu Glu Gln Glu
                1205                1210                1215
Glu Asp Leu Gly Glu Glu Gly Glu Glu Gly Glu Gly Val Ile Gly Asp
            1220                1225                1230
Ala Ile Ile Gln Ala Glu Glu Asp Pro Ile Glu Ser Asp Tyr Pro Ala
        1235                1240                1245
Asp Cys Cys Pro Glu Asn Cys Tyr Lys Lys Phe Pro Phe Leu Ala Gly
    1250                1255                1260
Asp Asp Asp Ala Pro Phe Trp Gln Gly Trp Ala Asn Leu Arg Leu Lys
1265                1270                1275                1280
Thr Phe Gln Leu Ile Glu Asn Lys Tyr Phe Glu Thr Ala Val Ile Leu
                1285                1290                1295
Met Ile Leu Leu Ser Ser Met Ala Leu Ala Leu Glu Asp Val His Leu
            1300                1305                1310
Gln Gln Arg Pro Ile Leu Gln Asp Ile Leu Tyr Tyr Met Asp Arg Ile
        1315                1320                1325
Phe Thr Val Ile Phe Phe Ile Glu Met Leu Ile Lys Trp Leu Ala Leu
    1330                1335                1340
Gly Phe Lys Lys Tyr Phe Thr Asn Ala Trp Cys Trp Leu Asp Phe Ile
1345                1350                1355                1360
Ile Val Met Val Ser Leu Ile Asn Phe Val Ala Ser Leu Cys Gly Ala
                1365                1370                1375
Gly Gly Ile Gln Ala Phe Lys Thr Met Arg Thr Leu Arg Ala Leu Arg
            1380                1385                1390
Pro Leu Arg Ala Met Ser Arg Met Gln Gly Met Arg Val Val Val Asn
        1395                1400                1405
Ala Leu Val Gln Ala Ile Pro Ser Ile Phe Asn Val Leu Leu Val Cys
    1410                1415                1420
Leu Ile Phe Trp Leu Ile Phe Ala Ile Met Gly Val Gln Leu Phe Ala
1425                1430                1435                1440
Gly Lys Tyr Phe Lys Cys Val Asp Ala Asn Lys Thr Thr Leu Ser His
                1445                1450                1455
Glu Ile Ile Pro Asp Arg Asn Ala Cys Leu Ala Glu Asn Tyr Thr Trp
            1460                1465                1470
Glu Asn Ser Pro Met Asn Phe Asp His Val Gly Lys Ala Tyr Leu Cys
        1475                1480                1485
Leu Phe Gln Val Ala Thr Phe Lys Gly Trp Ile Gln Ile Met Asn Asp
    1490                1495                1500
Ala Ile Asp Ser Arg Glu Leu Asn Lys Gln Pro Ile Arg Glu Thr Asn
1505                1510                1515                1520
Ile Tyr Met Tyr Phe Tyr Phe Val Phe Phe Ile Ile Phe Gly Ser Phe
                1525                1530                1535
Phe Thr Leu Asn Leu Phe Ile Gly Val Ile Ile Asp Asn Phe Asn Glu
            1540                1545                1550
Gln Lys Lys Lys Ala Gly Gly Ser Leu Glu Met Phe Met Thr Glu Asp
        1555                1560                1565
Gln Lys Lys Tyr Tyr Asn Ala Met Lys Lys Met Gly Ser Lys Lys Pro
    1570                1575                1580
Leu Lys Ala Ile Pro Arg Pro Arg Trp Arg Pro Gln Ala Ile Val Phe
1585                1590                1595                1600
```

```
Glu Ile Val Thr Asp Lys Lys Phe Asp Met Ile Ile Met Leu Phe Ile
                1605                1610                1615
Gly Leu Asn Met Leu Thr Met Thr Leu Asp His Tyr Gln Gln Thr Gln
            1620                1625                1630
Thr Phe Ser Asp Val Leu Asp Tyr Leu Asn Met Ile Phe Ile Val Ile
        1635                1640                1645
Phe Thr Ser Glu Cys Leu Met Lys Ile Phe Ala Leu Arg Tyr His Tyr
    1650                1655                1660
Phe Lys Glu Pro Trp Asn Leu Phe Asp Phe Val Val Val Ile Leu Ser
1665                1670                1675                1680
Ile Leu Gly Leu Val Leu Ser Asp Ile Ile Glu Lys Tyr Phe Val Ser
                1685                1690                1695
Pro Thr Leu Leu Arg Val Val Arg Val Ala Lys Val Gly Arg Val Leu
            1700                1705                1710
Arg Leu Val Lys Gly Ala Lys Gly Ile Arg Thr Leu Leu Phe Ala Leu
        1715                1720                1725
Ala Met Ser Leu Pro Ala Leu Phe Asn Ile Cys Leu Leu Leu Phe Leu
    1730                1735                1740
Val Met Phe Ile Phe Ala Ile Phe Gly Met Ser Phe Phe Met His Val
1745                1750                1755                1760
Lys Asp Lys Ser Gly Leu Asp Asp Val Tyr Asn Phe Lys Thr Phe Gly
                1765                1770                1775
Gln Ser Met Ile Leu Leu Phe Gln Met Ser Thr Ser Ala Gly Trp Asp
            1780                1785                1790
Gly Val Leu Asp Gly Ile Ile Asn Glu Glu Asp Cys Gln Glu Pro Asn
        1795                1800                1805
Asn Glu Ile Gly Tyr Pro Gly Asn Cys Gly Ser Ser Thr Ile Gly Ile
    1810                1815                1820
Ala Tyr Leu Leu Ser Tyr Leu Val Ile Ser Phe Leu Ile Val Ile Asn
1825                1830                1835                1840
Met Tyr Ile Ala Val Ile Leu Glu Asn Tyr Ser Gln Ala Thr Glu Asp
                1845                1850                1855
Val Gln Glu Gly Leu Thr Asp Asp Asp Tyr Asp Met Tyr Tyr Glu Ile
            1860                1865                1870
Trp Gln Gln Phe Asp Pro Asp Gly Thr Gln Tyr Ile Arg Tyr Asp Gln
        1875                1880                1885
Leu Ser Asp Phe Leu Asp Val Leu Glu Pro Pro Leu Gln Ile His Lys
    1890                1895                1900
Pro Asn Lys Tyr Lys Ile Val Ser Met Asp Ile Pro Ile Cys Lys Gly
1905                1910                1915                1920
Asp Met Met Phe Cys Val Asp Ile Leu Asp Ala Leu Thr Lys Asp Phe
                1925                1930                1935
Phe Ala Arg Lys Gly Asn Pro Ile Glu Glu Thr Ala Glu Ile Glu Val
            1940                1945                1950
Gln Thr Arg Pro Gly Glu Thr Gly Tyr Glu Pro Val Ser Ser Thr Leu
        1955                1960                1965
Trp Arg Gln Arg Glu Glu Tyr Cys Ala Arg Leu Ile Gln Asn Ala Trp
    1970                1975                1980
Arg Lys His Lys Gln Gln Arg Leu Gly Gly Pro Ser Glu Glu Ser Asp
1985                1990                1995                2000
Asp Ala Asp Thr Asp Pro Arg Val Arg Gln Thr Ala Val Leu Val Glu
                2005                2010                2015
Ser Asp Gly Phe Val Thr Lys Asn Gly His Arg Val Val Ile His Ser
            2020                2025                2030
Arg Ser Pro Ser Val Thr Ser Arg Thr Ala Asp Val
        2035                2040
```

<210> 3
<211> 1071
<212> DNA
<213> Apis mellifera

<220>

```
<221> source
<222> 1..1071
<223> /mol_type="unassigned DNA"
      /note="TipE subunit"
      /organism="Apis mellifera"

<400> 3
atggcggagg aaaaggagaa gcagacgttc ctgcagaagc tgctgttcta cacgaccgcg      60

ttcttcatcc tcctcagcac cttcagcctc tttgccttcc tcttcttggt cccccttcgtc     120

atcgaccccg ccttcaccac catcttcatg cagttcgaca ctcggccagc cgagtgtatc     180

actacctacg ttgagtcaag gagaggtacg agcaactgct catggacatc ctgtagagag     240

ggttgcacca aggagctgta cgattgcacg caaatacgcg tcaactacaa gctaccgact     300

aatacgtccg aggatagcga tggacagggt gagggaggcg gagcggtagg aggtgtcgag     360

gatgacgaag acagcacgac gatgggaaaa ccgcgttatg accgctccct aagggagtac     420

gattacgtcg aggaccttga cgaggatttc gccgaggacg acgaagcagg actgccgaaa     480

ccctttccta cagggctcat gggcaacgac tccgagtggt acttcatcgg ggccaagctg     540

ttccccaacg taaaaggctg tggataccct ccaatgctga attgcagtat tttctaccgg     600

cagtacgcca tcatagggca gaactttagc tgttactact cgaaagtgga ccccgggata     660

gtcatcagcg acctcgacat gtggcaggtg tacatgaatc tggtgtacgc gatggcgatt     720

ccgataccgt ctttcataat ctcggtgata tatctcacca tcgcctactt caagatctac     780

aacgaagacg aggtggtcct cgtgggcggg gaggagggcg aggagggcgg ggagggcgaa     840

gggagtaacg cgacacccct gccactgacg agcggcgccc ttacgcccgg cagcgaggct     900

ttcagggaag acctagcgag ctttgggcat cagctgaagg tcgccatggc cgacgacatc     960

agcagggaaa gcctggatgg gataccaaac tcgtactcca ttcagggaaa cttgagcaag    1020

acgatgacga ccagtatctc gactccccct gggccgacgg cggcagtctg a             1071


<210> 4
<211> 356
<212> PRT
<213> Apis mellifera

<220>
<223> TipE subunit

<400> 4
Met Ala Glu Glu Lys Glu Lys Gln Thr Phe Leu Gln Lys Leu Leu Phe
1               5                   10                  15
Tyr Thr Thr Ala Phe Phe Ile Leu Leu Ser Thr Phe Ser Leu Phe Ala
            20                  25                  30
Phe Leu Phe Leu Val Pro Phe Val Ile Asp Pro Ala Phe Thr Thr Ile
        35                  40                  45
Phe Met Gln Phe Asp Thr Arg Pro Ala Glu Cys Ile Thr Thr Tyr Val
    50                  55                  60
Glu Ser Arg Arg Gly Thr Ser Asn Cys Ser Trp Thr Ser Cys Arg Glu
65                  70                  75                  80
```

```
Gly Cys Thr Lys Glu Leu Tyr Asp Cys Thr Gln Ile Arg Val Asn Tyr
                85                  90                  95
Lys Leu Pro Thr Asn Thr Ser Glu Asp Ser Asp Gly Gln Gly Glu Gly
            100                 105                 110
Gly Gly Ala Val Gly Gly Val Glu Asp Asp Glu Asp Ser Thr Thr Met
        115                 120                 125
Gly Lys Pro Arg Tyr Asp Arg Ser Leu Arg Glu Tyr Asp Tyr Val Glu
    130                 135                 140
Asp Leu Asp Glu Asp Phe Ala Glu Asp Asp Glu Ala Gly Leu Pro Lys
145                 150                 155                 160
Pro Phe Pro Thr Gly Leu Met Gly Asn Asp Ser Glu Trp Tyr Phe Ile
                165                 170                 175
Gly Ala Lys Leu Phe Pro Asn Val Lys Gly Cys Gly Tyr Pro Pro Met
            180                 185                 190
Leu Asn Cys Ser Ile Phe Tyr Arg Gln Tyr Ala Ile Ile Gly Gln Asn
        195                 200                 205
Phe Ser Cys Tyr Tyr Ser Lys Val Asp Pro Gly Ile Val Ile Ser Asp
    210                 215                 220
Leu Asp Met Trp Gln Val Tyr Met Asn Leu Val Tyr Ala Met Ala Ile
225                 230                 235                 240
Pro Ile Pro Ser Phe Ile Ile Ser Val Ile Tyr Leu Thr Ile Ala Tyr
                245                 250                 255
Phe Lys Ile Tyr Asn Glu Asp Glu Val Val Leu Val Gly Gly Glu Glu
            260                 265                 270
Gly Glu Glu Gly Gly Glu Gly Glu Gly Ser Asn Ala Thr Pro Leu Pro
        275                 280                 285
Leu Thr Ser Gly Ala Leu Thr Pro Gly Ser Glu Ala Phe Arg Glu Asp
    290                 295                 300
Leu Ala Ser Phe Gly His Gln Leu Lys Val Ala Met Ala Asp Asp Ile
305                 310                 315                 320
Ser Arg Glu Ser Leu Asp Gly Ile Pro Asn Ser Tyr Ser Ile Gln Gly
                325                 330                 335
Asn Leu Ser Lys Thr Met Thr Thr Ser Ile Ser Thr Pro Pro Gly Pro
            340                 345                 350
Thr Ala Ala Val
        355
```

<210> 5
<211> 891
<212> DNA
<213> Apis mellifera

<220>
<221> source
<222> 1..891
<223> /mol_type="unassigned DNA"
/note="Teh1 subunit"
/organism="Apis mellifera"

<400> 5

```
atgcgcggga gcagctctga gctcctgctg gaggcgagct gcgtccagtg tcagagcgat      60

catcacgccg accagcacac cggcaccagg catcaccagc aacatcaggt gaacgagaaa     120

cacgacttcc aggagcagga gctacgtcgt cgcaagctgt tggagctcgg gttcggcgct     180

tcgcggcgcc gaccgccacg gcgtacctgc cgtcagcggt tcaattttta cgcaacgtcg     240

gcgttggcct tcgtcgcgac atccggtggc gcggccttgc tatttttggt gccactttac     300

gtcgatccgg cgatcagcac tctggccgcc gatttctcgc cggacccggt catctgcacc     360

acctccagac gggaggagtt ggccggattg ttcaattgta cttggagttc ttgccgcgag     420
```

```
ggttgtacaa gcgacgttta cagatgtacg catatatacg tcacctacac gccatggagc      480

aacgcgagca tgaaaaacga caccgggggt agaggcaact cgaccggcat cgcgcacacc      540

tcgactactt ccgtgccgac tcccggcgat gtggaggcgg tgctcctggt gaacatcaaa      600

gggtgcggct atccgccgat cgtcgactgc gagaacttca cccgcgaaat gggctacgaa      660

ggcgcgaaat tcccctgcca ttacagccgt gtgaacggca gcatagtgat ggcgaactat      720

aaccgcgagg ctcaggtcac caccatcata cacttcttcg cggcgccttt cgtagtgact      780

ctcgcgacca gcgtcgctct gtgtgtgatg cactgtgact gcaggtgcag cccaccccca      840

cgccattcat cgcgaggaat cagaagaggc aggggcaacg atctcaggta a               891
```

```
<210> 6
<211> 296
<212> PRT
<213> Apis mellifera

<220>
<223> Teh1 subunit

<400> 6
Met Arg Gly Ser Ser Ser Glu Leu Leu Leu Glu Ala Ser Cys Val Gln
1               5                   10                  15
Cys Gln Ser Asp His His Ala Asp Gln His Thr Gly Thr Arg His His
            20                  25                  30
Gln Gln His Gln Val Asn Glu Lys His Asp Phe Gln Glu Gln Glu Leu
        35                  40                  45
Arg Arg Arg Lys Leu Leu Glu Leu Gly Phe Gly Ala Ser Arg Arg Arg
    50                  55                  60
Pro Pro Arg Arg Thr Cys Arg Gln Arg Phe Asn Phe Tyr Ala Thr Ser
65                  70                  75                  80
Ala Leu Ala Phe Val Ala Thr Ser Gly Gly Ala Ala Leu Leu Phe Leu
                85                  90                  95
Val Pro Leu Tyr Val Asp Pro Ala Ile Ser Thr Leu Ala Ala Asp Phe
            100                 105                 110
Ser Pro Asp Pro Val Ile Cys Thr Thr Ser Arg Arg Glu Glu Leu Ala
        115                 120                 125
Gly Leu Phe Asn Cys Thr Trp Ser Ser Cys Arg Glu Gly Cys Thr Ser
    130                 135                 140
Asp Val Tyr Arg Cys Thr His Ile Tyr Val Thr Tyr Thr Pro Trp Ser
145                 150                 155                 160
Asn Ala Ser Met Lys Asn Asp Thr Gly Gly Arg Gly Asn Ser Thr Gly
                165                 170                 175
Ile Ala His Thr Ser Thr Thr Ser Val Pro Thr Pro Gly Asp Val Glu
            180                 185                 190
Ala Val Leu Leu Val Asn Ile Lys Gly Cys Gly Tyr Pro Pro Ile Val
        195                 200                 205
Asp Cys Glu Asn Phe Thr Arg Glu Met Gly Tyr Glu Gly Ala Lys Phe
    210                 215                 220
Pro Cys His Tyr Ser Arg Val Asn Gly Ser Ile Val Met Ala Asn Tyr
225                 230                 235                 240
Asn Arg Glu Ala Gln Val Thr Thr Ile Ile His Phe Phe Ala Ala Pro
                245                 250                 255
Phe Val Val Thr Leu Ala Thr Ser Val Ala Leu Cys Val Met His Cys
            260                 265                 270
Asp Cys Arg Cys Ser Pro Pro Pro Arg His Ser Ser Arg Gly Ile Arg
        275                 280                 285
```

```
Arg Gly Arg Gly Asn Asp Leu Arg
    290                 295

<210> 7
<211> 717
<212> DNA
<213> Apis mellifera

<220>
<221> source
<222> 1..717
<223> /mol_type="unassigned DNA"
      /note="Teh2 subunit"
      /organism="Apis mellifera"

<400> 7
atgggcgccc aggaagagga caccaagtcg agcaccatag gcggggtgct gcctgtggtg       60

gaggtggtga cattggtgga gaaggctaaa ttctacacct ccctctgcct gggcatcacg      120

gccatactcg ccgtcttcgc cttcctcttt ctgatcccgt tcatcgtgga accggcggtg      180

accacgatcc tggccgactt ttccccgcac gctgtcgcct gcgtcgtcac cgatcacgtt      240

tacgccgagg gattgaagaa ttgctcgtgg gcaagctgta gagaaggttg tacgagcgca      300

gcgcttcgtt gccatcagat cagggtgaac tacacgaggc tgtcgttcga ggaattcgtt      360

gcgaaaccgt tgggctccat acagtgggac gtttcggaca cgaagttttt cgtgaacaca      420

gagggctgcg gttacccgcc cagggtgaac tgctccgact tcgccaagaa ctacggatac      480

tcgaacatgg ggaaaatatt cccttgttat tacagcagaa cacatccgga gactgtcgtc      540

gcaagatact cttgggatga aaatctgagg catctagtgt tagccttggt agtgcctaca      600

gttgttttcg gagtgtccct cggtgtatta tgctattggt attgtccacc gatgggcaag      660

acttgcggag gaccaggtcg caacttgatt gacaagtacg caaggaagga agagtaa         717


<210> 8
<211> 238
<212> PRT
<213> Apis mellifera

<220>
<223> Teh2 subunit

<400> 8
Met Gly Ala Gln Glu Glu Asp Thr Lys Ser Ser Thr Ile Gly Gly Val
1               5                   10                  15
Leu Pro Val Val Glu Val Val Thr Leu Val Glu Lys Ala Lys Phe Tyr
            20                  25                  30
Thr Ser Leu Cys Leu Gly Ile Thr Ala Ile Leu Ala Val Phe Ala Phe
        35                  40                  45
Leu Phe Leu Ile Pro Phe Ile Val Glu Pro Ala Val Thr Thr Ile Leu
    50                  55                  60
Ala Asp Phe Ser Pro His Ala Val Ala Cys Val Val Thr Asp His Val
65                  70                  75                  80
Tyr Ala Glu Gly Leu Lys Asn Cys Ser Trp Ala Ser Cys Arg Glu Gly
                85                  90                  95
Cys Thr Ser Ala Ala Leu Arg Cys His Gln Ile Arg Val Asn Tyr Thr
```

```
            100                 105                 110
Arg Leu Ser Phe Glu Glu Phe Val Ala Lys Pro Leu Gly Ser Ile Gln
        115                 120                 125
Trp Asp Val Ser Asp Thr Lys Phe Phe Val Asn Thr Glu Gly Cys Gly
    130                 135                 140
Tyr Pro Pro Arg Val Asn Cys Ser Asp Phe Ala Lys Asn Tyr Gly Tyr
145                 150                 155                 160
Ser Asn Met Gly Lys Ile Phe Pro Cys Tyr Tyr Ser Arg Thr His Pro
                165                 170                 175
Glu Thr Val Val Ala Arg Tyr Ser Trp Asp Glu Asn Leu Arg His Leu
            180                 185                 190
Val Leu Ala Leu Val Val Pro Thr Val Val Phe Gly Val Ser Leu Gly
        195                 200                 205
Val Leu Cys Tyr Trp Tyr Cys Pro Pro Met Gly Lys Thr Cys Gly Gly
    210                 215                 220
Pro Gly Arg Asn Leu Ile Asp Lys Tyr Ala Arg Lys Glu Glu
225                 230                 235

<210> 9
<211> 1341
<212> DNA
<213> Apis mellifera

<220>
<221> source
<222> 1..1341
<223> /mol_type="unassigned DNA"
      /note="Teh3 subunit"
      /organism="Apis mellifera"

<400> 9
atgcctaagc aagtgccagt ggagaacctg gtgatcccgc cgcaggacgg gcggatatgc      60

gggacgattt gcatctgcca gatgacggcg gtcctctcct ccgtcgccct ggtctacctg     120

accgttgcca tctacatgcc gagcaccagg gcgttccaat cgggtataag cgaggtgcca     180

gtcatgtgca cgaccatacg cgccgtgaac gcggacaact gtgaatgggg cagttgcggg     240

gagtggtgcc tgtcgaaaac gtcgggcccc tgcgtccaga tccacgtgaa cctacgtaga     300

aacggttcca ggatattgtt ggccaactgc accaacacga cgaacaagac ttgttacggg     360

atcgatcagg agaacgcgaa gaaatcgaaa tgcatagcgg acgagtgtcg aaatctgaca     420

ggcacgttca actgctcgtc gggggtgtgc atcaacatta cggacgcgtt cgagtgcata     480

ttccacgaca ccgatccacc gatgaagtgt tcgggtagac gaggcaagat aacctgcata     540

gacatagacg gcctgttcaa ctgtaaccgc ggtacgtgcg agcgtatcag aacgccgtac     600

aactgcgatc gcagatgcgt cgatataccg acaaggaaca agaacatgat cctgttgacg     660

ggcgacaagg tttatctcag ccagtgcgag agagctatag acgtgcagac gaatcgggag     720

atatggcacg aggatcgggg ggacgtgatg atggcttcgt gttacgggat attcaactcg     780

accctgggcg tcgaggctgt cgattgcatc aacgggtccg tgttggagaa ggatctgctc     840

accgatctca ccaacttcac ctatctctcc tatctcaaca tattcgctac gaaaccgttg     900

gacgagacaa ggatggtcgc gcccccgaa caggatctaa tcatagcgaa cgagagccgt      960
```

ctgttgatca atctcgaggg ttgcgtgaac actttgaggg aggagtgcaa ggagtttctt 1020

cacgagtacg ggaaggacgg gtcggatcac aacgcgcgag ctagattccc ttgttactac 1080

gccgagagca acacggggat cgttgtttcc cgtttcaatc tggagaacac gtacaaggag 1140

ttcatgatcg cgttgctgtt gcccagcatc ttgttcgtgg tcagttgcct gacgttgatc 1200

ttttgccaga agacggtggt agttggggac gacgccaaga tgagattcaa gggcaccgtt 1260

ggcgcactct cgtccatgga gaagagcgct tcgggtaacc tgggggatgc tggcggagga 1320

aactccgcta tggcgctctg a 1341

<210> 10
<211> 446
<212> PRT
<213> Apis mellifera

<220>
<223> Teh3 subunit

<400> 10
Met Pro Lys Gln Val Pro Val Glu Asn Leu Val Ile Pro Pro Gln Asp
1 5 10 15
Gly Arg Ile Cys Gly Thr Ile Cys Ile Cys Gln Met Thr Ala Val Leu
20 25 30
Ser Ser Val Ala Leu Val Tyr Leu Thr Val Ala Ile Tyr Met Pro Ser
35 40 45
Thr Arg Ala Phe Gln Ser Gly Ile Ser Glu Val Pro Val Met Cys Thr
50 55 60
Thr Ile Arg Ala Val Asn Ala Asp Asn Cys Glu Trp Gly Ser Cys Gly
65 70 75 80
Glu Trp Cys Leu Ser Lys Thr Ser Gly Pro Cys Val Gln Ile His Val
85 90 95
Asn Leu Arg Arg Asn Gly Ser Arg Ile Leu Leu Ala Asn Cys Thr Asn
100 105 110
Thr Thr Asn Lys Thr Cys Tyr Gly Ile Asp Gln Glu Asn Ala Lys Lys
115 120 125
Ser Lys Cys Ile Ala Asp Glu Cys Arg Asn Leu Thr Gly Thr Phe Asn
130 135 140
Cys Ser Ser Gly Val Cys Ile Asn Ile Thr Asp Ala Phe Glu Cys Ile
145 150 155 160
Phe His Asp Thr Asp Pro Pro Met Lys Cys Ser Gly Arg Arg Gly Lys
165 170 175
Ile Thr Cys Ile Asp Ile Asp Gly Leu Phe Asn Cys Asn Arg Gly Thr
180 185 190
Cys Glu Arg Ile Arg Thr Pro Tyr Asn Cys Asp Arg Arg Cys Val Asp
195 200 205
Ile Pro Thr Arg Asn Lys Asn Met Ile Leu Leu Thr Gly Asp Lys Val
210 215 220
Tyr Leu Ser Gln Cys Glu Arg Ala Ile Asp Val Gln Thr Asn Arg Glu
225 230 235 240
Ile Trp His Glu Asp Arg Gly Asp Val Met Met Ala Ser Cys Tyr Gly
245 250 255
Ile Phe Asn Ser Thr Leu Gly Val Glu Ala Val Asp Cys Ile Asn Gly
260 265 270
Ser Val Leu Glu Lys Asp Leu Leu Thr Asp Leu Thr Asn Phe Thr Tyr
275 280 285
Leu Ser Tyr Leu Asn Ile Phe Ala Thr Lys Pro Leu Asp Glu Thr Arg
290 295 300
Met Val Ala Pro Pro Glu Gln Asp Leu Ile Ile Ala Asn Glu Ser Arg

```
305                 310                 315                 320
Leu Leu Ile Asn Leu Glu Gly Cys Val Asn Thr Leu Arg Glu Glu Cys
                325                 330                 335
Lys Glu Phe Leu His Glu Tyr Gly Lys Asp Gly Ser Asp His Asn Ala
            340                 345                 350
Arg Ala Arg Phe Pro Cys Tyr Tyr Ala Glu Ser Asn Thr Gly Ile Val
        355                 360                 365
Val Ser Arg Phe Asn Leu Glu Asn Thr Tyr Lys Glu Phe Met Ile Ala
    370                 375                 380
Leu Leu Leu Pro Ser Ile Leu Phe Val Val Ser Cys Leu Thr Leu Ile
385                 390                 395                 400
Phe Cys Gln Lys Thr Val Val Val Gly Asp Asp Ala Lys Met Arg Phe
                405                 410                 415
Lys Gly Thr Val Gly Ala Leu Ser Ser Met Glu Lys Ser Ala Ser Gly
            420                 425                 430
Asn Leu Gly Asp Ala Gly Gly Gly Asn Ser Ala Met Ala Leu
        435                 440                 445
```

<210> 11
<211> 1386
<212> DNA
<213> Apis mellifera

<220>
<221> source
<222> 1..1386
<223> /mol_type="unassigned DNA"
/note="Teh4 subunit"
/organism="Apis mellifera"

<400> 11

```
atgggtcgaa agcataaacg ccgccttata ccggaacagg atcgcaggat atgcgggagt      60

atctgcttct gccagttcac cattgtgatc agttgcgtcg cgttggttta tctaagcgtg     120

gcgatctata tgccgtccca cagagctttt cacgcgggga tcgatcccga tccagtcatg     180

tgccaaaccg ttaacaccac cttaactaat aattgcggtt gggcgagttg cggggagtgg     240

tgcttgacaa gaaccacggg attctgccct cagattcacg cgaccgtaag aaggaatgga     300

acggacatcg ttttcgaaaa ttgcacgaaa ttcaacagca tatcctgccc tcaggtgaac     360

acggcttccc ttaaaaaata caattgcaac aacggcagcg agtgcagcgt gctttccggc     420

ttgtttaact gcagcctcgg ccactgcgtg aatataagcg agctgatgtt gtgccattac     480

aaggcagacg gaattgtcgt ggacagcgag aaggacaaca tgaaactgaa cggctacttc     540

agctgtcaca actcgagatg cacgaaaatc aagagtccgt tcagctgcga tcgttattgt     600

cctgatatcg tcacgtcgga cgtgaacgtg ttccttatgc aagatgacaa catcgtcaca     660

gtgaaatgcg aacgtggcct ggcgctgaac aaagcgaatg gaaatctgcc aggcgtcagg     720

ctaaccacgc ctcaccaatt ttgggaggat cgaaacggga gcattattgt tagctgtttg     780

gcggtggaca agaagatgaa cgatgtaagg acacaggact gcgtgaacgg caccttgtta     840

aaggagatcc cattacctca accaaccatc aacttcacga gttttctgaa catatacgag     900

aagagcttgc aatatccagt ggatcctacg aatatctatg tacctgcgca acgatccctc     960
```

```
acgatctaca acagttctcg cctctacatc aatttcgagg gttgcgtcaa taccctaaaa    1020

ggggaatgta aagatttcct cgccactcac ggtcgagatg gcgacaatca gacggcgcag    1080

agtagatacc cctgttacta caacaagaac aactctctgt tagtcgtcgc acgtttcgac    1140

ctgaacaaaa cgcggacgga attgctgatc gccatcatcg tgccgtccgg cctgttcgtc    1200

atcagtctta ccaccctggt cgtgataacg cgatcggttc aggtgggcga cgacgcgaag    1260

atgcgttgcc gctactgcgt ggacaagcaa gaagttgagg gtgaggacga agggctcgtc    1320

gaggccacgc cgtcctcgtc tcaaggccag atgaacgaga acgagatcaa aagcatggcg    1380

ctttag                                                                1386


<210> 12
<211> 461
<212> PRT
<213> Apis mellifera

<220>
<223> Teh4 subunit

<400> 12
Met Gly Arg Lys His Lys Arg Arg Leu Ile Pro Glu Gln Asp Arg Arg
1               5                   10                  15
Ile Cys Gly Ser Ile Cys Phe Cys Gln Phe Thr Ile Val Ile Ser Cys
            20                  25                  30
Val Ala Leu Val Tyr Leu Ser Val Ala Ile Tyr Met Pro Ser His Arg
        35                  40                  45
Ala Phe His Ala Gly Ile Asp Pro Asp Pro Val Met Cys Gln Thr Val
    50                  55                  60
Asn Thr Thr Leu Thr Asn Asn Cys Gly Trp Ala Ser Cys Gly Glu Trp
65                  70                  75                  80
Cys Leu Thr Arg Thr Thr Gly Phe Cys Pro Gln Ile His Ala Thr Val
                85                  90                  95
Arg Arg Asn Gly Thr Asp Ile Val Phe Glu Asn Cys Thr Lys Phe Asn
            100                 105                 110
Ser Ile Ser Cys Pro Gln Val Asn Thr Ala Ser Leu Lys Lys Tyr Asn
        115                 120                 125
Cys Asn Asn Gly Ser Glu Cys Ser Val Leu Ser Gly Leu Phe Asn Cys
    130                 135                 140
Ser Leu Gly His Cys Val Asn Ile Ser Glu Leu Met Leu Cys His Tyr
145                 150                 155                 160
Lys Ala Asp Gly Ile Val Val Asp Ser Glu Lys Asp Asn Met Lys Leu
                165                 170                 175
Asn Gly Tyr Phe Ser Cys His Asn Ser Arg Cys Thr Lys Ile Lys Ser
            180                 185                 190
Pro Phe Ser Cys Asp Arg Tyr Cys Pro Asp Ile Val Thr Ser Asp Val
        195                 200                 205
Asn Val Phe Leu Met Gln Asp Asp Asn Ile Val Thr Val Lys Cys Glu
    210                 215                 220
Arg Gly Leu Ala Leu Asn Lys Ala Asn Gly Asn Leu Pro Gly Val Arg
225                 230                 235                 240
Leu Thr Thr Pro His Gln Phe Trp Glu Asp Arg Asn Gly Ser Ile Ile
                245                 250                 255
Val Ser Cys Leu Ala Val Asp Lys Lys Met Asn Asp Val Arg Thr Gln
            260                 265                 270
Asp Cys Val Asn Gly Thr Leu Leu Lys Glu Ile Pro Leu Pro Gln Pro
        275                 280                 285
Thr Ile Asn Phe Thr Ser Phe Leu Asn Ile Tyr Glu Lys Ser Leu Gln
```

```
    290                 295                 300
Tyr Pro Val Asp Pro Thr Asn Ile Tyr Val Pro Ala Gln Arg Ser Leu
305                 310                 315                 320
Thr Ile Tyr Asn Ser Ser Arg Leu Tyr Ile Asn Phe Glu Gly Cys Val
                325                 330                 335
Asn Thr Leu Lys Gly Glu Cys Lys Asp Phe Leu Ala Thr His Gly Arg
            340                 345                 350
Asp Gly Asp Asn Gln Thr Ala Gln Ser Arg Tyr Pro Cys Tyr Tyr Asn
        355                 360                 365
Lys Asn Asn Ser Leu Leu Val Val Ala Arg Phe Asp Leu Asn Lys Thr
    370                 375                 380
Arg Thr Glu Leu Leu Ile Ala Ile Ile Val Pro Ser Gly Leu Phe Val
385                 390                 395                 400
Ile Ser Leu Thr Thr Leu Val Val Ile Thr Arg Ser Val Gln Val Gly
                405                 410                 415
Asp Asp Ala Lys Met Arg Cys Arg Tyr Cys Val Asp Lys Gln Glu Val
            420                 425                 430
Glu Gly Glu Asp Glu Gly Leu Val Glu Ala Thr Pro Ser Ser Ser Gln
        435                 440                 445
Gly Gln Met Asn Glu Asn Glu Ile Lys Ser Met Ala Leu
    450                 455                 460
```

```
<210> 13
<211> 21
<212> DNA
<213> Artificial Sequence

<220>
<221> source
<222> 1..21
<223> /mol_type="unassigned DNA"
      /note="Fwd â\200\223 para"
      /organism="Artificial Sequence"

<400> 13
gcagatggcg aactcgatgc g                                                21


<210> 14
<211> 26
<212> DNA
<213> Artificial Sequence

<220>
<221> source
<222> 1..26
<223> /mol_type="unassigned DNA"
      /note="Rev â\200\223 para "
      /organism="Artificial Sequence"

<400> 14
cgatggcatc gttcatgatt tgaatc                                           26


<210> 15
<211> 24
<212> DNA
<213> Artificial Sequence

<220>
<221> source
<222> 1..24
<223> /mol_type="unassigned DNA"
```

/note="Fwd â\200\223 TipE "
/organism="Artificial Sequence"

<400> 15
atggcggagg aaaaggagaa gcag 24

<210> 16
<211> 19
<212> DNA
<213> Artificial Sequence

<220>
<221> source
<222> 1..19
<223> /mol_type="unassigned DNA"
/note="Rev â\200\223 TipE"
/organism="Artificial Sequence"

<400> 16
tcagactgcc gccgtcggc 19

<210> 17
<211> 19
<212> DNA
<213> Artificial Sequence

<220>
<221> source
<222> 1..19
<223> /mol_type="unassigned DNA"
/note="Fwd â\200\223 Teh1 "
/organism="Artificial Sequence"

<400> 17
atgcgcggga gcagctctg 19

<210> 18
<211> 23
<212> DNA
<213> Artificial Sequence

<220>
<221> source
<222> 1..23
<223> /mol_type="unassigned DNA"
/note="Rev â\200\223 Teh1 "
/organism="Artificial Sequence"

<400> 18
ttacctgaga tcgttgcccc tgc 23

<210> 19
<211> 21
<212> DNA
<213> Artificial Sequence

<220>
<221> source
<222> 1..21

<223> /mol_type="unassigned DNA"
/note="Fwd â\200\223 Teh2"
/organism="Artificial Sequence"

<400> 19
atgggcgccc aggaagagga c 21

<210> 20
<211> 27
<212> DNA
<213> Artificial Sequence

<220>
<221> source
<222> 1..27
<223> /mol_type="unassigned DNA"
/note="Rev â\200\223 Teh2"
/organism="Artificial Sequence"

<400> 20
ttactcttcc ttccttgcgt acttgtc 27

<210> 21
<211> 24
<212> DNA
<213> Artificial Sequence

<220>
<221> source
<222> 1..24
<223> /mol_type="unassigned DNA"
/note="Fwd â\200\223 Teh3"
/organism="Artificial Sequence"

<400> 21
atgcctaagc aagtgccagt ggag 24

<210> 22
<211> 23
<212> DNA
<213> Artificial Sequence

<220>
<221> source
<222> 1..23
<223> /mol_type="unassigned DNA"
/note="Rev â\200\223 Teh3 "
/organism="Artificial Sequence"

<400> 22
tcagagcgcc atagcggagt ttc 23

<210> 23
<211> 23
<212> DNA
<213> Artificial Sequence

<220>
<221> source

<222> 1..23
<223> /mol_type="unassigned DNA"
/note="Fwd â\200\223 Teh4 "
/organism="Artificial Sequence"

<400> 23
atgggtcgaa agcataaacg ccg 23

<210> 24
<211> 24
<212> DNA
<213> Artificial Sequence

<220>
<221> source
<222> 1..24
<223> /mol_type="unassigned DNA"
/note="Rev â\200\223 Teh4"
/organism="Artificial Sequence"

<400> 24
ctaaagcgcc atgcttttga tctc 24

<210> 25
<211> 39
<212> DNA
<213> Artificial Sequence

<220>
<221> source
<222> 1..39
<223> /mol_type="unassigned DNA"
/note="Fwd â\200\223 para #1 with Kpn1 restriction site"
/organism="Artificial Sequence"

<400> 25
gagaggtacc gccgccacca tgtccgaaga ttctgactc 39

<210> 26
<211> 40
<212> DNA
<213> Artificial Sequence

<220>
<221> source
<222> 1..40
<223> /mol_type="unassigned DNA"
/note="Rev para #1"
/organism="Artificial Sequence"

<400> 26
gccagcgcca cagagcgacg ctacgaagtt aatgagtgac 40

<210> 27
<211> 40
<212> DNA
<213> Artificial Sequence

<220>

```
<221> source
<222> 1..40
<223> /mol_type="unassigned DNA"
      /note="Fwd â\200\223 para #2"
      /organism="Artificial Sequence"

<400> 27
gtcactcatt aacttcgtag cgtcgctctg tggcgctggc                          40


<210> 28
<211> 30
<212> DNA
<213> Artificial Sequence

<220>
<221> source
<222> 1..30
<223> /mol_type="unassigned DNA"
      /note="Rev â\200\223 para #2 with Xma1 restriction site"
      /organism="Artificial Sequence"

<400> 28
gagacccggg tcagacgtcc gcggttctcg                                     30


<210> 29
<211> 40
<212> DNA
<213> Artificial Sequence

<220>
<221> source
<222> 1..40
<223> /mol_type="unassigned DNA"
      /note="Fwd - TipE with BamH1 restriction site"
      /organism="Artificial Sequence"

<400> 29
gagaggatcc gccgccacca tggcggagga aaaggagaag                          40


<210> 30
<211> 28
<212> DNA
<213> Artificial Sequence

<220>
<221> source
<222> 1..28
<223> /mol_type="unassigned DNA"
      /note="Rev - TipE with HindIII restriction site"
      /organism="Artificial Sequence"

<400> 30
gagaaagctt gcttcagact gccgccgt                                       28


<210> 31
<211> 39
<212> DNA
<213> Artificial Sequence
```

```
<220>
<221> source
<222> 1..39
<223> /mol_type="unassigned DNA"
      /note="Fwd â\200\223 Teh1 with BamH1 restriction site"
      /organism="Artificial Sequence"

<400> 31
gagaggatcc gccgccacca tgcgcgggag cagctctga                      39


<210> 32
<211> 32
<212> DNA
<213> Artificial Sequence

<220>
<221> source
<222> 1..32
<223> /mol_type="unassigned DNA"
      /note="Rev - Teh1 with EcoR1 restriction site"
      /organism="Artificial Sequence"

<400> 32
gagagaattc ttacctgaga tcgttgcccc tg                             32


<210> 33
<211> 37
<212> DNA
<213> Artificial Sequence

<220>
<221> source
<222> 1..37
<223> /mol_type="unassigned DNA"
      /note="Fwd â\200\223 Teh2 with BamH1 restriction site"
      /organism="Artificial Sequence"

<400> 33
gagaggatcc gccgccacca tgggcgccca ggaagag                        37


<210> 34
<211> 36
<212> DNA
<213> Artificial Sequence

<220>
<221> source
<222> 1..36
<223> /mol_type="unassigned DNA"
      /note="Rev â\200\223 Teh2 with HindIII restriction site"
      /organism="Artificial Sequence"

<400> 34
gagaaagctt ttactcttcc ttccttgcgt acttgt                         36


<210> 35
<211> 40
<212> DNA
<213> Artificial Sequence
```

<220>
<221> source
<222> 1..40
<223> /mol_type="unassigned DNA"
/note="Fwd â\200\223 Teh3 with BamH1 restriction site"
/organism="Artificial Sequence"

<400> 35
gagaggatcc gccgccacca tgcctaagca agtgccagtg 40

<210> 36
<211> 30
<212> DNA
<213> Artificial Sequence

<220>
<221> source
<222> 1..30
<223> /mol_type="unassigned DNA"
/note="Rev â\200\223 Teh3 with EcoR1 restriction site"
/organism="Artificial Sequence"

<400> 36
gagagaattc tcagagcgcc atagcggagt 30

<210> 37
<211> 39
<212> DNA
<213> Artificial Sequence

<220>
<221> source
<222> 1..39
<223> /mol_type="unassigned DNA"
/note="Fwd â\200\223 Teh4 with EcoR1 restriction site"
/organism="Artificial Sequence"

<400> 37
gagagaattc gccgccacca tgggtcgaaa gcataaacg 39

<210> 38
<211> 28
<212> DNA
<213> Artificial Sequence

<220>
<221> source
<222> 1..28
<223> /mol_type="unassigned DNA"
/note="Rev â\200\223 Teh4 with HindIII restriction site"
/organism="Artificial Sequence"

<400> 38
gagaaagctt ctgctaaagc gccatgct 28

## Description

### FIELD OF INVENTION

The present invention relates to the cloning of the sodium channel Para and its regulatory subunits of a pollinator insect such as *Apis mellifera* and its uses thereof.

### BACKGROUND OF INVENTION

During the last decades, agriculture has changed to meet the increasing demand to produce food. There has been expansion of cultivated areas in monoculture and increases use of pesticides. The abusive use of pesticides is subjecting pollinator insects to stress as evidenced by a constant decrease in the density of these pollinator insects around agricultural fields in many parts of the world, thus causing economic losses. Pollinators are crucial for the pollination of agricultural crops and natural areas around the world.

Among these pollinator insects, some insects issued from the order of *hymenoptera* or bees are considered excellent pollinating insects in agro-ecosystems because they visit many flowers on the same day. Among these pollinator insects, Bumble bees for instance have already been red-listed and are in danger of extinction.

Another bee such as *Apis mellifera* or honey bee is interesting from an economic perspective because it provides products of great value, such as honey, propolis, royal jelly, wax, and apitoxin (bee venom).

The decline of the bee colonies and pollinator insects is not only due to the increase of their mortality but also modifications related to their memory or social behavior within the hives (Suchail S et al. 2001 Environ Toxicol Chem 20 (11):2482-6; Gels JA 2002 J Econ Entomol; 95(4):722-8). Some toxicity assays have already been developed by oral or topical application (OECD guidelines 2013 for the testing of chemicals OECD237) of a substance on the insect however these assays are not reproducible and are not adapted to the test of a large diversity of compounds. Consequently there is a need to develop high throughput assays highly reproducible and *in vitro* to determine whether a compound is toxic for pollinator insects.

Sodium channels are integral transmembrane proteins responsible for the rapidly rising phase of action potentials, and they are critical for electrical signaling in most excitable cells. In response to membrane depolarization, sodium channels open and allow sodium ions to flow into the cell, thereby depolarizing the membrane potential.

Because of their crucial role in membrane excitability, sodium channels are the target site of a great variety of neurotoxins, such as tetrodotoxin, scorpion toxins, and batrachotoxin, which are produced by plants and animals for defense or predation (Cestele and Catterall 2000 Biochimie; 82:883-892; Wang SY and Wang GK Cell Signal 2003; 15:151-159). Insecticidal pyrethrins, found in extracts of the flowers of *Chrysanthemum* species, also act on sodium channels (Soderlung 2010 Pestic Biochem Physiol. Jun 1;97(2):78-86). Sodium channels are also the primary target of dichlorodiphenyltrichloroethane (DDT) and modern synthetic pyrethroids, which are structural derivatives of the naturally occurring pyrethrins (Narahashi T.J Pharmacol Exp Ther. 2000 Jul; 294(1):1-26). Furthermore, recent evidence indicates that a new class of pyrazoline-like insecticides, oxadiazines, also target sodium channels (Silver and Soderlund Pestic Biochem Physiol. 2005; 81:136-143; Dong K 2007 Invert Neuroscience 7(1): 17-30).

The present application indeed unravels the yet unknown function of nucleic acid sequences and protein sequences of sodium channel from pollinator insects whose activity is modulated by some toxic compounds. Consequently, the present application discloses a method using the nucleic acid sodium channel and its regulatory subunits of pollinator insects to analyze the effect of compounds on sodium channel activity of a pollinator insect.

### SUMMARY

One object of the invention is a nucleic acid sequence of the regulatory subunit TipE as set forth in SEQ ID NO: 3 or variant thereof consisting of a nucleic acid sequence of less than 1500 bp and having at least 90% identity with SEQ ID NO: 3.

Another object of the invention is a nucleic acid sequence of the regulatory subunit Teh1 as set forth in SEQ ID NO: 5 or variant thereof consisting of a nucleic acid sequence having more than 99% identity with SEQ ID NO: 5.

Another object of the invention is a nucleic acid sequence of the regulatory subunit Teh2 as set forth in SEQ ID NO: 7 or variant thereof consisting of a nucleic acid sequence of less than 1000 bp and having at least 90% identity with SEQ ID NO: 7.

Another object of the invention is a nucleic acid sequence of the regulatory subunit Teh3 as set forth in SEQ ID NO: 9 or variant thereof consisting of a nucleic acid sequence of less than 3000 bp and having at least 90% identity with SEQ ID NO: 9.

Another object of the invention is a nucleic acid sequence of the regulatory subunit Teh4 as set forth in SEQ ID NO: 11 or variant thereof consisting of a nucleic acid sequence of less than 2500 bp and having at least 90% identity with SEQ ID NO: 11.

Another object of the invention is a vector comprising at least one nucleic acid sequence as described above.

Another object of the invention is a modified cell expressing at least one vector as described above.

Another object of the invention is a sodium channel of a pollinator insect comprising a Para subunit having a nucleic acid sequence as set forth in SEQ ID NO: 1 or variant thereof having at least 90% identity with SEQ ID NO: 1.

In one embodiment, the sodium channel further comprises at least one regulatory subunit of said sodium channel, wherein said regulatory subunit is TipE, Teh1, Teh2, Teh3 and/or Teh4 having the sequences as described above.

Another object of the invention is a vector comprising the sodium channel as described above.

Another object of the invention is a modified cell expressing a sodium channel as described above or a vector as described above.

In one embodiment, the modified cell is an oocyte of Xenopus.

Another object of the invention is an *in vitro* method to determine the effect of a test compound on the modulation of activity of a sodium channel of a pollinator insect comprising:

a. contacting modified cells as described above with a test compound,
b. measuring the effect of said test compound on the sodium channel activity, and

comparing said effect to the effect of a standard reference or a vehicle solution, thereby determining a modulation of activity of said sodium channel.

In one embodiment, the method is for determining the toxicity of a test compound on a pollinator insect.

Another object of the invention is an *in vitro* method for screening compounds that modulate the sodium channel activity of pollinator insects comprising:

a. contacting modified cells as described above with said compound,
b. measuring the effect of said compound on the sodium channel activity, and

comparing said effect to the effect of a standard reference or a vehicle solution, thereby determining a modulation of activity of said sodium channel.

Another object of the invention is kit comprising at least one vector as described above or a modified cell as described above and reagents.

**DEFINITIONS**

In the present invention, the following terms have the following meanings:

- **"Nucleic acid sequence"** refers to encompass nucleic acids having the sequences set forth above as well as variants thereof including for example fragments, deletions, insertions and substitutions that maintain the ability to encode the different subunits of the sodium channel of pollinator insects.

- **"Functional sodium channel", "functional expression"** refers to the synthesis and any necessary post-translational processing of a sodium channel molecule and/or at least one of its regulatory subunit in a modified cell so that the channel or its regulatory subunit is inserted properly in the cell membrane and is capable of conducting sodium ions in response to an experimentally-imposed change in the cell membrane potential or upon exposure to appropriate pharmacological agents.

- **"Phytosanitary product"** refers to biological or chemical compounds used as insecticides, herbicides, fungicides, acaricides, fertilizers, antibiotics, or any products used in agriculture, in wine-making, in food storage, ship hulls, for animals or domestic purposes.

- **"Sub-lethal doses":** refer to a concentration of a potentially lethal test compound that is not high enough to cause death meaning a concentration under the median lethal dose (LD50) but still able to trigger death by a mechanism which is not acute (immediate) death. At sub-lethal dose, the test compound induces changes in biological mechanisms that include but are not limited to: colony level behavioral changes, individual level behavioral changes, memory changes, cellular mechanisms or molecular mechanisms changes.

- **"Minimal modulation doses"** refer to the lowest concentration of a test compound required to modulate the sodium channel activity.

- **"Test compound"** refers to a phytosanitary product, a molecule, an organism or extract thereof eventually able to bind and/or modulate the sodium channel activity of a pollinator insect.

- **"Derivative"** or **"analog"** of a compound refers broadly to the modification or substitution of one or more chemical moieties on a parent compound and may include functional derivatives, positional isomers, tautomers, zwitterions, enantiomers, diastereomers, racemates, isosteres or stereochemical mixtures thereof.

- **"Modulation", "modulating"** or **"modulator"** of sodium channel activity refers to the three distinct states that can be modulated and in which a $Na^+$ channel can be configured: closed, opened and inactivated. The transition between the closed to the open state refers to the activation state, the transition from an open to a closed state refers to the deactivation, the transition between an open state and inactivated state refers to the inactivation. Finally, the transition between the inactivated to open states or inactivated to closed state refers to a reactivation process. It also refers to compounds including **"sodium channel blockers", "sodium channel activators", "gating modifiers"** and **"sodium regulators"** affect the current flow within the $Na^+$ channel by inducing modification(s) of conformation on said sodium channel, subunits assembly or organization, modification of the targeting of the channels to the plasma membrane, modification of the life time of the said sodium channel to the plasma membrane, or blocking the channel gate or any modification of post-translational state like phosphorylation state of the said sodium channel.

- **"Sodium channel blockers"** refer to compounds that impair conduction of $Na^+$ ions through sodium channels. Extracellular $Na^+$ channel blockers block sodium channels by binding to and occluding the extracellular pore opening of the channel. Intracellular $Na^+$ channel blockers block the intracellular side of the channel.

- **"Sodium channel activators"** refer to compounds that persistently activate (open) sodium channels.

- **"Gating modifiers"** refer to compounds that modify the gating of $Na^+$ channels.

- **"Sodium channels regulators"** refer to any compounds that modify the targeting of the sodium channel to its effective site or modify the stability of the compound to its effective site.

## DETAILED DESCRIPTION

One object of the present invention is the isolated nucleic acid sequence or a variant thereof comprising at least one regulatory subunit of the sodium ($Na^+$) channel of a pollinator insect.

Pollinator insects of the invention include species from the order *hymenoptera*, the family *Apidae* and the subfamily *Apinae* particularly the non-invasive species that does not damage crops or parasite pollinator insects or damage hives.

Pollinator insects of the invention include in a non-limiting list: bees, honeybees such as *Apis mellifera, Apis cerana*, *Apis dorsata, Apis florea,* stingless bees such as *Melipona beecheii* or *Melipona yucatanica, Melipona quadrifasciata anthidioides*, orchid bees, bumble bees such as *Bombus franklini*, *Bombus terricola, Bombus affinis* and *Bombus occidentalis*. Preferably, the pollinator insect of the invention is a honey bee, and most preferably *Apis mellifera.*

In one embodiment of the invention, the isolated nucleic acid sequence comprises TipE as set forth in SEQ ID NO: 3 or a variant thereof consisting of a nucleic acid sequence of less than 1500 base pairs (bp) and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 3.

In one embodiment, a variation may occur at positions 67, 76, 202-216, 344, 348, 435, 492 of SEQ ID NO: 3.

In one embodiment of the invention, the isolated nucleic acid sequence comprises Teh1 as set forth in SEQ ID NO: 5 or a variant thereof having more than 99, 99.1; 99.2; 99.3; 99.4; 99.5; 99.6; 99.7; 99.8; 99.9% of identity with SEQ ID NO: 5.

In one embodiment, a variation may occur at positions 231, 285, 777 of SEQ ID NO: 5.

In one embodiment of the invention, the isolated nucleic acid sequence comprises Teh2 as set forth in SEQ ID NO: 7 or a variant thereof consisting of a nucleic acid sequence of less than 1000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 7.

In one embodiment, a variation may occur at positions 420, 453, 468, 546, 549 of SEQ ID NO: 7.

In one embodiment of the invention, the isolated nucleic acid sequence comprises Teh3 as set forth in SEQ ID NO: 9 or a variant thereof consisting of a nucleic acid sequence of less than 3000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 9.

In one embodiment, a variation may occur at positions 63, 78, 81, 234, 292, 300, 435, 441, 561, 567, 576, 651, 684, 696, 891, 936, 948, and 1050 of SEQ ID NO: 9.

In one embodiment of the invention, the isolated nucleic acid sequence comprises Teh4 as set forth in SEQ ID NO: 11 or a variant thereof consisting of a nucleic acid sequence of less than 2500 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 11.

In one embodiment, a variation may occur at positions 504, 678, 684, 685, 711, 789, 1089, 1219, 1314 of SEQ ID NO: 11.

Another object of the invention is a sodium channel comprising a Para subunit of a pollinator insect having an isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In one embodiment, the sodium channel of the invention comprising a Para subunit of a pollinator insect as described here above, further comprises at least one regulatory subunit of said sodium channel as described here above.

In one embodiment, the sodium channel of the invention comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence TipE as set forth in SEQ ID NO: 3 or a variant thereof consisting of a nucleic acid sequence of less than 1500 base pairs (bp) and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 3.

In another embodiment, the sodium channel of the invention comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh1 as set forth in SEQ ID NO: 5 or a variant thereof having more than 99, 99.1; 99.2; 99.3; 99.4; 99.5; 99.6; 99.7; 99.8; 99.9% of identity with SEQ ID NO: 5.

In another embodiment, the sodium channel of the invention comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh2 as set forth in SEQ ID NO: 7 or a variant thereof consisting of a nucleic acid sequence of less than 1000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 7.

In another embodiment, the sodium channel of the invention comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh3 as set forth in SEQ ID NO: 9 or a variant thereof consisting of a nucleic acid sequence of less than 3000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 9.

In another embodiment, the sodium channel of the invention comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh4 as set forth in SEQ ID NO: 11 or a variant thereof consisting of a nucleic acid sequence of less than 2500 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 11.

Another object of the invention is an expression vector comprising at least one nucleic acid sequence encoding at least one regulatory subunit of the sodium channel of a pollinator insect and able to express said regulatory subunit of said sodium channel in a suitable modified cell.

In one embodiment of the invention, the vector comprises the nucleic acid sequence TipE as set forth in SEQ ID NO: 3 or a variant thereof consisting of a nucleic acid sequence of less than 1500 base pairs (bp) and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 3.

In another embodiment of the invention, the vector comprises the nucleic acid sequence Teh1 as set forth in SEQ ID NO: 5 or a variant thereof having more than 99, 99.1; 99.2; 99.3; 99.4; 99.5; 99.6; 99.7; 99.8; 99.9% of identity with SEQ ID NO: 5.

In another embodiment of the invention, the vector comprises the nucleic acid sequence Teh2 as set forth in SEQ ID NO: 7 or a variant thereof consisting of a nucleic acid sequence of less than 1000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 7.

In another embodiment of the invention, the vector comprises the nucleic acid sequence Teh3 as set forth in SEQ ID NO: 9 or a variant thereof consisting of a nucleic acid sequence of less than 3000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 9.

In another embodiment of the invention, the vector comprises the nucleic acid sequence Teh4 as set forth in SEQ ID NO: 11 or a variant thereof consisting of a nucleic acid sequence of less than 2500 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 11.

Another object of the invention is an expression vector comprising the isolated nucleic acid sequence of Para and at least one nucleic acid sequence encoding a regulatory subunit of the sodium channel of a pollinator insect and able to express said regulatory subunit of said sodium channel in a suitable modified cell.

In one embodiment of the invention, the expression vector comprises the isolated nucleic acid sequence of Para as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In another embodiment, the expression vector comprises the isolated nucleic acid sequence of Para as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence TipE as set forth in SEQ ID NO: 3 or a variant thereof consisting of a nucleic acid sequence of less than 1500 base pairs (bp) and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 3.

In another embodiment, the expression vector comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh1 as set forth in SEQ ID NO: 5 or a variant thereof having more than 99, 99.1; 99.2; 99.3; 99.4; 99.5; 99.6; 99.7; 99.8; 99.9% of identity with SEQ ID NO: 5.

In another embodiment, the expression vector comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh2 as set forth in SEQ ID NO: 7 or a variant thereof consisting of a nucleic acid sequence of less than 1000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 7.

In another embodiment, the expression vector comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh3 as set forth in SEQ ID NO: 9 or a variant thereof consisting of a nucleic acid sequence of less than 3000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 9.

In another embodiment, the expression vector comprises the isolated nucleic acid sequence as set forth in SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh4 as set forth in SEQ ID NO: 11 or a variant thereof consisting of a nucleic acid sequence of less than 2500 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 11.

Expression vectors can be constructed by well-known molecular biological methods as described for example in Sambrook et al. (1989) Molecular Cloning: A Laboratory Manual Cold Spring Harbor Laboratory Press, Cold Spring Harbor, N.Y., or any of a myriad of laboratory manuals on recombinant DNA technology that are widely available. Expression vectors into which the nucleic acids of the present invention can be cloned under the control of a suitable promoter are also commercially available. Recombinant viral vectors, including retroviral, baculoviral, parvoviral and densoviral vectors are particularly preferred.

In one embodiment the expression vector comprises a strong constitutive or inducible promoter operably linked to the nucleic acid encoding the regulatory subunit of the sodium channel. Suitable promoters are well known and readily available to those of ordinary skill in the art, and include for example, the heat shock promoter, metallothionein promoter, dexamethasone promoter, alcohol dehydrogenase promoter, and the baculovirus promoters, i.e., the early promoter (e.g., IE-1 and etl), the late promoters (e.g., vp39 and p6.9), the very late promoters (e.g., polh and p10) and the hybrid promoter (e.g., vp39/polh).

Another object of the invention is a modified cell that expresses at least one vector described here above.

Modified cells as used herein refers to eukaryote cell. Modified cells are well known in the state of the art for genetic engineering. These cells include in a non-limiting list: prokaryotic cells, eukaryotic cells, in particular bacteria such as *Escherichia coli, Bacillus sp.,* or yeasts such as *Saccharomyces cerevisiae,* fungus such as *Aspergillus niger,* insect cells such as SF9, SL1, or mammalian cells such as CHO, HEK293, PER-C6, amphibians cells such as for example oocytes of Xenopus.

Technics for genetic engineering as used in the present application are well known by the person skilled in the art. These technics are described in Guide to Molecular Cloning Technics (Editors Berger SL and Kimmel AR 1987 Methods in Enzymology 152: 359-371).

Depending on the cell to be modified, the person skilled in the art can determine the technology needed for the introduction of the nucleotide sequences of the present application in the selected modified cell and the vector used.

Techniques for introducing the nucleic acid molecules into the modified cells may involve the use of expression vectors which comprise the nucleic acid molecules. These expression vectors (such as plasmids and viruses; viruses including bacteriophage) can then be used to introduce the nucleic acid molecules into suitable modified cells. For example, sodium channel expression can be studied in Xenopus oocytes. DNA encoding the sodium channel of a pollinator insect can be injected into the oocyte nucleus or transformed into the oocyte using a suitable vector, or mRNA encoding the said sodium channel can be injected directly into the oocyte, in order to obtain expression of a functional sodium channel in the oocyte.

Various methods are known in the art for introducing nucleic acid molecules into modified cells. One method is microinjection, in which DNA is injected directly into the nucleus of cells through fine glass needles (or RNA is injected directly into the cytoplasm of cells). Alternatively, DNA can be incubated with an inert carbohydrate polymer (dextran) to which a positively charged chemical group (DEAE, for diethylaminoethyl) has been coupled. The DNA sticks to the DEAE-dextran via its negatively charged phosphate groups. These large DNA-containing particles stick in turn to the surfaces of cells, which are thought to take them in by a process known as endocytosis. Some of the DNA evades destruction in the cytoplasm of the cell and escapes to the nucleus, where it can be transcribed into RNA like any other gene in the cell. In another method, cells efficiently take in DNA in the form of a precipitate with calcium phosphate. In electroporation, cells are placed in a solution containing DNA and subjected to a brief electrical shock that causes holes to open transiently in their membranes. DNA enters through the holes directly into the cytoplasm, bypassing the endocytotic vesicles through which they pass in the DEAE-dextran and calcium phosphate procedures (passage through these vesicles may sometimes destroy or damage DNA). DNA can also be incorporated into artificial lipid vesicles, liposomes, which fuse with the cell membrane, delivering their contents directly into the cytoplasm. In an even more direct approach, used primarily with plant cells and tissues, DNA is absorbed to the surface of tungsten microprojectiles and fired into cells with a device resembling a shotgun.

Several methods of microinjection, electroporation, and liposome fusion, have been adapted to introduce nucleic acids into cells and are well known by the skilled artisan. Further methods for introducing nucleic acid molecules into cells involve the use of viral vectors. Since viral growth depends on the ability to get the viral genome into cells, viruses have devised clever and efficient methods for doing it. One such virus widely used for protein production is an insect virus, baculovirus. Baculovirus attracted the attention of researchers because during infection, it produces one of its structural proteins (the coat protein) to spectacular levels. If a foreign gene were to be substituted for this viral gene, it too ought to be produced at high level. Baculovirus, like vaccinia, is very large, and therefore foreign genes must be placed in the viral genome by recombination. To express a foreign gene in baculovirus, the gene of interest is cloned in place of the viral coat protein gene in a plasmid carrying a small portion of the viral genome. The recombinant plasmid is cotransfected into insect cells with wild-type baculovirus DNA. At a low frequency, the plasmid and viral DNAs recombine through homologous sequences, resulting in the insertion of the foreign gene into the viral genome. Virus plaques develop, and the plaques containing recombinant virus look different because they lack the coat protein. The plaques with recombinant virus are picked and expanded. This virus stock is then used to infect a fresh culture of insect cells, resulting in high expression of the foreign protein. Various viral vectors have also been used to transform cells, such as bacteriophage, vaccinia virus, adenovirus, and retrovirus.

Another object of the invention is a modified cell expressing a vector as described here above.

In one embodiment of the invention, the modified cell comprises an expression vector comprising the nucleic acid sequence of SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In another embodiment of the invention, the modified cell comprises an expression vector comprising the nucleic acid sequence of SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the nucleic acid sequence of TipE as set forth in SEQ ID NO: 3 or a variant thereof consisting of a nucleic acid sequence of less than 1500 base pairs (bp) and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 3.

In another embodiment of the invention, the modified cell comprises an expression vector comprising the nucleic acid sequence of SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh1 as set forth in SEQ ID NO: 5 or a variant thereof having more than 99, 99.1; 99.2; 99.3; 99.4; 99.5; 99.6; 99.7; 99.8; 99.9% of identity with SEQ ID NO: 5.

In another embodiment of the invention, the modified cell comprises an expression vector comprising the nucleic acid sequence of SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh2 as set forth in SEQ ID NO: 7 or a variant thereof consisting of a nucleic acid sequence of less than 1000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 7.

In another embodiment of the invention, the modified cell comprises an expression vector comprising the nucleic acid sequence of SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh3 as set forth in SEQ ID NO: 9 or a variant thereof consisting of a nucleic acid sequence of less than 3000 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 9.

In another embodiment of the invention, the modified cell comprises an expression vector comprising the nucleic acid sequence of SEQ ID NO: 1 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the isolated nucleic acid sequence Teh4 as set forth in SEQ ID NO: 11 or a variant thereof consisting of a nucleic acid sequence of less than 2500 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 11.

Another object of the invention is the modified cells comprising the functional expression of at least one regulatory subunit of the sodium channel of the pollinator insect.

In one embodiment, said modified cell expresses the isolated protein sequence of TipE as set forth in SEQ ID NO: 4 or a variant thereof having at least 99, 99.1; 99.2; 99.3; 99.4; 99.5; 99.6; 99.7; 99.8; 99.9% of identity with SEQ ID NO: 4.

In another embodiment, said modified cell expresses the isolated protein sequence of Teh1 as set forth in SEQ ID NO: 6 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 6.

In another embodiment, said modified cell expresses the isolated protein sequence of Teh2 as set forth in SEQ ID NO: 8 or a variant thereof consisting of a protein sequence of less than 250 amino acids and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 8.

In another embodiment, said modified cell expresses the isolated protein sequence of Teh3 as set forth in SEQ ID NO: 10 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 10.

In another embodiment, said modified cell expresses the isolated protein sequence of Teh4 as set forth in SEQ ID NO: 12 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 12.

In one embodiment, the modified cell of the invention expresses the isolated protein sequence of the sodium channel as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2.

In another embodiment, modified cell of the invention expresses the isolated protein sequence of the sodium channel as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and at least one regulatory subunit of sodium channel as described here above.

In another embodiment, modified cell of the invention expresses the isolated protein sequence of the sodium channel as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and the isolated protein sequence of TipE as set forth in SEQ ID NO: 4 or a variant thereof having at least 99, 99.1; 99.2; 99.3; 99.4; 99.5; 99.6; 99.7; 99.8; 99.9% of identity with SEQ ID NO: 4.

In another embodiment, modified cell of the invention expresses the isolated protein sequence of the sodium channel as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and the isolated protein sequence of Teh1 as set forth in SEQ ID NO: 6 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 6.

In another embodiment, modified cell of the invention expresses the isolated protein sequence of the sodium channel as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and the isolated protein sequence of Teh2 as set forth in SEQ ID NO: 8 or a variant thereof consisting of a protein sequence of less than 250 amino acids and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 8.

In another embodiment, modified cell of the invention expresses the isolated protein sequence of the sodium channel as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and the isolated protein sequence of Teh3 as set forth in SEQ ID NO: 10 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 10.

In another embodiment, modified cell of the invention expresses the isolated protein sequence of the sodium channel as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and the isolated protein sequence of Teh4 as set forth in SEQ ID NO: 12 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 12.

Sodium channel of insects comprises a single sodium channel gene Para that constitute the pore-forming subunit. Alternative splicing and RNA editing of this gene can generate many sodium channel variants with different gating and pharmacological properties. The sodium channel Para activity is completed by regulatory subunits that modulate Para activity. These regulatory subunits can also generate variants with different properties.

In one embodiment, the modified cells expressing the sodium channel of a pollinator insect carry mutations that confer reduced sensitivity to a test compound.

Another object of the invention is an *in vitro* method to determine the effect of a test compound on the activity of a sodium channel of a pollinator insect comprising:

a. contacting modified cells described herein with a test compound,
b. measuring the effect of said test compound on the sodium channel activity, and

comparing said effect to the effect of a standard reference or a vehicle solution, thereby determining a modulating activity of said sodium channel.

Another object of the invention is an *in vitro* method to determine the effect of a test compound on the activity of a sodium channel of a pollinator insect comprising:

a. contacting a modified cell described herein with a vehicle solution,
b. inducing at least one pulse on said modified cell,
c. measuring the effect of said vehicle solution on the sodium channel activity,
d. contacting said modified cell described herein with a test compound,
e. inducing at least one pulse on said modified cell,
f. measuring the effect of said test compound on the sodium channel activity, and

comparing said effect to the effect of said vehicle solution, thereby determining a modulating activity of said sodium channel.

Another object of the invention is an *in vitro* method to determine the effect of a test compound on the activity of a sodium channel of a pollinator insect comprising:

a. contacting a modified cell described herein with a vehicle solution,
b. inducing at least one pulse on said modified cell,
c. measuring the effect of said vehicle solution on the sodium channel activity,
d. inducing at least one pulse on said modified cell,
e. contacting said modified cell described herein with a test compound,
f. measuring the effect of said test compound on the sodium channel activity, and

comparing said effect to the effect of said vehicle solution, thereby determining a modulating activity of said sodium channel.

The vehicle solution as used herein refers to the control solution, i.e. the same solution that comprises the test compound.

The standard reference as used herein refers to a compound whose effect on the sodium channel activity is well known by the skilled artisan.

In one embodiment of the invention, the standard reference is a compound known to be toxic on sodium channel of pollinator insect.

In another embodiment of the invention, the standard reference is a compound known to be toxic on sodium channel of other species such as invasive species.

Examples of these invasive species include but are not limited to: introduced species, imported bees, species from the order *hymenoptera*, the family *Vespidae* and the subfamily *Vespinae* particularly Asian predatory wasp (*Vespa velutina*), spiders, parasites, endoparasites, ectoparasites from the class *Arachnida*, the family *Varroidae* such as: *Varroa jacobsoni*, *Varroa destructor, Varroa underwoodi*, *Varroa rindereri,*

Examples of compounds used as standard reference include for example: pyrethroid or known derivatives thereof, DTT, permethrin. tetrodotoxin (TTX, a neurotoxin $Na^+$ channel antagonist from the pupper fish Fugu), veratridin (polycyclic alkaloid extracted for Veratrum album which slows the $Na^+$ channel inactivation).

The pulse as used herein refers to an electrical stimulation comprised between -120 mV and - 20 mV for 0 to several seconds, preferably 5 ms at various frequencies from 1 Hz to 100 Hz. Pulse protocols are well-known by the skilled artisan. Indeed, known protocols can be applied for measuring specifically voltage-dependent activation, steady-state of voltage-dependent inactivation, voltage-dependent reactivation/recovery from inactivation, or measuring any state dependent effect on the sodium channel.

In one embodiment, the standard reference modulates sodium channel activity.

In one embodiment, lethal dose of a test compound is added to the culture medium.

In another embodiment, sub-lethal dose of a test compound is added to the culture medium.

In another embodiment, minimal modulation dose of a test compound is added to the culture medium.

Examples of test compounds comprise phytosanitary product which include but are not limited to: insecticides, pesticides, drugs, veterinary drugs (such as veterinary drugs against parasites), herbicides, acaricids, chemical or organic fertiliser that include in a non-limiting list derivatives or analogs of: pyrethroid and derivatives (pyrethrin I and II, cinerin 1, cinerin 2, jasmolin 1, jasmolin 2, Allethrin, Bifenthrin, Cyfluthrin, Cypermethrin, Cyphenothrin, Deltamethrin, Esfenvalerate, Etofenprox, Fenpropathrin, Fenvalerate, Flucythrinate, Flumethrin, Imiprothrin, lambda-Cyhalothrin, Metofluthrin, Permethrin, Prallethrin, Resmethrin, Silafluofen, Sumithrin, tau-Fluvalinate, Tefluthrin, Tetramethrin, Tralomethrin, Transfluthrin), oxadiazines and derivatives, DCJW (N-decarbomethoxyllated JW062), and derivatives thereof.

Examples of test compounds comprise molecules which include but are not limited to: chemical compounds, new chemical entities, siRNAs, shRNAs, antisense oligonucleotide, ribozymes or aptamers, agonist or antagonist of sodium channels, antibodies or fragments thereof, diabodies modulating activity of a sodium channel of a pollinator insect. These test compounds can also include: neurotoxins such as tetrodotoxin, and batrachotoxin, toxins from plants, venom of insects, spiders, cones, mollusks, snails, vertebrates such as snakes, scorpion toxins, or any other natural products used in integrated pest management.

Examples of test compounds also comprise but are not limited to compounds already known to be toxic on other species such as invasive species as described here above.

In one embodiment of the invention, said test compound is already known to modulate the sodium channel activity of a pollinator insect.

In another embodiment of the invention, said test compound is not yet known to modulate the sodium channel activity of a pollinator insect.

In another embodiment of the invention, said test compound is a new chemical entity.

In one embodiment of the invention, said test compound increases or decreases the functional expression of sodium channel in the modified cell.

In one embodiment of the invention, said test compound induces or reduces the sodium channel expression at the cell surface.

In one embodiment, said test compound modulates sodium channel activity via TipE.

In another embodiment, said test compound modulates sodium channel activity via Teh1.

In another embodiment, said test compound modulates sodium channel activity via Teh2.

In another embodiment, said test compound modulates sodium channel activity via Teh3.

In another embodiment, said test compound modulates sodium channel activity via Teh4.

In another embodiment, said test compound modulates sodium channel activity via para and/or at least one of its regulatory subunits.

According to the method of the invention, said test compound modulates sodium ions current *via* Para and/or at least one of its regulatory subunit.

Sodium channels are critical for the initiation and propagation of action potentials in neurons and other excitable cells. In response to a sub-threshold depolarization (in this case, an action potential), the gates open, allowing positively-charged $Na^+$ ions to flow into the neuron through the channels, and causing the voltage across the neuronal membrane to move forward the $Na^+$ equilibrium potential. At the peak of the action potential, when enough $Na^+$ has entered into the neuron and the membrane's potential has become high enough, the $Na^+$ channels inactivate themselves. This causes $Na^+$ flow through the channel to stop and the $Na^+$ channel is inactivated. With the $Na^+$ channel no longer contributing to the membrane potential, the potential decreases back to its resting potential as the neuron repolarizes.

To fulfill their functional role, voltage-gated $Na^+$ channels must be able to complete their state transitions *in vivo* among open, closed and inactivated states in a few milliseconds or less.

In one embodiment, said test compound modulates the activity of the sodium channel of the invention.

In one embodiment, said test compound modify the gating kinetics of the sodium channel.

In another embodiment, said test compound modulates (increases or decreases) the amplitude of action potential of a pollinator insect sodium channel.

In another embodiment, said test compound modulates (increases or decreases) the inactivation during a depolarizing pulse.

In another embodiment, said test compound modulates (increases or decreases) the activation kinetics or voltage dependence of either channel activation and/or deactivation.

In another embodiment, said test compound modulates $Na^+$ channel activity following repetitive depolarization.

In another embodiment, said test compound induces deleterious neuronal hyperexcitability.

In another embodiment, said test compound is dependent of the functional states of the sodium channel with preferred affinity for the close and/or open and/or inactivated states.

In another embodiment, said test compound modulates (increases or decreases) the sorting, the targeting or the translocation of the sodium channel of the invention.

In another embodiment, said test compound modulates (increases or decreases) the stability of the sodium channel of the invention.

In another embodiment, said test compound modulates the subunits assembly of the sodium channel of the invention.

In one embodiment, the method of the invention is a cell-based assay.

In one embodiment, the method of the invention is a high-throughput assay.

In another embodiment, the method of the invention is an electrophysiological method.

In another embodiment, the method of the invention is a fluorometry or luminometry method.

The methods of the invention can be in conventional laboratory format or adapted for high-throughput. The term "high throughput screening" (HTS) refers to an assay design that allows easy analysis of multiple samples simultaneously, and capacity for robotic manipulation. Another desired feature of high throughput assays is an assay design that is optimized to reduce reagent usage, or minimize the number of manipulations in order to achieve the analysis desired.

Methods for measuring the effect of a test compound on a sodium channel are well known in the state of the art. For example, the person skilled in the art knows that electrophysiological measurements in modified cells expressing functional sodium channel can be used to test a compound.

Sodium transport can for example be monitored by pre-incubating cells in a medium containing one or more chemical compound, adding a medium containing radiosodium ($^{22}Na^+$), incubating the cells further in this medium, and isolating cells by filtration. Sodium transport is detected by the measurement of $^{22}Na^+$ within the cells by liquid scintillation counting or other radiometric techniques (Bloomquist and Soderlund 1988 Mol Pharmacol 33:543-550). Alternatively, [$^{14}C$]guanidinium ion can be employed as the radiotracer in the place of sodium using the same procedure (Jacques et al. 1978).

In another embodiment, the function of the sodium channel can be evaluated by pre-incubating cells to equilibrium with a sodium-selective fluorescent chelating agent (e.g., SBFI [sodium-binding benzofuran isophthalate]), washing the cells, exposing the cells to a test compound, and monitoring the increase in intracellular sodium by measuring the fluorescence of the SBFI-sodium complex (Deri and Adam-Vizi 1993 J Neurochem 61:818-825).

The method of the invention as in Connolly et al. (1990 Biosens. Biores. 5:223-234), displays a planar array of microelectrodes developed for monitoring the electrical activity of cells in culture. The device allows the incorporation of surface topographical features in an insulating layer above the electrodes. Semiconductor technology is employed for the fabrication of the gold electrodes and for the deposition and patterning of an insulating layer of silicon nitride. The electrodes were tested using a cardiac cell culture of chick embryo myocytes, and the physical beating of the cultured cells correlated with the simultaneous extracellular voltage measurements obtained.

In one embodiment, measuring the effect of a test compound comprises measuring the activation kinetics of the sodium channel.

In another embodiment, measuring the effect of a test compound comprises measuring the deactivation kinetics of the sodium channel.

In another embodiment, measuring the effect of a test compound comprises measuring the inactivation kinetics of the sodium channel.

In another embodiment, measuring the effect of a test compound comprises measuring the reactivation kinetics of the sodium channel

In another embodiment of the invention, the *in vitro* method can be used to screen a compound that inhibits, prevents or stops the action of a compound known to intoxicate a pollinator insect through its sodium channel subunits.

In one embodiment of the invention, the *in vitro* method determine the effect of a test compound to provide modulation reference patterns and databases of modulation reference patterns for a wide range of molecules. The reference patterns are then used for the identification and classification of test molecules. Evaluation of test compounds may be used to achieve different results.

Another object of the invention is an *in vitro* method to determine the toxicity of a test compound on a pollinator insect comprising:

a. contacting modified cells described herein with a test compound,
b. measuring the effect of said test compound on the sodium channel activity, and comparing the effect to the effect of a standard reference or a vehicle solution, thereby determining the toxicity on said sodium channel.

In one embodiment of the invention, a test compound is considered as toxic once a modulating effect is measured on the sodium channel of a pollinator insect.

In one embodiment, a test compound is considered as toxic once it modifies the gating kinetics of the sodium channel.

In another embodiment, a test compound is considered as toxic once it modulates (increases or decreases) the amplitude of sodium current of a pollinator insect sodium channel.

In another embodiment, a test compound is considered as toxic once it modulates (increases or decreases) the inactivation during a depolarizing pulse.

In another embodiment, a test compound is considered as toxic once it alters the activation kinetics or voltage dependence of either channel activation or steady-state inactivation.

In another embodiment, a test compound is considered as toxic once its affinity is modulated (increased or decreased) following repetitive depolarization.

In another embodiment, a test compound is considered as toxic once it induces deleterious neuronal hyperexcitability.

In another embodiment, a test compound is considered as toxic once its affinity is modulated (increased or decreased) upon channel opening.

In another embodiment, a test compound is considered as toxic once it modulated channel closed-state.

In another embodiment, a test compound is considered as toxic once it is dependent of the functional states of the sodium channel with preferred affinity for the close and/or open and/or inactivated states.

In another embodiment, a test compound is considered as toxic once the sorting, the targeting or the translocation of the sodium channel of the invention is modified.

In another embodiment, a test compound is considered as toxic once the stability of the sodium channel of the invention is modified.

In another embodiment, a test compound is considered as toxic once the subunits assembly of the sodium channel of the invention is modified.

In one embodiment, the test compound is not toxic for a pollinator insect of the invention.

In another embodiment, the test compound is toxic for a pollinator insect of the invention.

In another embodiment, the test compound is toxic for invasive species as described here above while said test compound is not toxic for the pollinator insects of the invention.

In another embodiment, the test compound is toxic for insects spreading diseases to a human subject. Examples of such insects are mosquitoes. Examples of such diseases include but are not limited to: malaria, dengue fever, Japanese encephalitis, Ross River virus infection, Barmah Forest virus infection, Murray Valley encephalitis, yellow fever, West Nile Virus.

In another embodiment, the test compound is toxic for an organism such as endoparasites or ectoparasites, mites, viruses, fungus, bacteria, pathogens.

Another object of the invention is an *in vitro* method for screening compounds that modulate the sodium channel activity of a pollinator insect that comprises:

a. contacting modified cells described herein with a test compound,

b. measuring the effect of said test compound on the sodium channel activity, and comparing said effect to the effect of a standard reference or a vehicle solution, thereby classifying said compound as a sodium channel blocker, sodium channel activator, gating modifier.

In one example, the cells are coupled with a substrate such that electrophysiological changes in the cells in response to external stimuli can be measured, e.g., for use as a high-throughput screen for compounds. The cells can also be transfected with DNA that targets, expresses, or knocks-out specific genes or gene products in the cell. By providing such chip-mounted cells coupled with measuring devices, such as a computer, many compounds can be screened rapidly and accurately. The cells or chips could also be coupled to the measuring device in arrays for large-scale parallel screening.

Another object of the invention is an *in vitro* method to detect the binding of a test compound on the sodium channel of a pollinator insect comprising:

a. contacting modified cells described herein with a test compound, and

b. measuring the binding of said test compound on the sodium channel of a pollinator insect.

Another object of the invention is a kit comprising the vector of the invention or the modified cell expressing the sodium channel of the invention and reagents for conducting any one of the above described methods of the invention.

Optionally the kit can comprise culture medium, recombinant nucleic acid sequences, reagents, standard reference compounds, etc. Such kit would typically comprise a compartmentalized carrier suitable to hold in close confinement at least one container. The carrier would further comprise reagents useful for performing said methods. The carrier may also contain a means for detection such as labeled enzyme substrates or the like. Instructions can be provided to detail the use of the components of the kit, such as written instructions, video presentations, or instructions in a format that can be opened on a computer (e.g. a diskette or CD-ROM disk). These instructions indicate, for example, how to use the cells to screen test compounds of interest (such as inotropic drugs).

The present invention also relates to an apparatus and array, respectively, for use in the methods and assays of the present invention described herein. For example, a cell-potential measurement apparatus having a plurality of microelectrodes and which may be used and/or adapted in accordance with the teaching of the present invention is described in European patent application EP 0 689 051 A3.

Furthermore, international application WO98/54294 describes an apparatus and method for monitoring cells and a method for monitoring changes in cells upon addition of a compound to the cell's environment, comprising a device which includes an array of microelectrodes disposed in a cell culture chamber, upon which array a portion of cells adhere to the surfaces of the microelectrodes. The diameter of the cells is larger than the diameters of the microelectrodes. A voltage signal is applied across each of the microelectrodes and a reference electrode. Detection and monitoring of the signals resulting from the application of the voltage signal provides information regarding the electrical characteristics of the individual cells, including impedance (combined cell membrane capacitance and conductance), action potential parameters, cell membrane capacitance, cell membrane conductance, and cell/substrate seal resistance.

The present invention also relates to an automated Voltage-Clamp Screening System for Xenopus oocytes comprising HiClamp robot, USB video camera, HiClamp software, accessories, and consumables which is a well-known technics in the state of the art.

The HiClamp is a fully-automated all-in-one solution for high-throughput functional secondary screening of test compounds based on the standard Xenopus expression system. The HiClamp functionality is based on the use of a novel system in which the oocyte is exposed to a test solution by moving it physically into the solution of interest, whereas in a standard system the solution is applied on the cell.

## BRIEF DESCRIPTION OF THE DRAWINGS

**Figure 1** represents the expression of the Para channel and its regulatory subunits in honeybee tissues. The tissue-specific expression of proteins was assessed by RT-PCR. All the honeybee tissue samples underwent the same preparation steps from dissection to gel electrophoresis. The expected weights of the amplicons are displayed in base pairs (bp).

**Figure 2** represents the effect of the co-expression of the regulatory subunits on the expression of the Para channel. **(a)** Representative current traces from the pulse protocols applied to oocytes expressing either Para alone or Para and one of its regulatory subunits. The pulse protocols consisted of imposing-80 mV to +40 mV voltage steps in 5 mV increments. The oocytes were kept at the holding potential (-80 mV) for 1 s between voltage steps. **(b)** Relative amplitudes of the peak currents from oocytes expressing the Para channel alone or with one regulatory subunit. The peak currents from 13-14 oocytes in response to a -80 mV to -20 mV voltage step were measured for each bar. The mean was then normalized to the mean current amplitude measured in the presence of the TipE subunit. The differences between the means were then evaluated for statistical significance using a t-test with Bonferroni's correction (***significant difference with Para alone, $p < 0.001$). The oocytes came from the same batch and were tested the same day. **(c)** Representative current traces of an oocyte expressing Para and TipE in response to a -80 mV to -20 mV voltage step. The current trace in orange was acquired in normal Ringer's solution while the current trace in black was acquired in Ringer's solution in which the sodium was replaced by choline.

**Figure 3** represents the effect of the different regulatory subunits on the biophysical properties of the Para channel. **(a)** The voltage-dependence of activation (G-V). Activation curves were derived from I-V curves (n = 7-14). The I-V curves were obtained using the protocol shown in the inset. The peak current measured at each potential was then divided by ($V-V_{rev}$), where V is the test potential and $V_{rev}$ is the reversal potential. The conductances were then normalized and fitted to a standard Boltzmann equation (see Materials and Methods). (**b-c**) Plots of the fitted equation activation parameters ($V_{1/2}$ and $k_v$). Statistical differences were tested using a one-way ANOVA with Bonferroni's correction, **(d)** The voltage-dependence of steady-state inactivation. The amplitude of the peak current was plotted as a function of the voltage imposed during the conditioning pulse (n = 8-14) using the protocol in the inset. The data was then fitted to a Boltzmann equation (see Materials and Methods). **(e-f)** Plots of the inactivation parameters ($V_{1/2}$ and $k_v$) of the fitted equation. Statistical differences were tested using a one-way ANOVA with Bonferroni's correction. **(g)** Time constants of current decay. The protocol in **(a)** was used to generate transient sodium currents at different voltages. The decay of the transient current was fitted with a single exponential. For each condition, the time constant extracted from the fit was plotted as a function of the voltage imposed (n = 7-14). Typical raw data obtained as a result of a depolarization to -20 mV is shown in the inset to illustrate the different decay kinetics. **(h)** Recovery from inactivation. The amplitude of the peak current measured during the second pulse was divided by the amplitude measured during the first pulse and was then plotted against the time interval between the pulses (n = 8-14) using the protocol in the inset. **(i)** The resulting curves were fitted with a single exponential in order to compare the time constants. A statistical analysis using one-way ANOVA with Bonferroni's correction was performed to assess significant differences between the oocytes expressing regulatory subunits and oocytes expressing Para alone. *$p < 0.05$, and ** and ***$p$<0.01 and 0.001.

**Figure 4** represents the Apis Para single-channel conductance. **(a)** Single-channel traces from cell-attached oocytes patch are illustrated for -10, -20, -30 and -40 mV. Arrows indicate the onset of patch depolarization from -100 mV to the indicated voltage for 150 ms. **(b)** All-points histogram of the single-channel current amplitudes at -30 mV. The smooth curve is a Gaussian fit with amplitude peaks at 1.77 pA. **(c)** Plot of the current-voltage relationship ($n \geq 7$). The straight line is a linear regression yielding a unitary conductance of 24.5 $\pm$ 0.2 pS. Data are presented as mean $\pm$ SEM.

**Figure 5** represents the effect of permethrin on the honeybee Para channel. **(a-c)** The presence of 10 $\mu$M permethrin had no significant effect on the voltage-dependence of activation **(a),** the voltage-dependence of steady-state inactivation **(b),** or the recovery from inactivation **(c)** of Para channels co-expressed with TipE. It did, however, induce a tail current. The maximal amplitude of the tail current after 200 conditioning pulses was recorded at different voltages **(d).** The normalized amplitude was plotted as a function of the voltage imposed during the recording (n = 9). The data was then fitted with a linear function ($R^2$ = 0.9792, y0 = -8.3641%, slope = 1.037%/V).

**Figure 6** represents Permethrin induced tail currents in oocytes expressing the honeybee Para channel. The presence of 10 $\mu$M permethrin induced tail currents in oocytes co-expressing the Para channel and the TipE regulatory subunit. **(a)** The amplitude of the tail current was highly dependent on the number of depolarizations imposed before the recording (pulses from -80mV to -20mV repeated at a frequency of 100 Hz). **(b)** The amplitude of the tail current was also dependent on the concentration of permethrin in the extracellular medium. **(c)** The number of conditioning pulses required to attain the maximal amplitude of the tail current in the presence of 10 $\mu$M permethrin was then determined (n = 4). **(d)** The fraction of channels bound to permethrin was plotted as a function of the dose applied using 200 conditioning pulses. The data was then fitted with a Hill curve in order to extract the Hill coefficient (h = 0.8263) and the half maximal effective concentration ($EC_{50}$ = 3.712 $\mu$M). The error bars represent the SEM.

**Figure 7** represents Fenvalerate induced tail currents in oocytes expressing the honeybee Para channel. The presence of 10 $\mu$M fenvalerate induced a tail current in oocytes co-expressing the Para channel and the TipE regulatory subunit. **(a)** The amplitude of the tail current was highly dependent on the number of depolarizations imposed before the recording (pulses from -80mV to -20mV repeated at a frequency of 100 Hz). **(b)** The amplitude of the tail current was also dependent on the concentration of fenvalerate in the extracellular medium. **(c)** The number of conditioning pulses required to attain the maximal amplitude of the tail current in the presence of 10 $\mu$M fenvalerate was then determined (n = 3). **(d)** The fraction of channels bound to fenvalerate was plotted as a function of the dose applied using 1700 conditioning

pulses. The data was then fitted with a Hill curve in order to extract the Hill coefficient (h = 1.1817) and the half maximal effective concentration ($EC_{50}$ = 289 nM). The error bars represent the SEM.

## EXAMPLES

### *Materials and Methods*

<u>*Sequencing and cloning*</u>

Total RNA from honeybee heads was extracted using Trizol kits (Sigma). cDNA was produced using Transcriptor first strand cDNA synthesis kits (Roche). The cDNA corresponding to the Para, TipE, and TEH1-4 proteins was obtained by PCR amplification.

The cDNA of all the regulatory subunits were inserted into the pPol_Not1 vector (a generous gift from Dr. Paul Isenring, Université Laval), an oocyte expression vector containing the T7 promoter (5' to 3'), the Xenopus laevis ß-globin 5'-untranslated region, a multiple cloning site, the Xenopus laevis ß-globin 3'-untranslated region, a linearizing site, and polyA and polyC tracts.

For the Para channel, two overlapping fragments were cloned in the TOPO plasmid. Insertion of the whole channel in the pPOL plasmid is still to be performed.

Prior to insertion in a plasmid with a standard digestion and ligation method, restriction sites were introduced at the beginning and end of each fragment cloned using PCR amplification with the following primers **(Table 1):**

**Table 1**

| Name | Restriction site | Primers | SEQ ID NO |
|---|---|---|---|
| Fwd-para #1 | Kpn1 | GAGA**GGTACC**GCCGCCACCATGTCCGAAGATTCTGACTC | SEQ ID NO: 25 |
| Rev-para #1 | | GCCAGCGCCACAGAGCGACGCTACGAAGTTAATGAGTGAC | SEQ ID NO: 26 |
| Fwd-para #2 | | GTCACTCATTAACTTCGTAGCGTCGCTCTGTGGCGCTGGC | SEQ ID NO : 27 |
| Rev-para #2 | Xma1 | GAGA<u>**CCCGGG**TCA</u>GACGTCCGCGGTTCTCG | SEQ ID NO: 28 |
| Fwd-TipE | BamH1 | GAGA<u>**GGATCC**</u>GCCGCCACC<u>ATG</u>GCGGAGGAAAAGGAGAAG | SEQ ID NO: 29 |
| Rev-TipE | HindIII | GAGA<u>**AAGCTT**</u>GCT<u>TCA</u>GACTGCCGCCGCCGT | SEQ ID NO : 30 |
| Fwd-Teh1 | BamH1 | GAGA<u>**GGATCC**</u>GCCGCCACC<u>ATG</u>CGCGGGAGCAGCTCTGA | SEQ ID NO : 31 |
| Rev-Teh1 | EcoR1 | GAGA<u>**GAATTC**TTA</u>CCTGAGATCGTTGCCCCTG | SEQ ID NO : 32 |
| Fwd-Teh2 | BamH1 | GAGA<u>**GGATCC**</u>GCCGCCACC<u>ATG</u>GGCGCCCAGGAAGAG | SEQ ID NO : 33 |
| Rev-Teh2 | HindIII | GAGA<u>**AAGCTT**TTA</u>CTCTTCCTTCCTTGCGTACTTGT | SEQ ID NO : 34 |
| Fwd-Teh3 | BamH1 | GAGA<u>**GGATCC**</u>GCCGCCACC<u>ATG</u>CCTAAGCAAGTGCCAGTG | SEQ ID NO: 35 |
| Rev-Teh3 | EcoR1 | GAGA<u>**GAATTC**TCA</u>GAGCGCCATAGCGGAGT | SEQ ID NO : 36 |
| Fwd-Teh4 | EcoR1 | GAGA<u>**GAATTC**</u>GCCGCCACC<u>ATG</u>GGTCGAAAGCATAAACG | SEQ ID NO : 37 |
| Rev-Teh4 | HindIII | GAGA<u>**AAGCTT**</u>CTG<u>CTA</u>AGCTAAAGCGCCATGCT | SEQ ID NO: 38 |
| Para fragment #1 (from ATG to the 4131$^{th}$ nucleotide, starting with A from ATG)<br>Para fragment #2 (from 4092$^{th}$ nucleotide to the stop codon) | | | |

The vectors containing the regulatory subunits were amplified in Escherichia coli XL2 Blue (Agilent) and were purified using GenElute HP Plasmid Maxiprep kits (Sigma).

The constructs were linearized with Not1, and T7 RNA polymerase was used to make sense RNA using mMESSAGE mMACHINE T7 kits (Ambion). mRNA was also generated using mMESSAGE mMACHINE kits with a custom DNA featuring a T7 promoter, the Para channel sequence, the Xenopus laevis ß-globin 3'-untranslated region, and polyA and polyC tracts.

To identify sequence variations, genomic DNA from whole bees was extracted and was sequenced using QIAamp DNA mini kits (Qiagen).

<u>*RT-PCR*</u>

Total RNA from honeybee tissues was extracted using Trizol kits (Sigma). cDNA preparations were then produced using Transcriptor first strand cDNA synthesis kits (Roche). cDNA preparations corresponding to the Para, TipE, and TEH1-4 proteins were obtained by PCR amplification using the following oligonucleotide primers **(Table 2):**

**Table 2: list of primers**

| Name | Primers | SEQ ID NO |
|---|---|---|
| Fwd - para | GCAGATGGCGAACTCGATGCG | SEQ ID NO : 13 |
| Rev - para | CGATGGCATCGTTCATGATTTGAATC | SEQ ID NO : 14 |
| Fwd - TipE | ATGGCGGAGGAAAAGGAGAAGCAG | SEQ ID NO : 15 |
| Rev - TipE | TCAGACTGCCGCCGTCGGC | SEQ ID NO : 16 |
| Fwd - Teh1 | ATGCGCGGGAGCAGCTCTG | SEQ ID NO : 17 |
| Rev - Teh1 | TTACCTGAGATCGTTGCCCCTGC | SEQ ID NO : 18 |
| Fwd - Teh2 | ATGGGCGCCCAGGAAGAGGAC | SEQ ID NO : 19 |
| Rev - Teh2 | TTACTCTTCCTTCCTTGCGTACTTGTC | SEQ ID NO : 20 |
| Fwd - Teh3 | ATGCCTAAGCAAGTGCCAGTGGAG | SEQ ID NO : 21 |
| Rev - Teh3 | TCAGAGCGCCATAGCGGAGTTTC | SEQ ID NO : 22 |
| Fwd - Teh4 | ATGGGTCGAAAGCATAAACGCCG | SEQ ID NO : 23 |
| Rev - Teh4 | CTAAAGCGCCATGCTTTTGATCTC | SEQ ID NO : 24 |

The resulting cDNA preparations were run on agarose gels to determine whether the genes of interest were expressed in the tissues under investigation. Controls were performed with total RNA preparations using the same protocol but without reverse transcriptase.

*Xenopus oocytes*

Xenopus oocytes were handled in accordance with the principles and guidelines of the Université Laval animal protection committee and the Canadian Council on Animal Care. They were prepared as described previously (Chahine M et al. (1992) J Mol Cell Cardiol 24: 1231-1236). Briefly, oocytes were surgically removed from frogs anesthetized with MS-222. They were then treated with 2 mg/ml collagenase (Collagenase type 1A from Clostridium histolyticum, Sigma) in OR2 solution for 1 h. They were then incubated for at least 1 h at 18°C in OR3 solution. Stage IV and V oocytes were microinjected with mRNA corresponding to the Para channel and the selected regulatory subunit (1 μg/μl for Para and 0.3 μg/μl for regulatory subunits, 50 nl/oocyte). The injected oocytes were incubated at 18°C in OR3 solution for at least 12 h before taking recordings.

*Electrophysiological experiments,*

Macroscopic currents from mRNA-injected oocytes were recorded in Ringer's solution using the two-microelectrode voltage-clamp technique. The membrane potential for the two-microelectrode voltage-clamp technique was controlled using a Warner oocyte clamp (Warner Instrument Corp.). The currents were filtered at 5 kHz (-3 dB; four-pole Bessel filter). The headstage of the Warner amplifier was attached to a plastic pool containing a 3M NaCl solution through a silver chloride wire connected to the bath solution using an agar bridge. The bridge contained 3% agar (w/v), 500 mM NMDG, and 10 mM HEPES (pH 7.4) and was threaded with a thin platinum/iridium wire to lower high-frequency impedance.

For the cell-attached patch experiments, the vitelline membrane was removed with forceps after briefly placing an oocyte in a hyperosmotic solution. Command pulses were generated and currents were recorded using pCLAMP software v10.3 and an Axopatch 200B amplifier (Molecular Devices). Patch electrodes (3-7 MΩ) fashioned from borosilicate glass (Coming 8161) were coated with HIPEC® (Dow Coming) to reduce capacitance and noise emissions. Single-channel currents were filtered at 2 KHz and were sampled at 100 kHz. Traces were low-pass filtered at 1.5 kHz using a digital filter included in the Clampfit software. Capacity transients were eliminated by averaging recordings without openings and subtracting this average from all recordings.

*Solutions and reagents*

The Ringer's bath solution was composed of 116 mM NaCl, 2 mM KCl, 2 mM $CaCl_2$, 2.9 mM $MgCl_2$, and 5 mM HEPES. The pH was adjusted to 7.6 at 22°C using 1 M NaOH.

The hyperosmotic solution used for single-channel recordings was composed of 200 mM K-aspartate, 20 mM KCl, 1 $MgCl_2$, 10 mM EGTA, and 10 mM HEPES (pH 7.5). For cell-attached experiments, the bath solution was composed of 100 mM K-aspartate, 50 mM KCl, 1.5 mM $CaCl_2$, 1 mM $MgCl_2$, 10 mM glucose, and 10 mM HEPES (pH 7.4). The pipette solution was composed of 150 mM NaCl, 2 mM KCl, 1.5 mM $CaCl_2$, 1 mM $MgCl_2$, 10 mM glucose, and 10 mM HEPES (pH 7.4).

The $OR_3$ solution was composed of a 1:2 dilution of Leibovitz's L-15 medium supplemented with 15 mM HEPES and 50 mg/ml of gentamycin. The pH was adjusted to 7.6 at 22°C using 1 M NaOH (Chahine M et al. (1992) J Mol Cell Cardiol 24: 1231-1236).

The $OR_2$ solution was composed of 82.5 mM NaCl, 2.5 mM KCl, 1 mM $MgCl_2$, and 5 mM HEPES. The pH was adjusted to 7.6 at 22°C using 1 M NaOH.

All the chemicals and drugs were from Sigma, except for tetrodotoxin, which was from Latoxan (France) and Leibovitz's L-15 medium, which was purchased from Invitrogene (USA) in powder form. All the experiments were carried out at 22°C.

*Data analysis and statistics*

The electrophysiological data were analyzed using macros in Clampfit (pCLAMP v10.0; Molecular Devices) and custom scripts written using MATLAB (The MathWorks Inc.). The results are expressed as means ± SEM. Statistical comparisons were performed using a one-way ANOVA with Bonferroni's post hoc test in SigmaPlot (Systat Software). Differences were deemed significant at $p < 0.05$. The *p* values and number of measurements are indicated in the text or figure legends.

The fraction of channels modified by pyrethroid insecticides was calculated using the following equation:

$$M(\%) = 100\% \times \frac{I_{tail}/(E_{tail}-E_{rev})}{I_{dep}/(E_{dep}-E_{rev})},$$

where $I_{tail}$ is the maximal amplitude of the tail current measured in the 170s following the last conditioning pulse (the first 5ms are excluded to avoid the inclusion of a capacitive component in the current measured), $E_{tail}$ is the membrane potential at which the tail current was measured, $E_{rev}$ is the reversal potential of sodium ions as calculated using an I-V curve, and $I_{dep}$ is the peak current observed in response to a depolarization at the $E_{dep}$ potential.

The dose-response curves obtained from these calculations were fitted to a Hill curve using the following equation:

$$M = \frac{M_{Max}}{1+(EC_{50}/dose)^h},$$

where h is the Hill coefficient, $M_{max}$ is the maximal fraction of channels affected, $EC_{50}$ is the half-maximal effective concentration, and dose is the concentration at which the observation was made.

The voltage-dependence of activation was fitted to the following Boltzmann equation:

$$\frac{G_{(V)}}{G_{Max}} = \frac{1}{1+\exp^{(V-V_{1/2})/k}},$$

while the voltage-dependence of inactivation was fitted to the following equation:

$$\frac{I_{(V)}}{I_{Max}} = \frac{1}{1+\exp^{(V-V_{1/2})/k}},$$

where $V_{1/2}$ is the half-maximal voltage of activation (or inactivation), G is the conductance, I is the current measured at a given voltage (V), and k is the slope factor (in mV).

Current decay and tail current kinetics were fitted to the following equation when a single exponential was used:

$$y = y_o + A(1 - \exp(^{-x}/_{\tau})),$$

and to the following equation when double exponential was used:

$$y = y_o + A_1(1 - \exp(^{-x}/_{\tau_1})) + A_2(1 - \exp(^{-x}/_{\tau_2})),$$

where $y_0$ is the initial value, A is the weight of the exponential, and $\tau$ is the time constant of the exponential.
All fits were performed using the Levenberg-Marquardt algorithm.

***Results***

The present invention is further illustrated by the following examples.

Example 1: Analysis of the Para channel and the regulation of its regulatory subunits

An RT-PCR analysis showed that RNA corresponding to the Para channel is expressed in all the honeybee tissues studied (brain, antenna, muscle, legs, gut, and ganglion). Para channel's RNA was also present in the larvae **(Figure 1).** This suggests that the Para channel plays a major role in the physiological functions of the honeybee.
The regulatory subunits of the Para channel were also widely expressed in these tissues, with the exception of TEH1, which was not expressed in the gut. TEH2 and TEH4 also appeared to be expressed at very low levels in the gut **(Figure 1).**

Example 2: Biophysical characterization

*The regulatory subunits modulate the expression of the Para channel*

The co-expression of the regulatory subunits with the Para channel was not required to record sodium currents in Xenopus oocytes, indicating that it is robustly expressed on the membrane **(Figure 2a).** Raw data from standard pulse protocols showed that the honeybee TipE and TEH subunits affect the kinetics of the channel **(Figure 2a).** In addition, the co-expression of the honeybee TipE and TEH subunits with the Para channel increased the peak current **(Figure 2b).** However, only the co-expression of the Para channel with the TipE or TEH2 subunits yielded a statistically significant increase in expression levels.
As indicated by the sequence analysis, the Para channel is selective for sodium ions. Indeed, removing sodium ions from the extracellular medium abolished the current produced by the Para channel **(Figure 2c).**

*Voltage-dependence of activation of the Para channel*

Like its mammalian counterparts, the Para channel is activated by membrane depolarizations. The conductance-voltage relationship (G-V curve) of the channel fits a Boltzmann function with a $V_{1/2}$ of -31.7 ± 0.9 mV and a slope factor (k) of -2.9 ± 0.2 mV (n = 11).
An analysis of the activation properties of the Para channel in the presence of the various regulatory subunits showed that the TEH2, TEH3, and TEH4 subunits shift the voltage-dependent activation of the channel to the right **(Figure 3a).** When fitted to a Boltzmann function, this shift in the voltage-dependence of activation is represented by a shift of $V_{1/2}$ toward more positive values **(Figure 3b).** The TEH1, TEH3, and TEH4 subunits also had a significant effect on the slope factor of the Boltzmann curve **(Figure 3c),** causing a less steep voltage-dependence of activation curve than the curve for the Para channel alone. Overall, our data indicated that activation of the channel is slightly hindered in the presence of TEH2, TEH3, and TEH4, and is slightly facilitated in the presence of TEH1.

*Voltage dependence of fast inactivation of the Para channel*

The voltage-dependence of fast inactivation of the Para channel was also fitted with a Boltzmann function. The voltage of half-maximal inactivation ($V_{1/2}$) in the absence of subunits was -52.1 ± 0.6 mV and the slope factor (k) was 4.3 ± 0.1 mV (n = 11).

The voltage-dependence of fast inactivation of the channel in the presence and absence of the regulatory subunits was evaluated using a standard two-pulse protocol. The current measured during the test pulse was plotted as a function of the voltage imposed during the conditioning pulse **(Figure 3d).** Like the voltage-dependence of activation, the voltage-dependence of inactivation was fitted to a two-state Boltzmann function. A statistical analysis revealed a negative shift of the half-maximal voltage of inactivation ($V_{1/2}$) in the presence of TEH1 and a positive shift in the presence of TEH4 **(Figure 3e).** In addition, the slope factor (k) was higher in the presence of TEH3 and TEH4 **(Figure 3f).** Overall, this means that TEH1 increases the steady-state inactivation of the channel while TEH4 decreases the steady-state inactivation. Differences in the time course of current decay were also observed **(Figure 3g).** Fast inactivation was the main determinant of current decay. As indicated by the raw data presented in **Figure 2a,** the fast inactivation kinetics of the Para channel were much slower in the presence of the TEH3 and TEH4 regulatory subunits, but were not affected by TipE, TEH1, or TEH2.

*Recovery from inactivation*

Recovery from inactivation was also evaluated using a two-pulse protocol. Briefly, the relative amplitude of the test pulse was plotted as a function of the $\Delta t$ separating the conditioning and test pulses. The data points were fitted with a double exponential function. In the absence of the regulatory subunits, the faster exponential had a relative weight of 0.85 $\pm$ 0.05 and time constant ($\tau$) of 8.2 ms $\pm$ 0.5 ms (n = 11). The slower exponential had a relative weight of 0.15 $\pm$ 0.05 and time constant ($\tau$) of 80 ms $\pm$ 30 ms (n = 11). Despite slight apparent differences in the recovery from inactivation curves **(Figure 3h),** no statistically significant differences were observed in the presence of the regulatory subunits **(Figure 3i).**

*Honeybee Para single channel conductance*

The single channel conductance of the honeybee Para channel co-expressed with the TipE regulatory subunit was investigated using the patch clamp technique in the cell-attached configuration. Raw current traces were obtained at different voltages **(Figure 4a).** All-points histograms were then created in order to extract the mean single channel current amplitudes **(Figure 4b).** The extracted values were plotted as a function of the voltage imposed **(Figure 4c).** The data was fitted to a linear equation, yielding a slope value of 24.5 $\pm$ 0.2 pS for single channel conductance.

<u>Example 4: Pharmacological characterization</u>

*Sensitivity to pyrethroids*

To determine whether the honeybee Para channel could be used to test the sensitivity of the honeybee for pyrethroid insecticides, we tested the effect of permethrin and fenvalerate, respectively type I and type II pyrethroid insecticides.

Adding 10 $\mu$M permethrin to the extracellular medium did not induce statistically significant shifts in activation, inactivation, or recovery from inactivation **(Figure 5a-c).** Following conditioning pulses, extracellular permethrin induced a tail current that exhibited a linear voltage-dependence and an inversion at approximately +10mV **(Figure 5d).** A similar reversal potential was also observed for the transient current elicited in response to the pulse protocol used to create the G-V curve shown in **Figure 3a,** indicating that the transient current and the tail current have similar selectivity and are most likely of the same nature.

Permethrin induced tail currents following activation, indicating that it traps the channel in its open state. The amplitude of the tail current increased as a function of the number of conditioning pulses **(Figure 6a)** and the concentration of permethrin **(Figure 6b),** indicating that this insecticide binds to the channel in its open state. In order to characterize the affinity of the channel for permethrin, we measured the amplitude of the tail current using varying numbers of conditioning pulses. Plotting the amplitude of the tail current as a function of the number of conditioning pulses showed that the maximal effect is reached after 200 pulses **(Figure 6c).** Determining the number of pulses needed to reach the maximal effect made it possible to plot a dose-response curve **(Figure 6d).** When fitted with the Hill equation, the curve yielded an $R^2$ of 0.998, a Hill coefficient of 0.83, and an $EC_{50}$ of 3.71 $\mu$M.

Fenvalerate induced similar tail currents that depended on the number of conditioning pulses **(Figure 7a)** and the concentration of this insecticide **(Figure 7b).** Determining the number of conditioning pulses needed for maximal effect **(Figure 7c)** was necessary in order to create a dose-response curve for fenvalerate **(Figure 7d).** Fitting the data to the Hill curve yielded an $R^2$ of 0.9987, a Hill coefficient of 1.18, and an $EC_{50}$ of 289 nM.

Comparing the results obtained from the application of permethrin and fenvalerate showed that more conditioning pulses were required to reach a maximal effect with fenvalerate. However, fenvalerate induced larger tail currents and had a higher affinity for the honeybee Para channel than permethrin. In addition, the tail currents induced by fenvalerate took longer to deactivate **(Figure 6b and 7b).** The deactivation of permethrin-induced tail currents was fitted to a single exponential with a time constant of 0.93 s ± 0.11 s. The deactivation of the fenvalerate-induced tail currents was better fitted to a double exponential. The relative weight of the slow component was 85.4% ± 3.6%. The time constants for both exponentials were 9.9 s ± 2.4 s and 31.8 s ± 1.8 s respectively.

***Discussion***

RT-PCR experiments showed that the RNA corresponding to all the proteins investigated are expressed almost ubiquitously in honeybee tissues, indicating that the Para channel may be involved in a wide variety of physiological functions in the honeybee. Curiously, earlier reports indicate that no sodium current were detectable in the honeybee's muscle fibres (Collet C et al. (2007) J Exp Biol 210: 454-464). This may be due to the fact that Collet et al. conducted their investigation on dissociated muscle cells whereas our RT-PCR experiments were conducted on muscle tissue. As such, our preparation should include nerve endings which could contain Para channels and its corresponding RNA.

We observed very few sequence variations for Para, TipE, and TEH4. However, the sequence variations we mapped were seen in total RNA preparations from honeybee heads. Further investigations should focus on mapping all the sequence variations for the proteins reported here using other total RNA preparations. Examining the expression of sequence variants in specific tissues would be the first step toward determining their physiological roles.

The biophysical characterization of the honeybee Para channel with and without its regulatory subunits provided a brief overview of the basic properties of the channel. The regulatory subunits can be divided into two subfamilies based on their effect on the Para channel. TipE, TEH1, and TEH2 did not change or increase steady-state inactivation while TEH3 and TEH4 hindered entry into the inactivated state. TEH3 and TEH4 also hindered activation of the channel and had a marked effect on current decay. The proposed division into two subfamilies is supported by the sequence analyses we performed. The phylogenetic tree indicated that the sequences of TEH3 and TEH4 are more similar to each another than to the other regulatory subunits. They are also the only proteins of the family to feature EGF-like domains.

The experiments reported here provide all the protocols and results required to test the toxicity of compounds for honeybees. Similar experiments could also be performed on other cloned insect channels and could be compared to the results from the honeybee channel to make sure that new insecticides are toxic for other insects, but not the honeybee.

Permethrin and fenvalerate induced tail currents following activation. However, the $EC_{50}$ for permethrin was shifted by more than an order of magnitude, indicating that type I pyrethroids may have higher affinities for the honeybee Para channel. While we calculated an affinity of approximately 3.7 μM for the honeybee Para channel, this does not mean that permethrin is not chronically or acutely toxic for the honeybee. Rather, it indicates that careful dosage may help minimize the impact on honeybees, although chronic exposure may still be deleterious (Taylor KS, Waller GD, Crowder LA (1987) Impairment of a Classical Conditioned Response of the Honey Bee (Apis Mellifera L.) by Sublethal Doses of Synthetic Pyrethroid Insecticides. Apidologie 18: 243-252). The $EC_{50}$ we measured is very close to the $EC_{50}$ for permethrin on the sodium current of honeybee antennal olfactory receptor neurons (3.5 μM) (Kadala A et al. 2011 Neurotoxicology 32: 320-330), indicating that the undesirable effect of permethrin on the olfactory functions of the honeybee may be exclusively due to the binding of the compound to the Para channel. It also indicates that the *in vitro* technique proposed here replicates the results obtained from more complicated setups.

Fenvalerate had an $EC_{50}$ of approximately 290 nM for the honeybee Para channel. This higher affinity at lower concentrations than permethrin may make fenvalerate less suitable for agricultural uses. Fenvalerate also induced tail currents that decay less rapidly than the currents induced by permethrin, indicating that it is potentially more toxic for honeybees.

Interestingly, the drosophila Para channel seems more affected by permethrin than the honeybee channel ($EC_{50}$ = 12 nM for the drosophila channel (Vais H et al. 2003 Mol Pharmacol 64: 914-922) vs. 3.7 μM for the honeybee channel). This difference could be due in part to differences in the experimental procedures. Indeed, Vais et al. used ATX-II to inhibit inactivation while this toxin was not used in our study. Some key residues in the sequence of the honeybee Para channel may also explain the difference between the affinities of the honeybee and drosophila channels. Rinkevich et al. (2013 Pestic Biochem Physiol 106: 93-100) summarized the state of knowledge about 38 mutations associated with knock-down resistance to pyrethroids in arthropods. Five amino acids in the sequence of the honeybee Para channel are suggestive of mutations associated with knock-down resistance to pyrethroids in arthropods. While these five amino acids are unlikely to account for the shift in sensitivity of the honeybee channel compared to the drosophila channel, investigations focused on these residues are warranted. The E485K mutation (using *M. domestica* numbering) has been associated with knock-down resistance in *Blattella germanica* in the background of L1014F and L1014F+C785R (Tan J, et al. 2002 Insect Biochem Mol Biol 32: 445-454). The corresponding residue in the honeybee sequence is also negatively charged (D489). It is thus unlikely that this residue contributes significantly to a change in affinity for pyrethroids.

The L1596P mutation (using *M. domestica* numbering) in *V. destructor* induces a lower affinity for pyrethroids (Liu Z et al. 2006 Insect Biochem Mol Biol 36: 885-889). The corresponding residue in the honeybee is P1595. Interestingly, this residue is also a proline in other known insect Para channels. This may explain the differences in the affinity of the *V. destructor* and honeybee Para channels for pyrethroids, but not between the honeybee and drosophila channels since the proline residue is present in both these species.

The A1101T, A1215D, and M1823I mutations (using *M. domestica* numbering) have also been associated with knock-down resistance phenotypes. However, one should be careful in drawing conclusions from these results since none of these mutations have been confirmed *in vitro.* Further information on these mutations and the corresponding residues in the honeybee Para channel sequence can be found in **Table 3.**

**Table 3.**

| Original Amino Acid | Numbering *Musca Domestica* | Mutated Amino acid | *Apis Mellifera* | |
|---|---|---|---|---|
| | | | Amino Acid | Numbering |
| I | 254 | N | I | 264 |
| V | 410 | A, G, L, M | V | 419 |
| E | 435 | K | E | 444 |
| E | 485 | K | D | 489 |
| C | 785 | R | C | 789 |
| M | 827 | I, T | M | 831 |
| M | 918 | I, L, T, V | M | 922 |
| L | 925 | I | L | 929 |
| T | 929 | C, I, N, V | T | 933 |
| L | 932 | F | L | 936 |
| G, C | 933 | V, A | C | 937 |
| I | 936 | V | I | 940 |
| Q | 945 | R | Q | 949 |
| F | 979 | S | F | 983 |
| S | 989 | P | S | 993 |
| V | 1010 | L | V | 1014 |
| I | 1011 | M, V | I | 1015 |
| N | 1013 | S | N | 1017 |
| L | 1014 | C, F, H, S, W | L | 1018 |
| V | 1016 | G, I | V | 1020 |
| F | 1020 | C | F | 1024 |
| L | 1024 | V | L | 1028 |
| A | 1060 | T | A | 1099 |
| A | 1215 | D | Poor alignment | |
| A | 1410 | V | A | 1409 |
| A | 1494 | V | A | 1493 |
| M | 1524 | I | M | 1523 |
| F | 1528 | L | F | 1527 |
| F | 1534 | C | F | 1533 |
| F | 1538 | I | F | 1538 |
| D | 1549 | V | D | 1548 |
| E | 1553 | G | E | 1552 |
| N | 1575 | Y | N | 1574 |
| L | 1596 | P | P | 1595 |
| I | 1752 | V | I | 1751 |
| D | 1763 | Y | D | 1762 |
| M | 1823 | I | I | 1822 |
| P | 1879 | S | P | 1878 |

Furthermore, whereas kdr mutations in Para clearly provide a resistance to pyrethroids in many insect species, several other physiological processes (e.g. physical, metabolic) are also involved (Riveron et al. 2014 Genome Biol. 15: R27). From our results, the honeybee Para may apparently appear less sensitive to permethrin than drosophila Para, but substantial differences in protocols may lead to misinterpretation of data (ATX-II, a toxin that blocks Para inactivation was routinely used in Vais et al). Also, permethrin is known to be extremely toxic to honeybees since contact DL50 for permethrin is in the ppb range (24 ng/bee). Conversely, affinity of fenvalerate for the honeybee Para in vitro appear much lower than that of fenvalerate, but DL50 shows that it is ~17 times less toxic than permethrin. The reason for this discrepancy might for instance be found either in detoxication capacities, in the existence of a more toxic metabolite or in the existence of a secondary target for permethrin.

We further report the creation of a structural model of the honeybee Para sodium channel, which paves the way to *in silico* screening of compounds that can bind to the pore or to the pyrethroid binding site. This structural model may be used as a first step in the intelligent design of compounds intended either to target or to avoid targeting the honeybee Para sodium channel. Our model indicated that the binding site for pyrethroids in the honeybee Para sodium channel is similar to the site recently identified in A. *aegypti* (Du et al. 2013 Proc Natl Acad Sci U S A 110: 11785-11790). This may be an obstacle for the creation of insecticides that specifically target the Para channels of insect pests but not the honeybee. However, given the low homology between the Para channels of the honeybee and *V. destructor,* pyrethroid derivatives could be used to target this parasite which now invades the hives of both eastern and western honeybees (Rosenkranz P et al. 2010 Biology and control of Varroa destructor. J Invertebr Pathol 103 Suppl 1: S96-119).

The binding poses showed pyrethroid insecticides in close proximity to the residues corresponding to those mapped by Du et al. (2013 Proc Natl Acad Sci U S A 110: 11785-11790). The distance of docked compounds from these residues could thus be used as an objective criterion to predict potential interactions with the Para channel.

We report the creation of new *in vitro* and *in silico* tools to test the toxicity of compounds for honeybees. These tools could be used to screen insecticides for agricultural and in-hive use. Compounds that induce statistically significant changes to the properties of the honeybee Para channel at their recommended doses must be used with care. Efforts to reduce or eliminate toxic products from the environment should help alleviate pressure on honeybee colonies. Regardless of whether the cause of CCD is an infection, the overuse of chemicals, or a combination of these causes, taking the toxicity of certain compounds for honeybees into account should help in the fight against the disappearance of our principal pollinator.

## Claims

1. A nucleic acid sequence of the regulatory subunit TipE as set forth in SEQ ID NO: 3 or variant thereof consisting of a nucleic acid sequence of less than 1500 bp and having at least 90% identity with SEQ ID NO: 3.

2. A nucleic acid sequence of the regulatory subunit Teh1 as set forth in SEQ ID NO: 5 or variant thereof consisting of a nucleic acid sequence having more than 99% identity with SEQ ID NO: 5.

3. A nucleic acid sequence of the regulatory subunit Teh2 as set forth in SEQ ID NO: 7 or variant thereof consisting of a nucleic acid sequence of less than 1000 bp and having at least 90% identity with SEQ ID NO: 7.

4. A nucleic acid sequence of the regulatory subunit Teh3 as set forth in SEQ ID NO: 9 or variant thereof consisting of a nucleic acid sequence of less than 3000 bp and having at least 90% identity with SEQ ID NO: 9.

5. A nucleic acid sequence of the regulatory subunit Teh4 as set forth in SEQ ID NO: 11 or variant thereof consisting of a nucleic acid sequence of less than 2500 bp and having at least 90% identity with SEQ ID NO: 11.

6. A vector comprising at least one nucleic acid sequence as in anyone of claims **1** to **5.**

7. A modified cell expressing at least one vector of claim **6.**

8. A sodium channel of a pollinator insect comprising a Para subunit having a nucleic acid sequence as set forth in SEQ ID NO: 1 or variant thereof having at least 90% identity with SEQ ID NO: 1.

9. The sodium channel according to claim **8** further comprising at least one regulatory subunit of said sodium channel, wherein said regulatory subunit is TipE, Teh1, Teh2, Teh3 and/or Teh4 having the sequences as in anyone of claims **1** to **5**.

10. A vector comprising the sodium channel according to claim **8** or **9.**

11. A modified cell expressing a sodium channel according to claim **8** or **9** or a vector according to claim **10.**

12. The modified cell according to claim **7** or **11,** wherein said cell is an oocyte of Xenopus.

13. An *in vitro* method to determine the effect of a test compound on the modulation of activity of a sodium channel of a pollinator insect comprising:

    a. contacting modified cells according to anyone of claims **7** or **11** to **12** with a test compound,
    b. measuring the effect of said test compound on the sodium channel activity, and

    comparing said effect to the effect of a standard reference or a vehicle solution, thereby determining a modulation of activity of said sodium channel.

14. The method according to claim **13,** for determining the toxicity of a test compound on a pollinator insect.

15. An *in vitro* method for screening compounds that modulate the sodium channel activity of pollinator insects comprising:

    a. contacting modified cells according to anyone of claims **7** or **11** to **12** with said compound,
    b. measuring the effect of said compound on the sodium channel activity, and

    comparing said effect to the effect of a standard reference or a vehicle solution, thereby determining a modulation of activity of said sodium channel.

16. A kit comprising at least one vector according to claim **6** or **10** or a modified cell according to claim **7** or **11** to **12** and reagents.

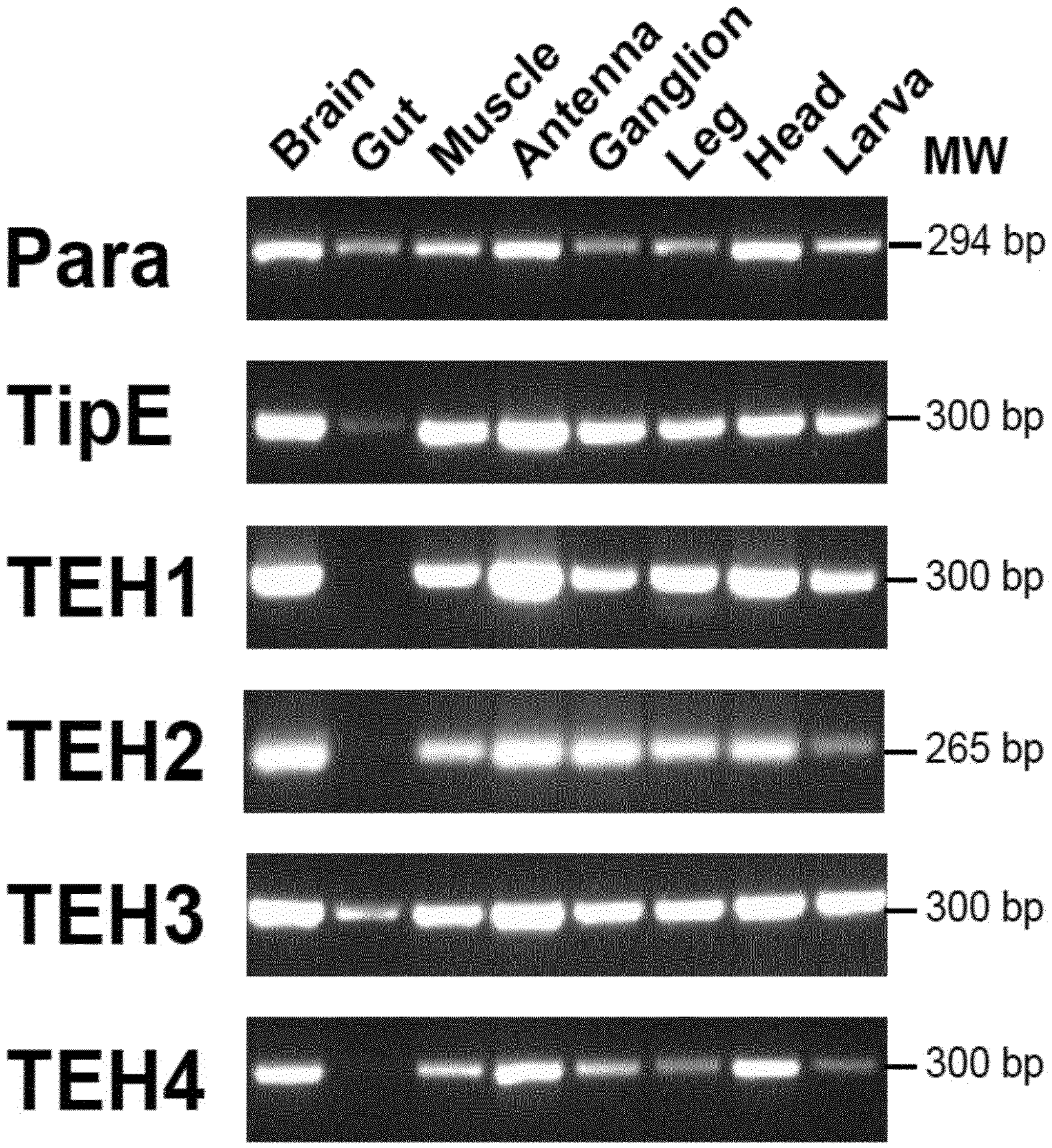
Brain
Gut
Muscle
Antenna
Ganglion
Leg
Head
Larva
MW
Para
294 bp
TipE
300 bp
TEH1
300 bp
TEH2
265 bp
TEH3
300 bp
TEH4
300 bp

FIG. 1

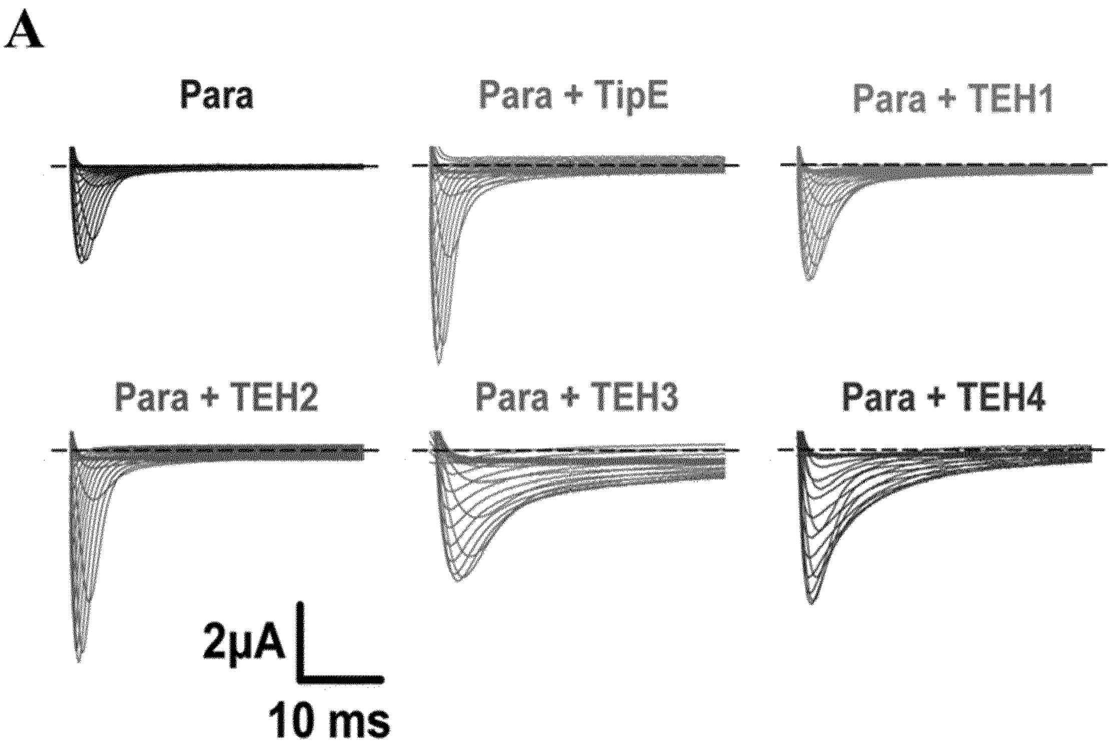
A
Para
Para + TipE
Para + TEH1
Para + TEH2
Para + TEH3
Para + TEH4
2µA
10 ms

FIG. 2

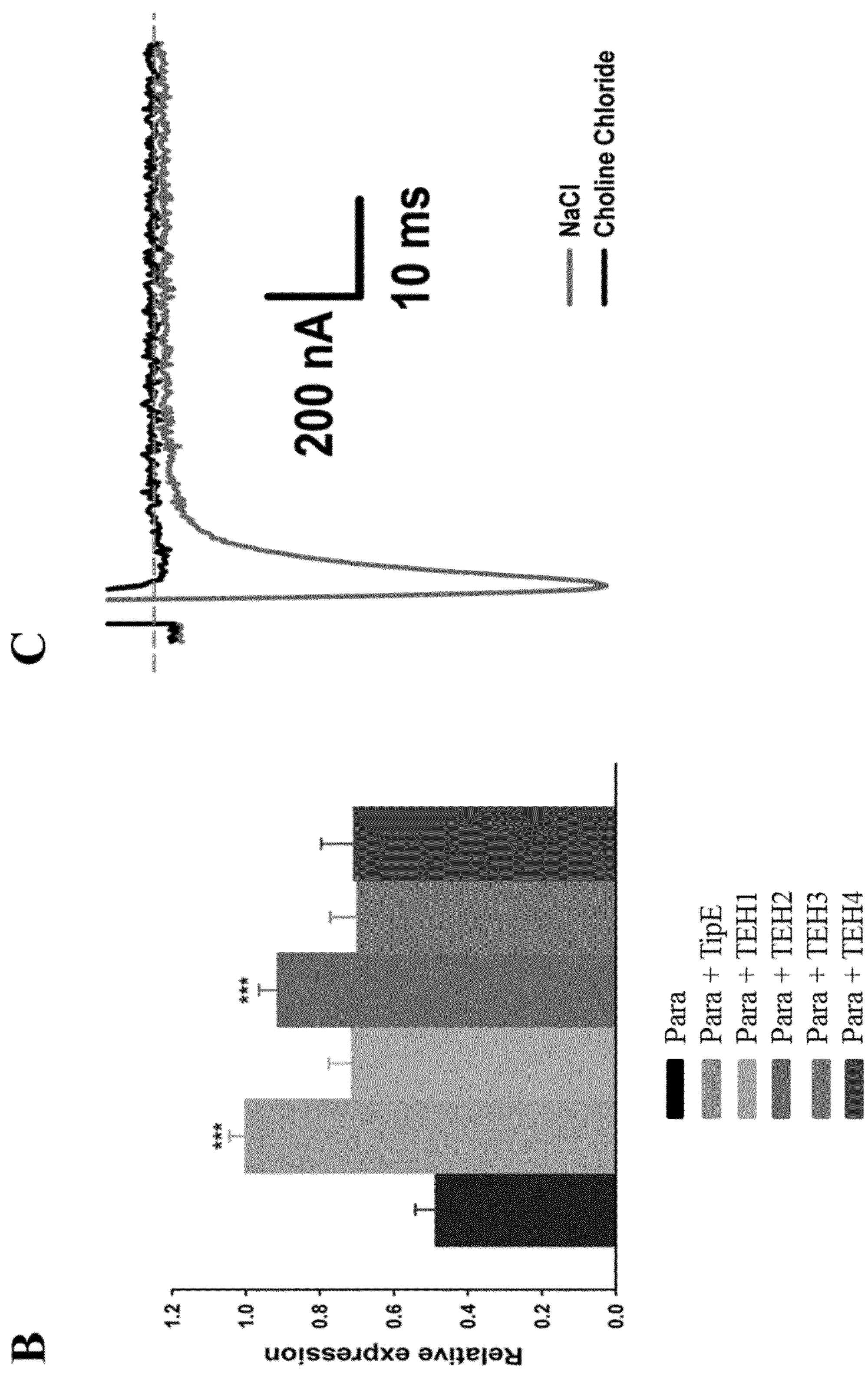
B
Relative expression
0.0
0.2
0.4
0.6
0.8
1.0
1.2
***
***
Para
Para + TipE
Para + TEH1
Para + TEH2
Para + TEH3
Para + TEH4
C
200 nA
10 ms
NaCl
Choline Chloride

FIG. 2 (cont.)

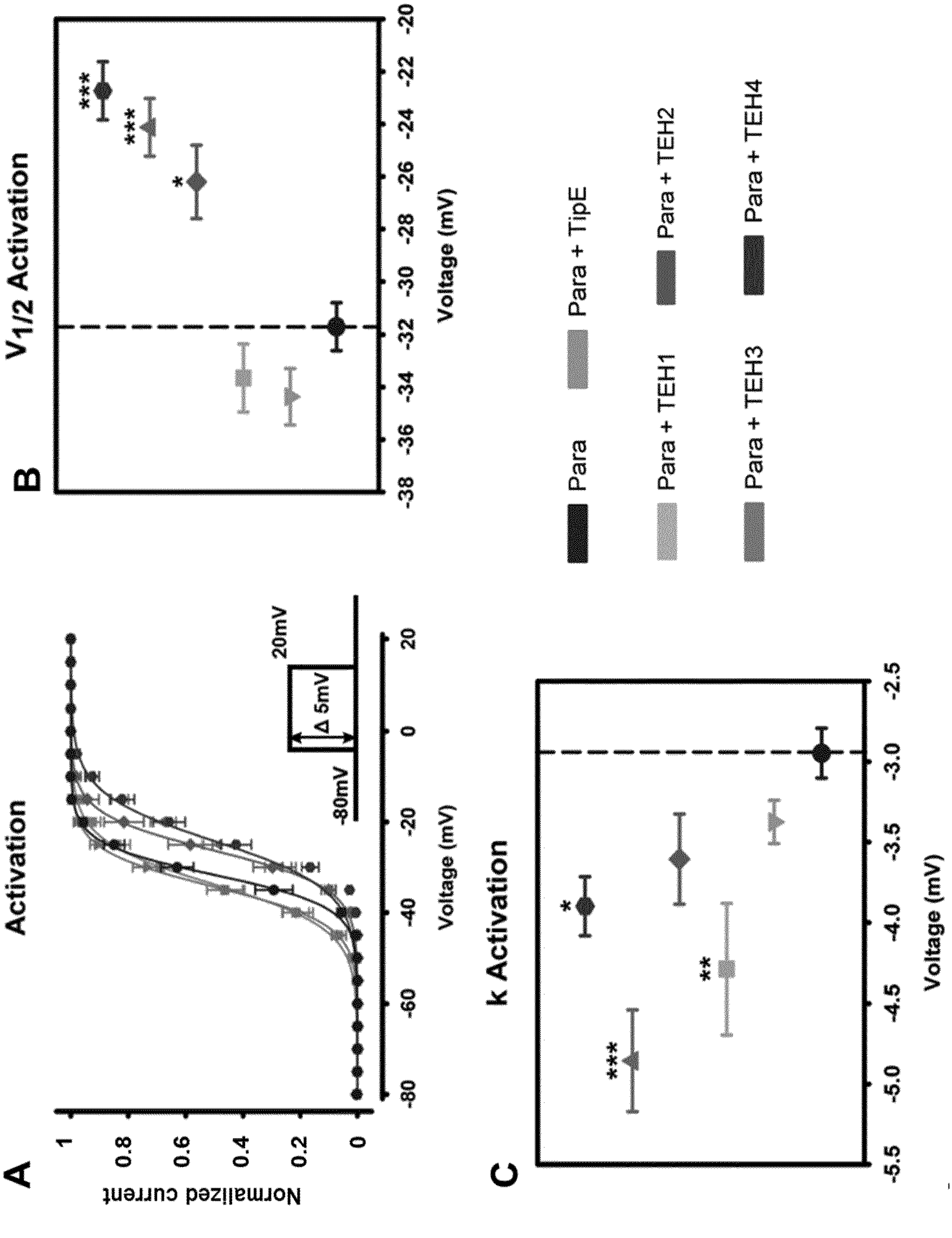
A
Activation
Normalized current
1
0.8
0.6
0.4
0.2
0
-80
-60
-40
-20
0
20
Voltage (mV)
-80mV
Δ 5mV
20mV
B
V1/2 Activation
-38
-36
-34
-32
-30
-28
-26
-24
-22
-20
Voltage (mV)
*
***
***
Para
Para + TipE
Para + TEH1
Para + TEH2
Para + TEH3
Para + TEH4
C
k Activation
-5.5
-5.0
-4.5
-4.0
-3.5
-3.0
-2.5
Voltage (mV)
***
**
*

FIG. 3

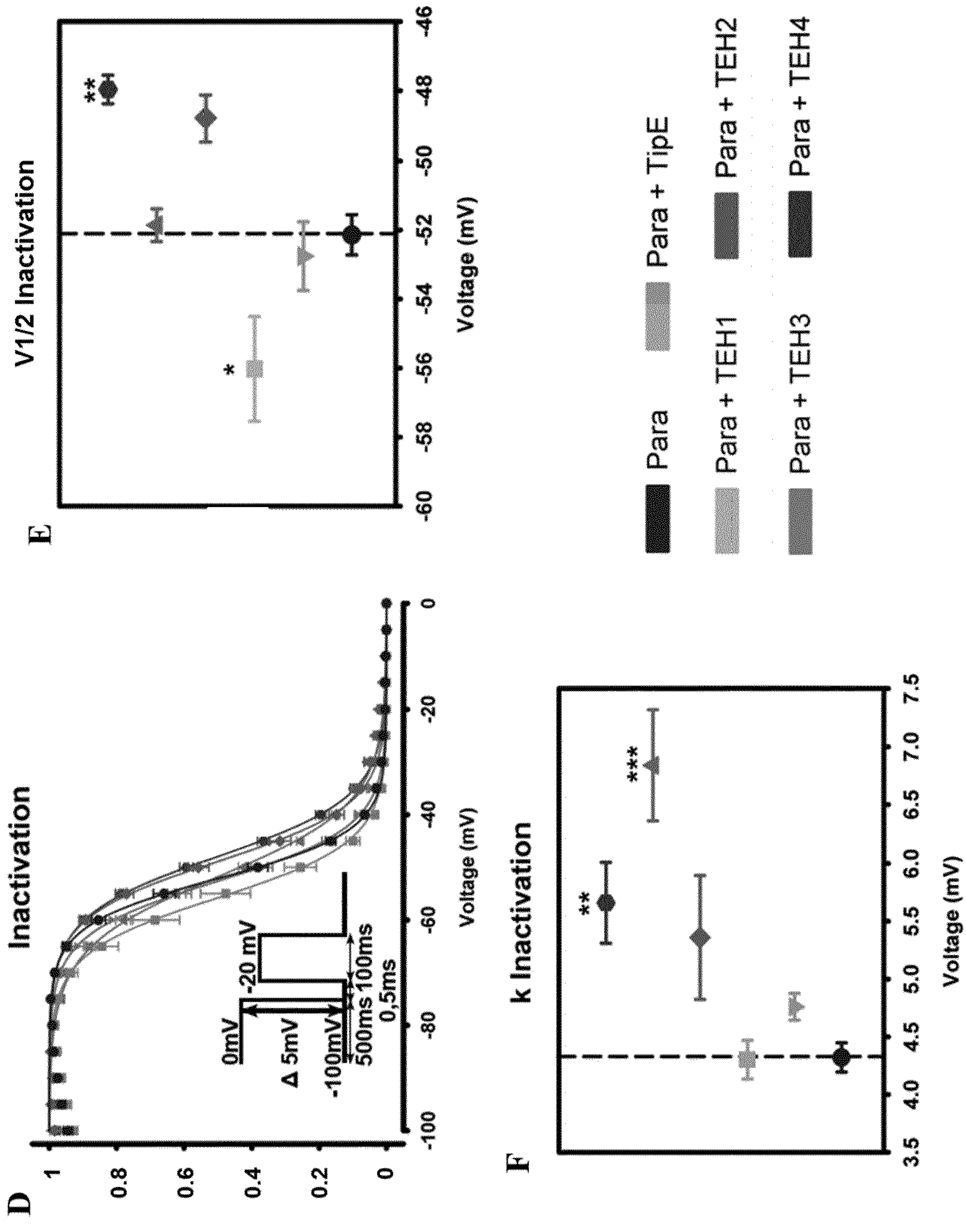
D
Inactivation
Normalized current
Voltage (mV)
0mV
-20 mV
Δ 5mV
-100mV
500ms 100ms
0,5ms
E
V1/2 Inactivation
Voltage (mV)
F
k Inactivation
Voltage (mV)
Para
Para + TipE
Para + TEH1
Para + TEH2
Para + TEH3
Para + TEH4

FIG. 3 (cont.)

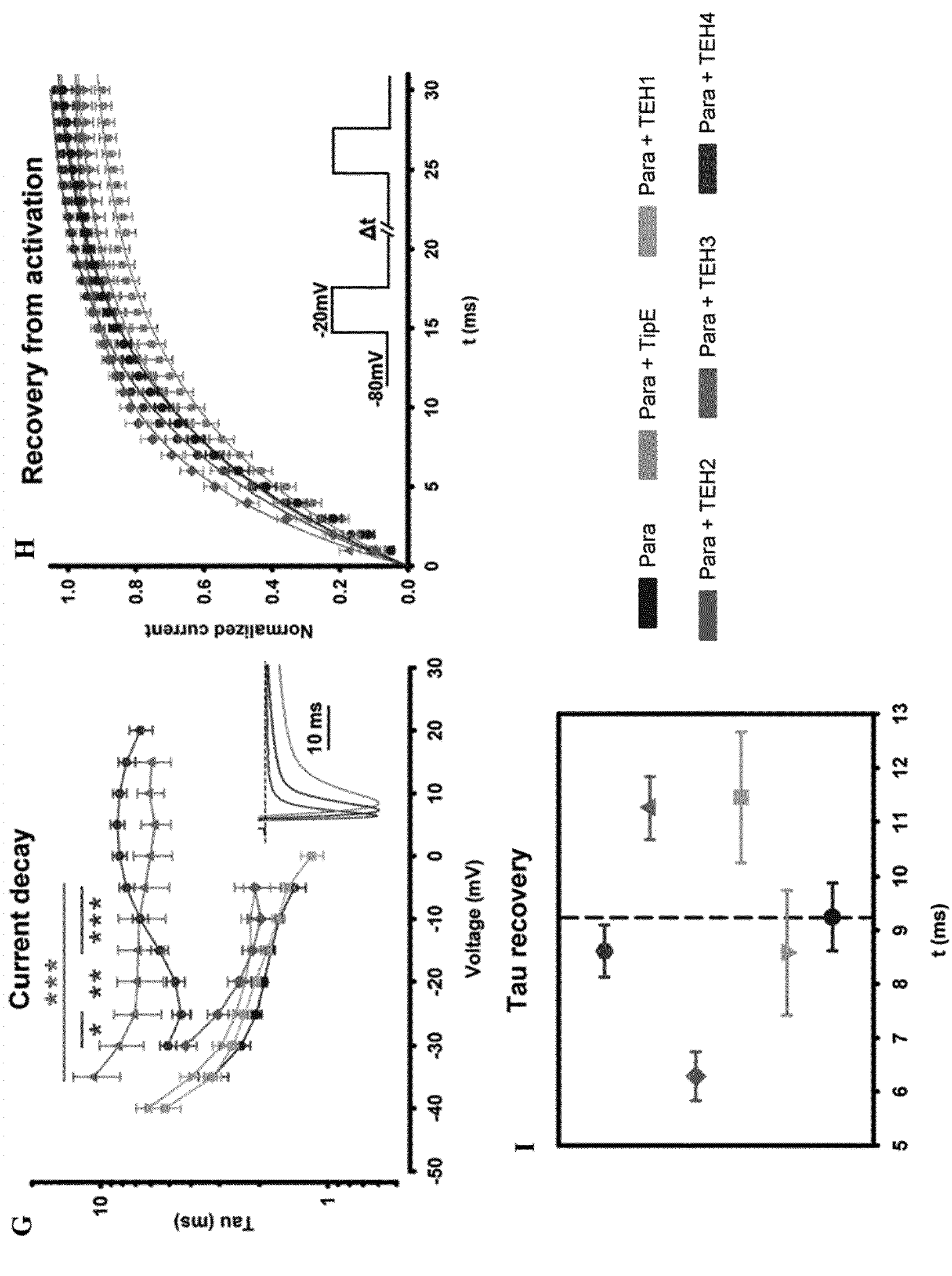
G
Current decay
Tau (ms)
Voltage (mV)
10 ms
H
Recovery from activation
Normalized current
t (ms)
-80mV
-20mV
Δt
I
Tau recovery
t (ms)
Para
Para + TipE
Para + TEH1
Para + TEH2
Para + TEH3
Para + TEH4

FIG. 3 (cont.)

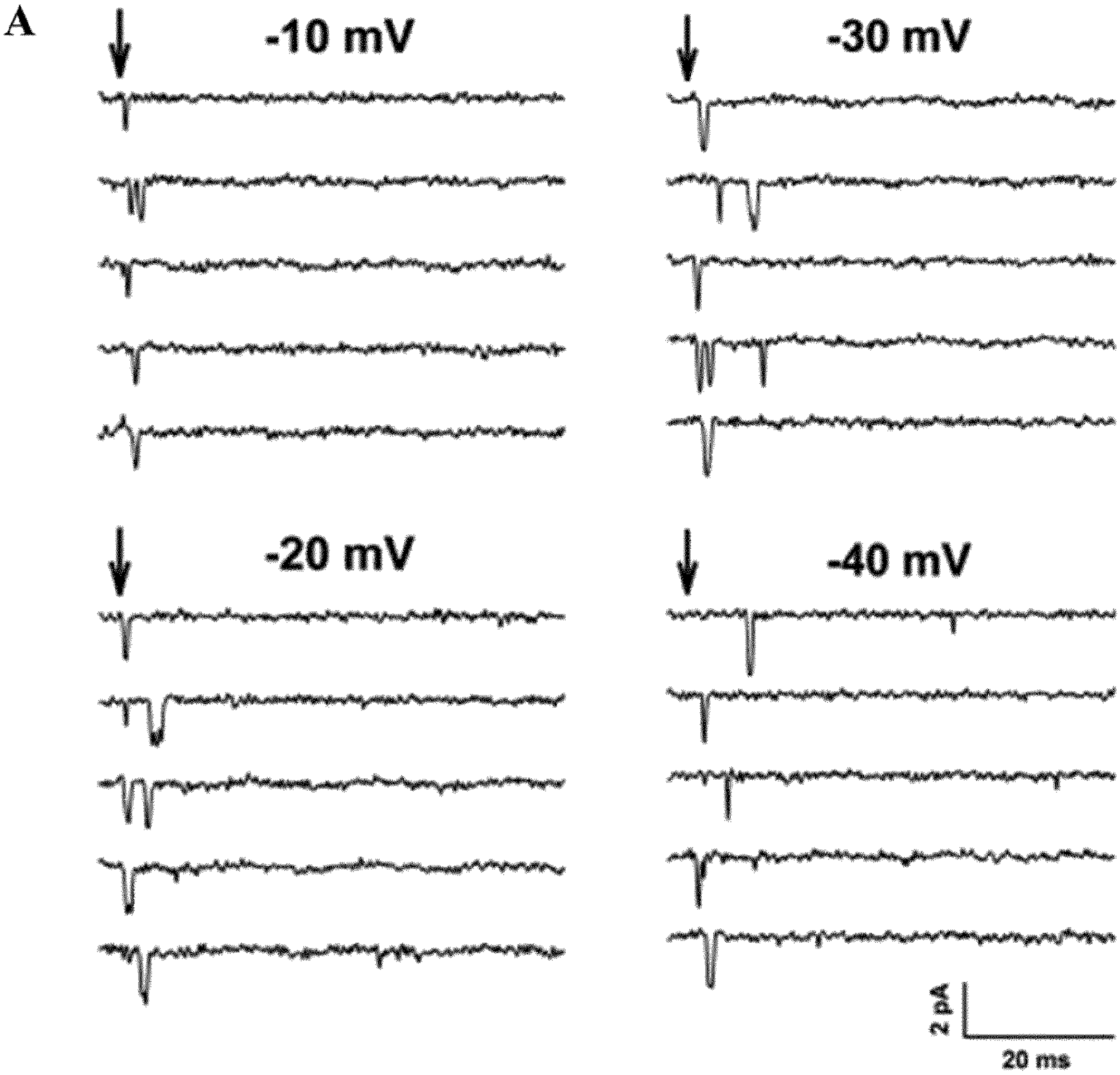


FIG. 4

B

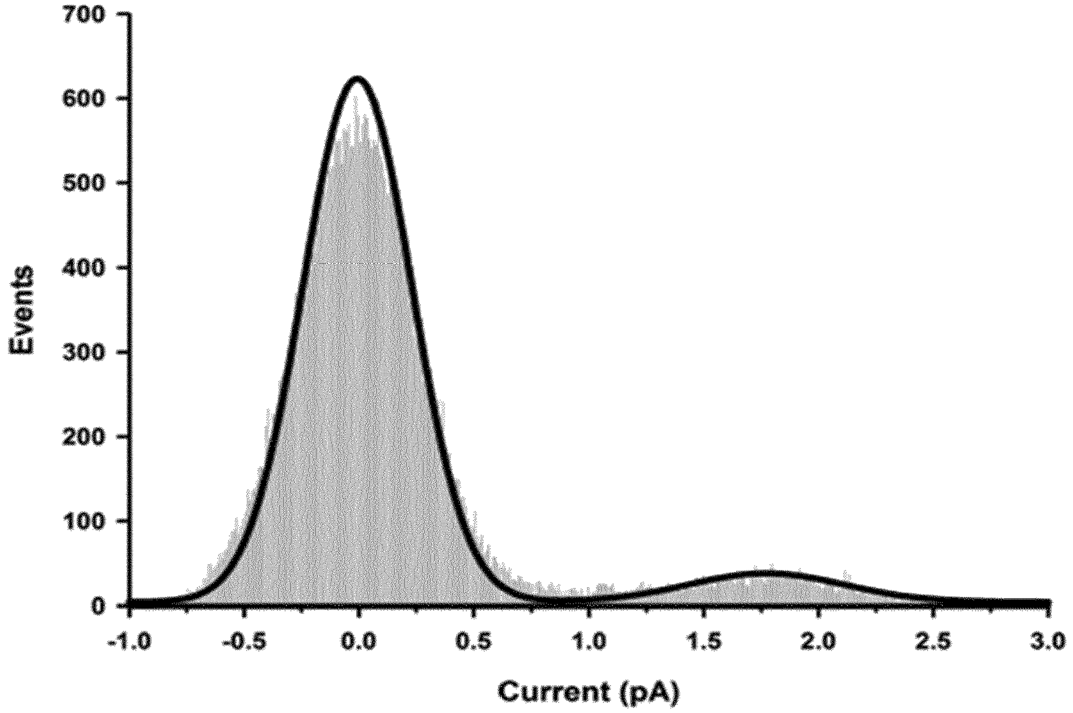
Events
700
600
500
400
300
200
100
0
-1.0
-0.5
0.0
0.5
1.0
1.5
2.0
2.5
3.0
Current (pA)

C

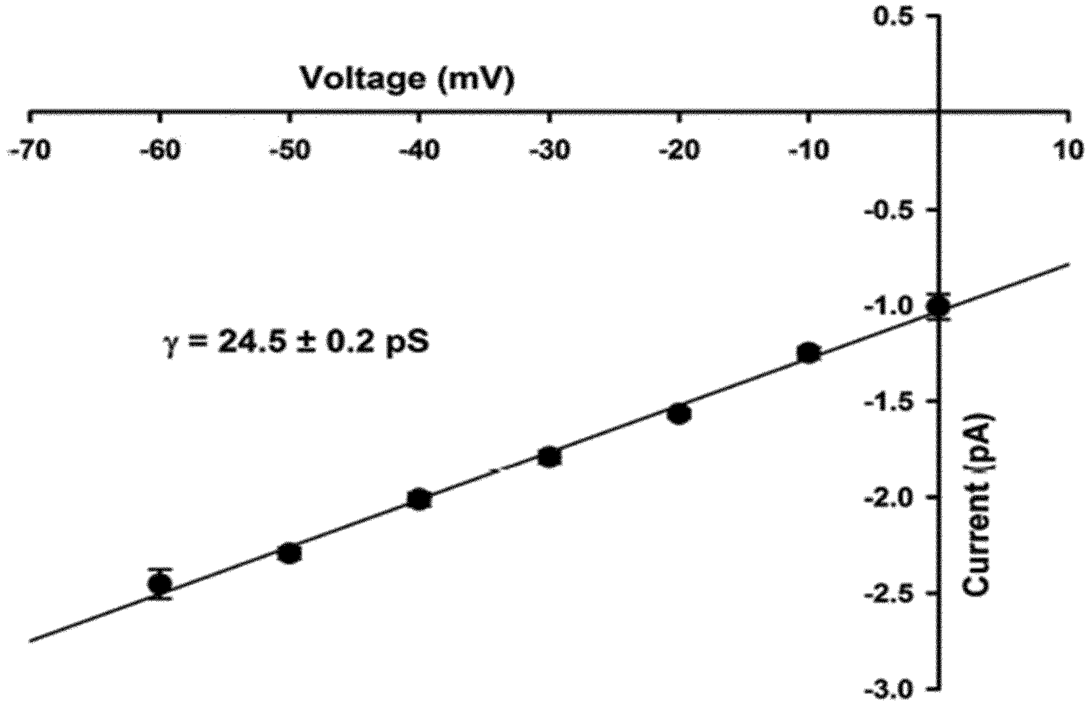
Voltage (mV)
-70
-60
-50
-40
-30
-20
-10
10
0.5
-0.5
-1.0
-1.5
-2.0
-2.5
-3.0
Current (pA)
γ = 24.5 ± 0.2 pS

FIG. 4 (cont.)

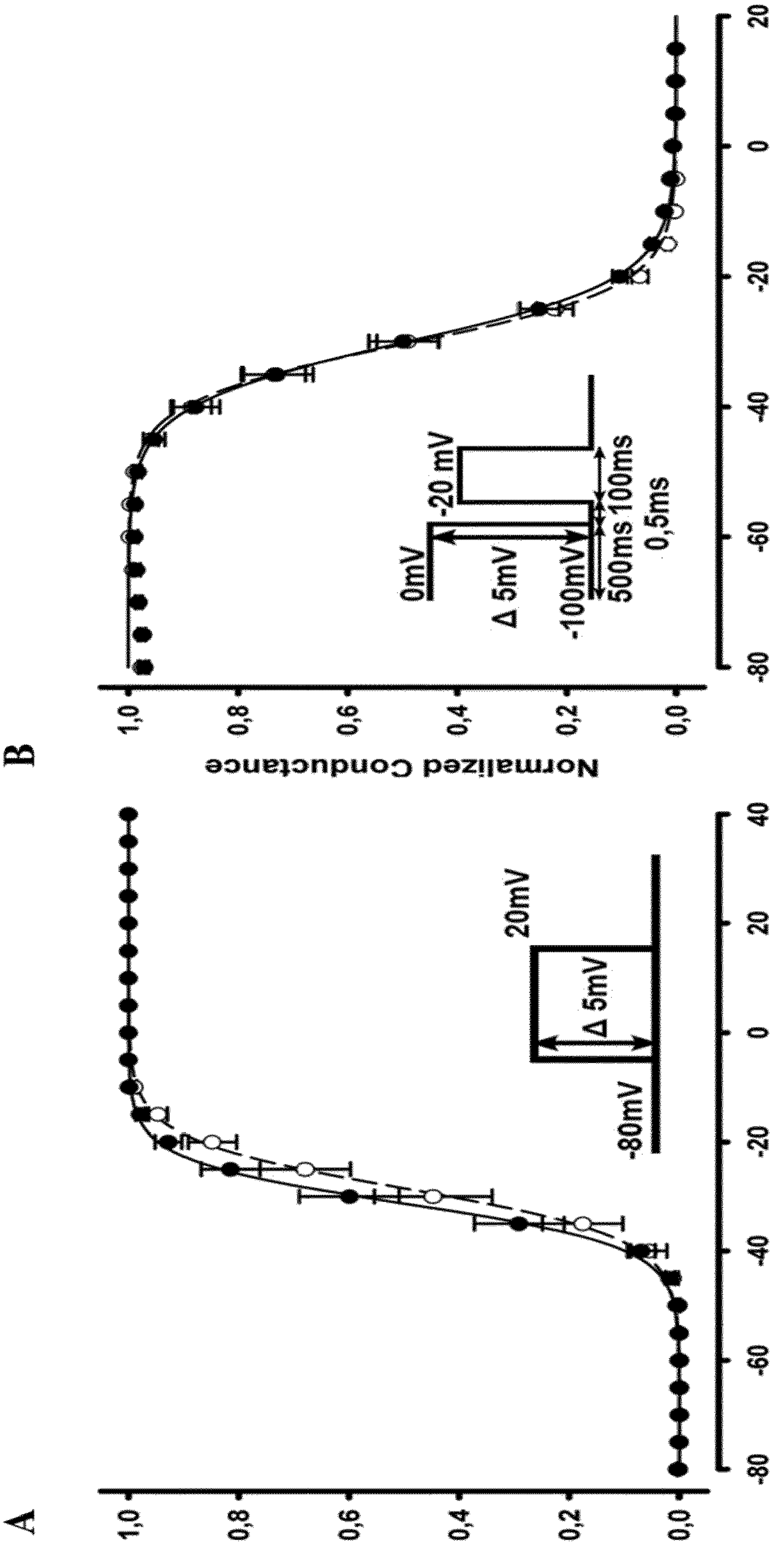
A
B
Normalized Conductance
Voltage (mV)
20mV
Δ 5mV
-80mV
0mV
-20 mV
-100mV
500ms 100ms
0,5ms

FIG. 5

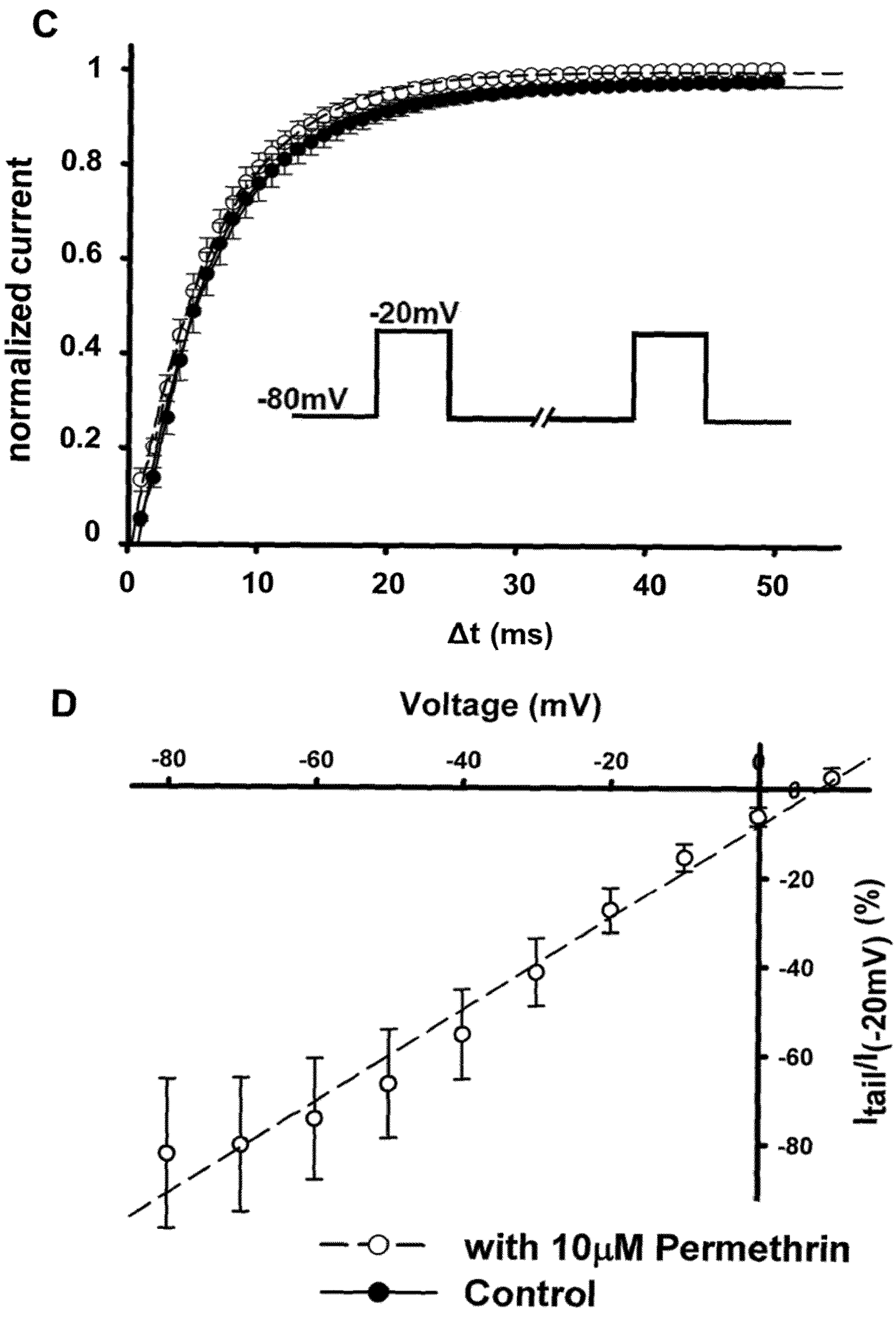
C
normalized current
1
0.8
0.6
0.4
0.2
0
-20mV
-80mV
0
10
20
30
40
50
Δt (ms)
D
Voltage (mV)
-80
-60
-40
-20
0
-20
-40
-60
-80
$I_{tail}/I_{(-20mV)}$ (%)
with 10μM Permethrin
Control

FIG. 5 (cont.)

# Permethrin

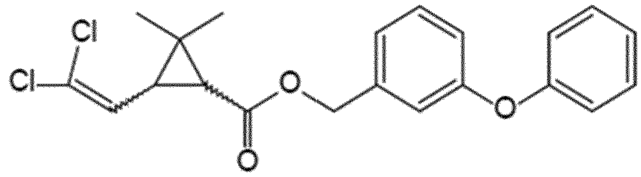

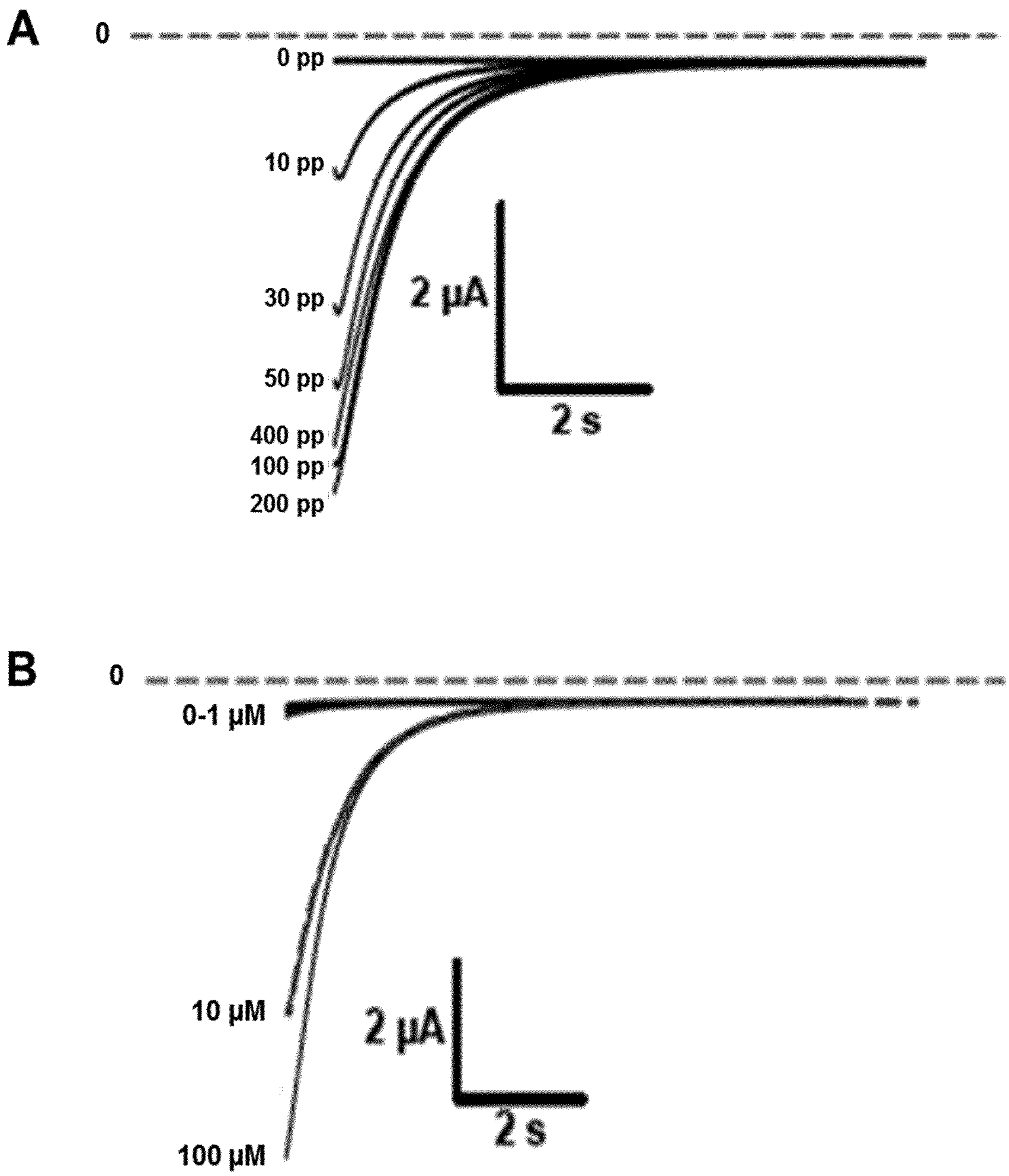


FIG. 6

C

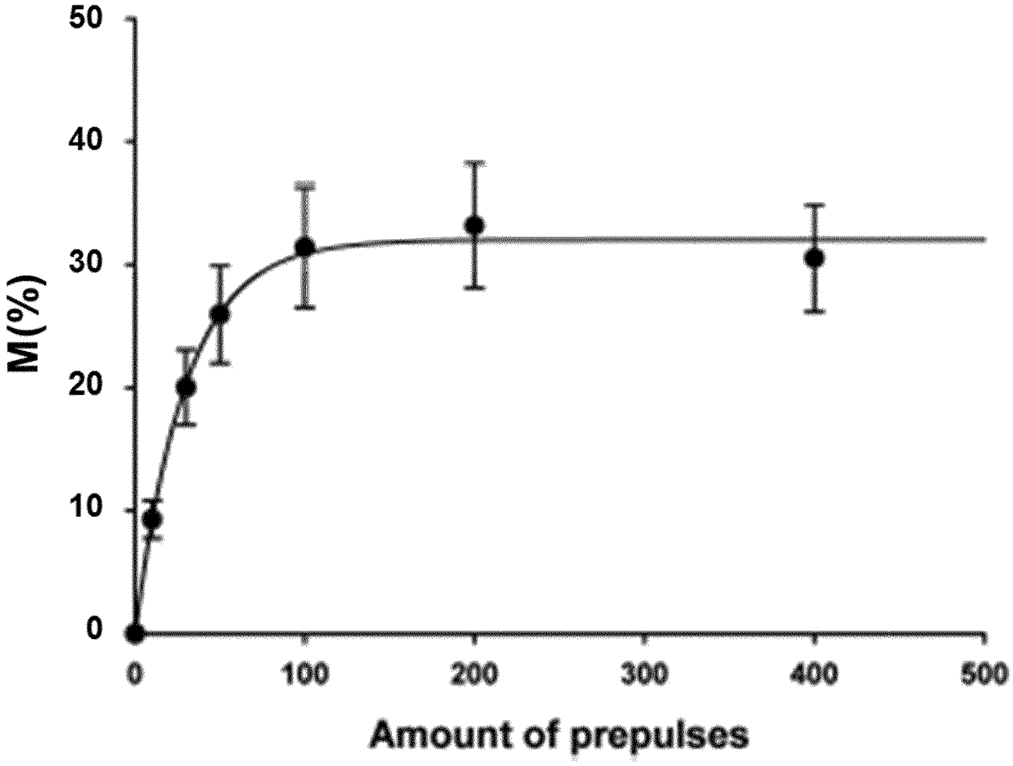

50
40
30
20
10
0
M(%)
0
100
200
300
400
500
Amount of prepulses


D

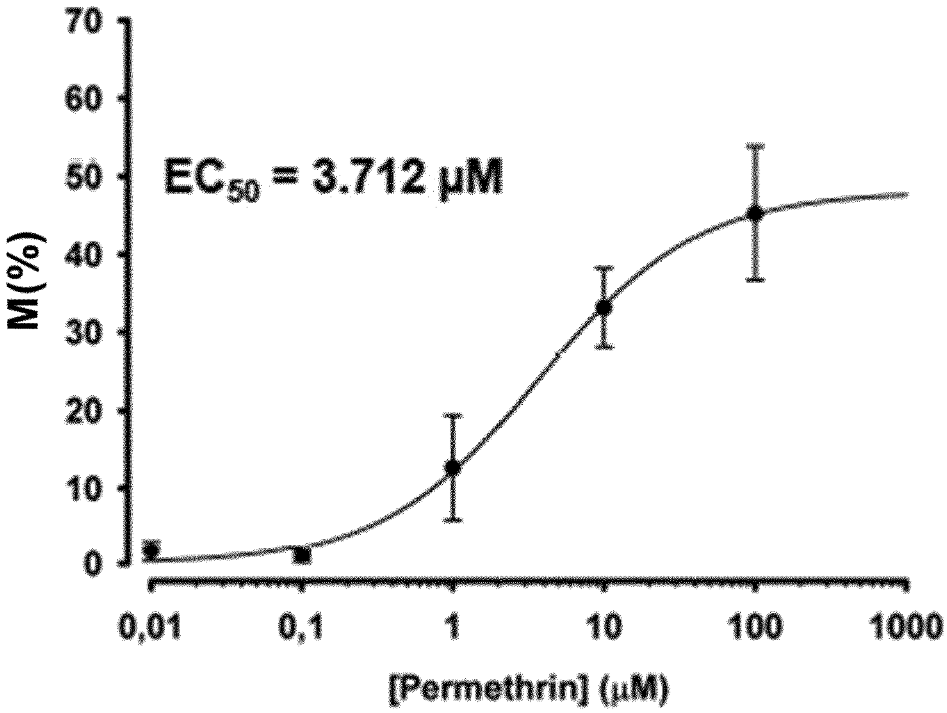

70
60
50
40
30
20
10
0
M(%)
EC50 = 3.712 μM
0,01
0,1
1
10
100
1000
[Permethrin] (μM)


FIG. 6 (cont.)

**Fenvalerate**

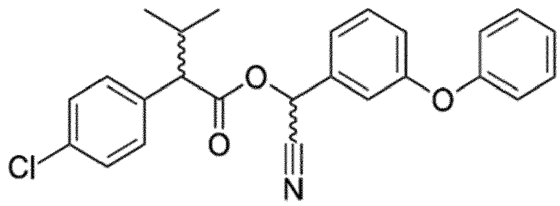

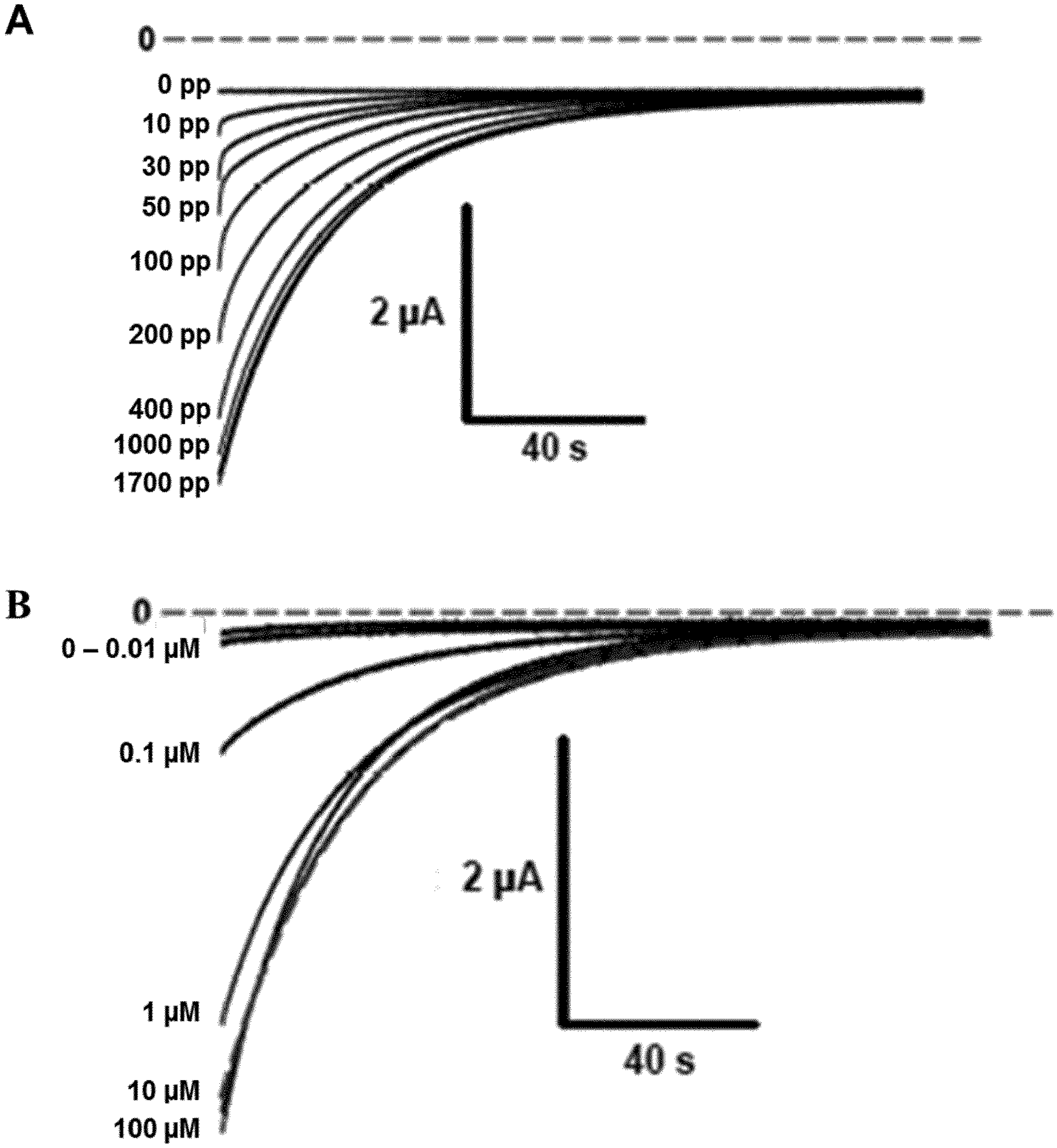


FIG. 7

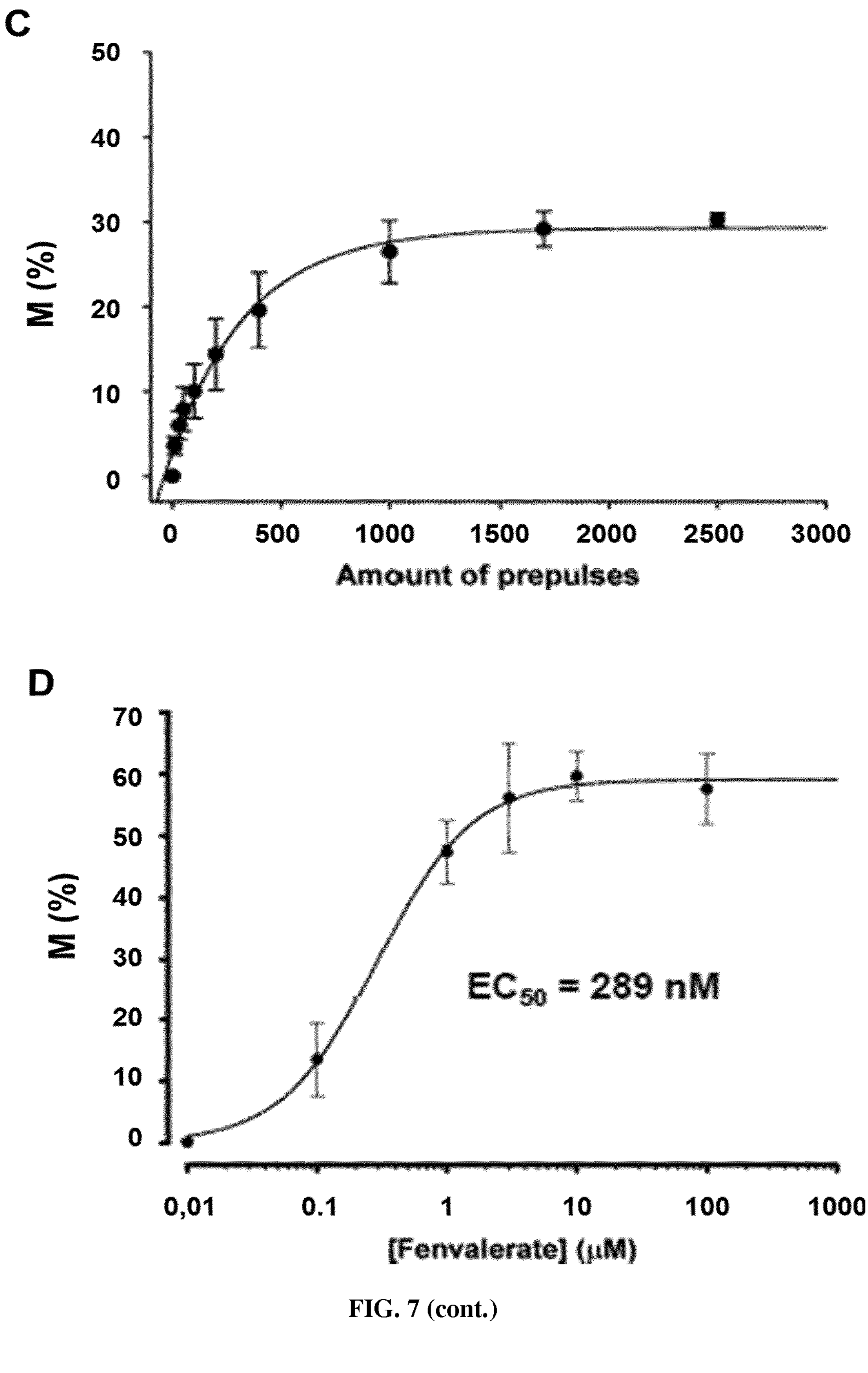
C
50
40
30
20
10
0
M (%)
0
500
1000
1500
2000
2500
3000
Amount of prepulses
D
70
60
50
40
30
20
10
0
M (%)
EC50 = 289 nM
0,01
0.1
1
10
100
1000
[Fenvalerate] (μM)

FIG. 7 (cont.)

Europäisches Patentamt
European Patent Office
Office européen des brevets

# EUROPEAN SEARCH REPORT

Application Number
EP 14 20 0741

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | DATABASE EMBL [Online]<br><br>24 September 2009 (2009-09-24), "Apis mellifera paralytic (PARA) mRNA, complete cds.", XP002740800, retrieved from EBI accession no. EM_STD:GQ202022 Database accession no. GQ202022<br>* sequence *<br>----- | 8,10-16<br><br>9 | INV.<br>C07K14/435<br>C12N5/075<br>C12N5/10<br>C12Q1/02 |
| X<br><br>Y | DERST C ET AL: "Four novel sequences in Drosophila melanogaster homologous to the auxiliary Para sodium channel subunit TipE", BIOCHEMICAL AND BIOPHYSICAL RESEARCH COMMUNICATIONS, ACADEMIC PRESS INC. ORLANDO, FL, US, vol. 339, no. 3, 20 January 2006 (2006-01-20), pages 939-948, XP024923399, ISSN: 0006-291X, DOI: 10.1016/J.BBRC.2005.11.096 [retrieved on 2006-01-20]<br>* page 943, left-hand column, paragraph 2 - right-hand column, paragraph 1 *<br>* table 1 *<br>----- | 1-4,6,7<br><br>9 | |
| A | KE DONG: "Insect sodium channels and insecticide resistance", INVERTEBRATE NEUROSCIENCE, SPRINGER, BERLIN, DE, vol. 7, no. 1, 6 January 2007 (2007-01-06), pages 17-30, XP019476055, ISSN: 1439-1104, DOI: 10.1007/S10158-006-0036-9<br>* page 19, left-hand column, paragraph 2 - page 20, left-hand column, paragraph 2 *<br>-----<br>-/-- | 1-16 | TECHNICAL FIELDS SEARCHED (IPC)<br>C07K<br>C12N |

The present search report has been drawn up for all claims

1

| Place of search | Date of completion of the search | Examiner |
|---|---|---|
| Munich | 11 June 2015 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 20 0741

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2014/155021 A1 (CENTRE NAT RECH SCIENT [FR]; AGRONOMIQUE INST NAT RECH [FR]; UNIVERSIT) 2 October 2014 (2014-10-02)<br>* abstract *<br>----- | 1-16 | |

TECHNICAL FIELDS SEARCHED (IPC)

The present search report has been drawn up for all claims

1

| Place of search | Date of completion of the search | Examiner |
|---|---|---|
| Munich | 11 June 2015 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 20 0741

This annex lists the patent family members relating to the patent documents cited in the above-mentioned European search report. The members are as contained in the European Patent Office EDP file on
The European Patent Office is in no way liable for these particulars which are merely given for the purpose of information.

11-06-2015

| Patent document cited in search report | Publication date | Patent family member(s) | Publication date |
|---|---|---|---|
| WO 2014155021 A1 | 02-10-2014 | FR 3003863 A1<br>WO 2014155021 A1 | 03-10-2014<br>02-10-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0689051 A3
- WO 9854294 A


### Non-patent literature cited in the description


- **SUCHAIL S et al.** *Environ Toxicol Chem,* 2001, vol. 20 (11), 2482-6
- **GELS JA.** *J Econ Entomol,* 2002, vol. 95 (4), 722-8
- **CESTELE ; CATTERALL.** *Biochimie,* 2000, vol. 82, 883-892
- **WANG SY ; WANG GK.** *Cell Signal,* 2003, vol. 15, 151-159
- **SODERLUNG.** *Pestic Biochem Physiol.,* 01 June 2010, vol. 97 (2), 78-86
- **NARAHASHI T.J.** *Pharmacol Exp Ther.,* July 2000, vol. 294 (1), 1-26
- **SILVER ; SODERLUND.** *Pestic Biochem Physiol.,* 2005, vol. 81, 136-143
- **DONG K.** *Invert Neuroscience,* 2007, vol. 7 (1), 17-30
- **SAMBROOK et al.** Molecular Cloning: A Laboratory Manual. Cold Spring Harbor Laboratory Press, 1989
- Guide to Molecular Cloning Technics. Methods in Enzymology. 1987, vol. 152, 359-371
- **BLOOMQUIST ; SODERLUND.** *Mol Pharmacol,* 1988, vol. 33, 543-550
- **DERI ; ADAM-VIZI.** *J Neurochem,* 1993, vol. 61, 818-825
- **CONNOLLY et al.** *Biosens. Biores.,* 1990, vol. 5, 223-234
- **CHAHINE M et al.** *J Mol Cell Cardiol,* 1992, vol. 24, 1231-1236
- **COLLET C et al.** *J Exp Biol,* 2007, vol. 210, 454-464
- **TAYLOR KS ; WALLER GD ; CROWDER LA.** Impairment of a Classical Conditioned Response of the Honey Bee (Apis Mellifera L.) by Sublethal Doses of Synthetic Pyrethroid Insecticides. *Apidologie,* 1987, vol. 18, 243-252
- **KADALA A et al.** *Neurotoxicology,* 2011, vol. 32, 320-330
- **VAIS H et al.** *Mol Pharmacol,* 2003, vol. 64, 914-922
- **RINKEVICH et al.** *Pestic Biochem Physiol,* 2013, vol. 106, 93-100
- **TAN J et al.** *Insect Biochem Mol Biol,* 2002, vol. 32, 445-454
- **LIU Z et al.** *Insect Biochem Mol Biol,* 2006, vol. 36, 885-889
- **DU et al.** *Proc Natl Acad Sci U S A,* 2013, vol. 110, 11785-11790
- **ROSENKRANZ P et al.** Biology and control of Varroa destructor. *J Invertebr Pathol,* 2010, vol. 103 (1), 96-119